# EUROPEAN PATENT SPECIFICATION

(11) **EP 2 002 711 B1**
(45) Date of publication and mention of the grant of the patent: **17.11.2010**
(21) Application number: 08010783.2
(22) Date of filing: 13.06.2008
(51) Int. Cl.: A01H 5/10, C12N 15/82

(54) **New hybrid system for brassica napus**
Neues Hybridsystem für Brassica napus (Raps)
Nouveau système hybride de brassica napus

(30) Priority: 13.06.2007 EP 07290741
(43) Date of publication of application: 17.12.2008
(60) Divisional application: 10159412.5
(73) Proprietor: Syngenta Participations AG, 4058 Basel (CH)
(72) Inventor: Stiewe, Gunther, 32657 Lemgo (DE); Pleines, Stephan, 32130 Enger (DE); Coque, Marie, 31140 Aucamville (FR); Gielen, Jan, 31620 Bouloc (FR)
(74) Representative: Kock, Michael Andreas

(56) References cited:
- WO-A-01/41558
- WO-A-98/56948
- DATABASE EMBL [Online] 25 February 2002 (2002-02-25), "BOMPL05TF BO_2_3_KB Brassica oleracea genomic clone BOMPL05, DNA sequence." XP002463861 retrieved from EBI accession no. EMBL:BH708933 Database accession no. BH708933
- DATABASE EMBL [Online] 30 November 2002 (2002-11-30), "llf03b03.b1 B.oleracea002 Brassica oleracea genomic, DNA sequence." XP002463862 retrieved from EBI accession no. EMBL:BZ061557 Database accession no. BZ061557
- DATABASE EMBL [Online] 19 December 2001 (2001-12-19), "BOGJK40TF BOGJ Brassica oleracea genomic clone BOGJK40, DNA sequence." XP002463863 retrieved from EBI accession no. EMBL:BH589881 Database accession no. BH589881
- LU G Y ET AL: "Molecular mapping of a dominant genic male sterility gene Ms in rapeseed (Brassica napus)" PLANT BREEDING, vol. 123, no. 3, June 2004 (2004-06), pages 262-265, XP002463858 ISSN: 0179-9541
- HONG DENGFENG ET AL: "AFLP and SCAR markers linked to the suppressor gene (Rf) of a dominant genetic male sterility in rapeseed (Brassica napus L.)" EUPHYTICA, vol. 151, no. 3, October 2006 (2006-10), pages 401-409, XP002463859 ISSN: 0014-2336
- TAKAGI Y: "MONOGENIC RECESSIVE MALE STERILITY IN OIL RAPE-D BRASSICA-NAPUS-D INDUCED BY GAMMA IRRADIATION" ZEITSCHRIFT FUER PFLANZENZUECHTUNG, vol. 64, no. 3, 1970, pages 242-247, XP009094294 ISSN: 0044-3298
- THEIS R ET AL: "ANTHER AND MICROSPORE DEVELOPMENT IN DIFFERENT MALE STERILE LINES OF OILSEED RAPE BRASSICA-NAPUS L" ANGEWANDTE BOTANIK, vol. 64, no. 5-6, 1990, pages 419-434, XP009094292 ISSN: 0066-1759
- DONG N V ET AL: "Molecular mapping of a rice gene conditioning thermosensitive genic male sterility using AFLP, RFLP and SSR techniques" THEORETICAL AND APPLIED GENETICS, SPRINGER, BERLIN, DE, vol. 100, no. 5, March 2000 (2000-03), pages 727-734, XP002188736 ISSN: 0040-5752
- TANG J H ET AL: "Genetic analyses and mapping of a new thermo-sensitive genic male sterile gene in maize" THEORETICAL AND APPLIED GENETICS, vol. 113, no. 1, June 2006 (2006-06), pages 11-15, XP002463860 ISSN: 0040-5752
- HONG D F ET AL: "Development and characterization of SCAR markers associated with a dominant genic male sterility in rapeseed" PLANT BREEDING, vol. 127, no. 1, February 2008 (2008-02), pages 69-73, XP002499620 ISSN: 0179-9541

## Description

### FIELD OF THE INVENTION

The present invention relates to a nuclear conditional male sterility system in *Brassica napus.* Embodiments of the present invention provide methods for the production of the prebasic (male sterile) female (MsMsrfrf), the (male fertile) maintainer line (msmsrfrf), the basic (male sterile) female line (Msmsrfrf), and hybrid lines. Further embodiments of the present invention relate to markers associated to the sterility, fertility and maintainer alleles, respectively, and the use of those markers in providing a hybrid system.

### BACKGROUND OF THE INVENTION

Oilseed from Brassica plants is an increasingly important crop. As a source of vegetable oil, it presently ranks only behind soybeans and palm in commercial importance and it is comparable with sunflowers. The oil is used both as a salad and cooking oil, and play an increasingly important role in biofuels (biodiesel).
In its original form, Brassica oil, known as rapeseed oil, was harmful to humans due to its relatively high level of erucic acid. Erucic acid is commonly present in native cultivars in concentrations of 30-50% by weight based upon the total fatty acid content. This problem was overcome when plant scientists identified a germplasm of low erucic acid (Stefansson, 1983). Although these varieties with less than 2% of erucic acid in their total fatty acid profile (single zero quality) yielded edible oil, the continuing presence of sulfur compounds called glucosinolates (GSLs) in the high protein meal remained a major constraint to further market expansion. Wide acceptance of rapeseed meal for animal nutrition is hampered by the presence of GSLs in the seed. Furthermore, glucosinolates are also undesirable since they can lead to the production of antinutritional breakdown products (e.g., thiocyanates, isothiocyanate and nitrite) upon enzymatic cleavage during oil extraction and digestion when acted upon by the endogenous enzyme myrosinase during crushing. In consequence, so-called "double-low" varieties (low in erucic acid in the oil as well as low in glucosinolates in the solid meal after oil extraction) were developed, which have an erucic acid content of less than 2% by weight based upon the total fatty acid content, and a glucosinolate content of less than 30 µmol/gram of the oil-free meal. These high quality forms of rape, first developed in Canada, are known as canola. At present the maximum threshold set by European law is 25 µmol total glucosinolate (GSL) per gram (g) of seed at 8.5% moisture, as measured by HPLC (EU decree 2294/92). Double low spring canola varieties cultivated in Canada need to have GSL levels of less than 30 µmoles GSLs per gram of air-dried oil-free meal at 0% moisture, as measured by TMS. The GSL levels of commonly cultivated double zero oilseed rape varieties in Europe and Canada varies significantly below the threshold levels at 60% of the official threshold level or even lower. However, many countries are requiring even lower levels of glucosinolates in order to register canola varieties.
In addition, plant scientists have attempted to improve the fatty acid profile for rapeseed oil (Röbbelen, 1984; Ratledge et al., 1984; Röbbelen, 1975; Rakow & McGregor, 1973).
Especially winter oilseed rape (*Brassica napus* L. ssp. *oleifera* (Metzg.), Brassicaceae) is an important crop for the production of oilseed in temperate agricultural regions. In Germany in 2006 approximately 1.5 million hectares (12% of the total agricultural area) were sown with oilseed rape.
Oilseed rape is a predominantly self-pollinated crop with about one-third outcrossing (Becker et al., 1992). Breeding of rapeseed plants have been centered on open-pollinated seeds by taking advantage of high self-compatibility affinity of said plants. The significant heterosis for seed yield in oilseed rape has created interest in the development of hybrid cultivars (Riaz et al., 2001). Heterosis means the growth and yield advantage of hybrids in comparison to their parents gained by the crossing of two genetically different, homozygous genotypes (Shull, 1922). Rapeseed hybrids always show a significant heterosis in yield. Considerable heterosis for seed yield in F1 hybrids of oilseed rape (*Brassica napus* L.) has been reported by various authors at the beginning of hybrid breeding in rapeseed (Schuster, 1969; R6bbelen, 1985; Grant & Beversdorf, 1985; Lefort-Buson et al., 1987; Brandle & McVetty, 1989; Paulmann & Frauen, 1991; Stefansson, 1983; Brandle & McVetty, 1990; Shen, 2005). Principally the heterosis level in spring type rapeseed can be as high as 20% to 30%, and in winter type rapeseed about 30% to 40%.
An effective production of hybrid seeds requires both the identification of heterotic groups (genetic distinct genepools; Melchinger & Gumber, 1998, Becker & Link, 2000) and a method for targeted crossing of those heterotic groups.
In winter oilseed rape the first hybrid varieties that were registered in Europe are hybrid line-associations using the INRA ogura system and fully restored hybrids using the MSL system (see below for details). However, in comparison to elite inbred lines the heterosis effect is still moderate. The relatively low gain in yield is, however, not caused by the inefficiency of the hybrid system, but by the lack of genetic diverse gene pools in rapeseed In consequence of decades of inbreeding and governmental regulations (e.g., glucosinolate or erucic acid content), which still cause limited germplasm variability.

More diverse gene pools will lead to higher heterosis effects but their development was initiated only recently. Nevertheless, a commercially functional hybrid system is the predominant prerequisite for achieving significant yield increases in rapeseed in the future. Those increases are not only required by the increased demand for food purpose but the rapidly increasing demand in biofuels (biodiesel).
Beside chemical-induced male sterility (CHA), three genetics based hybrid system principles have been explored in Brassica varieties: Breeders use self-incompatible (SI), cytoplasmic male sterile (CMS), and nuclear male sterile (NMS; formerly also genetic male sterility, G MS) Brassica plants as the female parent (for review of hybrid systems in vegetables see Kumar et al., 2004). SI plants are not able to self pollinate due to their genetic constitution and CMS as well as NMS female plants are incapable of producing pollen. Thus, all these plants must be cross-pollinated by a male fertile parent. In using these plants, breeders are attempting to improve the efficiency of seed production and the quality of the F1 hybrids and to reduce the breeding costs. When hybridisation is conducted without using Si, CMS or NMS plants, it is more difficult to obtain and isolate the desired traits in the progeny (F1 generation), because the parents are capable of undergoing both cross-pollination and self-pollination.
A simple and efficient pollination control system is the key step for utilizing heterosis in commercial hybrid seed production. If one of the parents is a SI, CMS or NMS plant that is not able to self-pollinate or is incapable of producing pollen, only cross pollination will occur. By eliminating the pollen of one parental variety in a cross, a plant breeder is assured of obtaining hybrid seed of uniform quality, provided that the parents are of uniform quality and the breeder conducts a single cross.

**Self-incompatiblilty systems:** So far no commercially useable SI system for rapeseed has been developed. Canadian patent CA 2,143,781 describes a hybrid breeding method for crop plants in the family Brassicaceae in which an F1 seed is produced by crossing the female parent of a self-incompatible male sterile line with a male parent. However, the main question for SI is the reproduction of SI parent lines in large scale, which makes this system difficult for commercial applications.

**Cytoplasmic male sterility (CMS):** CMS is a maternally inherited phenomenon, the genetic determinants of which are located in the genome of the cytoplasmic organelles, the mitochondria. Such plants are severely impaired in their ability to produce functional pollen grains. Restorer genes for CMS systems are dominant nuclear genes, which suppress male sterile effects of the cytoplasm. The expression of male sterility in cms plants is the result of incompatibility between recessive nuclear gene (called maintainer allele; *rf)* and male sterile specific cytoplasmic genome.
CMS systems used in the commercial production of F1 hybrid of rapeseed plants are the Polima (pol; Fu, 1981), Kosena, and the Ogura system (Ogura, 1968; Makaroff, 1989; Pellan-Delourme et al., 1987; US 5,254,802; US20040237141, US20020032918, EP 0 599 042; US 6,229,072).
The Polima cytoplasmic male sterility (Pol CMS) system has been used mainly in hybrid rapeseed production in China. However, because of its inherent limitations, such as instability of the sterility, the limited number of restorers and potential negative influence of the cytoplasm, the use of hybrids with single CMS cytoplasm over large areas is not ideal. The main disadvantage of pol CMS is the instability of male sterility under high temperature. The male sterile lines could become partial fertile at relative low or high temperature situations (Yang & Fu, 1987).
The Ogura (ogu; Ogura, 1968; Pelletier et al., 1983; Heyn, 1976) and the Kosena system both rely on a radish-derived CMS gene. A fertility restorer for Ogura cytoplasmic male sterile plants has been transferred from *Raphanus sativus* (radish) to Brassica (Pelletier et al., 1987), because rapeseed lacks an Rf allele corresponding to said CMS gene. The Ogura system is described in EP0 599 042, EP 0 671 121, and WO2005/074671. The restorer allele originating from radish is phenotypically described (WO 92/05251; Delourme et al., 1991). Initial Ogura restorer material showed reduced female fertility and a high content of glucosinolates closely linked to its fertility restoring gene, which were overcome through lengthy backcrossing (Delourme et al., 1991, Renard et al., 1997). EP 1 493 328 describes a method of producing double low restorer lines of *Brassica napus* for Ogura cytoplasmic male sterility. Although the glucosinolate content is reduced in those lines after laborious backcrossing, there seem to be some inherent problems associated to the Rf alleles such as yield drag under higher temperatures, a decreased seed set, and a reduced number of ovules per silique (Pellan-Delourme & Renard, 1988; Delourme et al., 1994), lower seed yields, poor disease resistances and lodging susceptibility. Some of those properties have a close linkage to the restorer allele (Delourme et al., 1994, 1995) and data suggest that certain of those properties may be endogenous to the restorer allele and may not be able to overcome by breeding or even transgenic approaches with the isolated restorer allele.
One inherent disadvantage of the CMS system is the propagation of a homozygous female CMS line. Since the male-sterile, female CMS A-line cannot self-pollinate, it must be maintained by crossing said A-line with a maintainer B-line that is male fertile and genetically identical to the A-Line. The result of this cross is a male-sterile CMS A-line.

Nuclear male sterility (NMS): Nuclear male sterility (earlier termed as genic male sterility gms) is controlled by the gene(s) from the nuclear compartment. Most of the naturally occurring or induced male sterile mutants are recessive in nature with few exceptions in cole vegetables (e.g., cabbage and broccoli) and genetically transformed male sterile lines (Kaul, 1988, Williams et al., 1997). Although functional pollen is produced, the pollen of certain mutants fails to self fertilize, either due to non-dehiscence of pollen or the special flower morphology of the plants, e.g. positional sterility in tomato (Atanassova, 1999) and functional male sterility in eggplant (Phatak & Jaworski, 1989). The occurrence of predominantly recessive male sterility indicates that gms is the result of mutation in any gene(s) controlling microsporogenesis (pollen development process), stamen development or microgametogenesis (male gamete development process).
NMS systems have the advantage of complete and stable sterility with almost no negative cytoplasmic effects. Several kinds of NMS mutants have been discovered in *Brassica napus* and the sterility of these mutants has been reported to be controlled by one gene (Takagi, 1970; Mathias, 1985; Hu, 2000), two genes (Li et al., 1985, 1988, 1993, 1995; Hou et al., 1990; Tu et al., 1997a), three genes (Chen et al., 1998; Wang et al., 2001) and one gene with multiple alleles (Song, 2005). However, the systems are practically difficult to handle and often demonstrate high sensibility to environmental effects such as for example heat. Thus, compared to the CMS system, application of the dominant NMS method in rapeseed production is far behind, mainly due to the complicated fertility inheritance and the difficulties in distinguishing the different genotypes in a segregating population. The breeding procedure for a homozygous two-type line is very complicated, and the identification of the different genotypes (*Ms Rf*) with expected traits in backcross or selfed generations is laborious and time-consuming with traditional breeding methodology.
In China, three NMS systems have been used in hybrid breeding, and a few hybrids have been registered. Although a recessive NMS has advantages (it needs only two lines), it also has the fatal shortcoming of being difficult to drive a whole male sterile population. Since gms is maintained through backcrossing, in hybrid seed production in the field 50% male fertile segregants *(Msms)* need to be identified and removed before they shed pollen. The removal of the 50% male fertile plants from the female lines can be realized for example by marker-assisted selection (Tu et al., 1999). These methods, however, are expensive, labor extensive, and commercially not competitive. Because of more tedious maintenance process and non-availability of a suitable marker gene among the vegetable crops, utilization of gms is restricted to only a few vegetables. No system in rapeseed has been developed so far. The identification of fertilizing cytoplasm for specific nuclear male sterile gene (Homer & Palmer, 1995) is an interesting research area, which upon success, may provide opportunity for most efficient utilization of lines, like cms line.
One special form of the nuclear ms systems is the transgenic male sterility system, for which early developments were made in the beginning of 1990's (Mariani, 1992). These systems have become possible because of the isolation, cloning and characterization of anther or pollen specific genes and promoter sequences (Williams et al., 1997). However, transgenic systems have to undergo a lengthy and costly governmental approval process, which puts those systems into a significant competitive disadvantage in comparison to other systems.

**Environmental sensitive male sterility (enms) systems:** Certain nms lines in plants are conditional mutants, meaning thereby that in a particular environment male sterile mutant plants turn into male fertile. After determination of the critical environment (usually temperature or photoperiod) for sterility and fertility expression, such GMS mutants are classified as environmental sensitive nuclear male sterile (enms) lines. In vegetable crops, mostly temperature sensitive enms lines have been reported (Table 1). From a practical application viewpoint, it is necessary to identify the critical temperature or photoperiod for the fertility/sterility expression in temperature and photoperiod sensitive genetic male sterility systems, respectively.

**Table 1: Environmental sensitive male sterility mutants in vegetables**

| | | |
|---|---|---|
| | | |

| Vegetable | Mutant | Reference |
|---|---|---|
| Cabbage | TGMS, PGMS | Rundfeldt, 1961 |
| Brussels sprout | TGMS | Nieuwhof, 1968 |
| Broccoli | TGMS | Dickson, 1970 |
| Pepper | TGMS, TCMS | Daskalov, 1972; Shifriss, 1997* |
| Carrot | TGMS | Kaul, 1988 |
| Tomato | TGMS | Rick, 1948; Sawhney, 1983 |
| TGMS-Thermo sensitive genic male sterility | | |
| PGMS-Photoperiod sensitive genic male sterility | | |
| *TCMS-Thermo sensitive cytoplasmic male sterility | | |
| Table from Kumar et al., 2004 | | |

Hybrid seed production using enms lines is more attractive because of the ease in seed multiplication of male sterile line. Seeds of enms lines can be multiplied in an environment where it expresses the male fertility trait, while hybrid seeds can be produced in another environment, where it expresses male sterility.

**NPZ ("Norddeutsche Pflanzenzucht Lembke") MSL system:** The MSL (Male Sterility Lembke) system is a system provided by NPZ/Lembke, that is currently commercialized in Europe (Pinochet et al., 2000). In 2006, hybrids produced with this system covered an area of about 1.15 million ha in Europe (therefrom 850,000 ha in Germany (Frauen et al., 2007)). The MSL system is claimed to be based on a spontaneous mutation and selection in the NPZ breeding station in 1984 (Frauen, 1999; Paulmann & Frauen, 1999). In Germany, the first restored MSL hybrid varieties were approved in 1996 (Frauen & Baer, 1996) and demonstrate an increased yield of approx. 12 % in comparison with elite conventional non-hybrid varieties. The system is described as an alternative CMS system (Frauen, 1999; "Hybridraps-flebel", Rapool promotion material), which uses a fertile maintainer line for the propagation of a sterile mother line to derive fully restored hybrids. All known conventional rapeseed cultivars and lines are restorer lines for this male sterility system. This and the claim that the system originates from a spontaneous mutation suggest that it is different from other known sterility systems. Neither the parental lines of the MSL system nor the method of the preparation of the hybrid seed are known to the public, but are kept as a trade secret. This limits the use of the MSL system and its applications to a broader spectrum of rapeseed varieties.

**MS Takagi:** Takagi (1970) induced in the Japanese variety 'Murasaki natane' male sterility by gamma-irradiation mutagenesis. This system was described by Takagi as monogen recessive. Thels (1990) describes in his Ph.D. thesis (p.14-18) that MS Takagi is controlled by two genes, one of which is a homozygotic recessive male sterility gene and one of which is a dominant "modifier gene". Theis describes that MS Takagi is sterile under normal field conditions, but may revert to fertility under a one week temperature treatment (38°C day temperature / 18°C night temperature) after 7 to 10 days (p. 49). Theis further proposes the production of a 100% fertile parental line by pollination of sterile plants with pollen of sterile, temperature treated plants (p.55). However, no commercial use of this system is known in the public and no commercially viable hybrid system thereof has been developed. Neither Takagi nor Theis describe homozygous lines for the sterility gene or the "modifier gene". When discussing nuclear male sterility systems Denis et al. (1993) describe with reference to the Takagi system that one reason for the difficulties related to this system is the absence of marker genes which does not permit the sorting of male sterile or male fertile plants in the progeny, which is required for providing homozygous lines (for detailed discussion see also below).

As mentioned above, only two hybrid systems are currently commercially employed: (1) The NPZ MSL system, for which the genetic is not known, and (2) the Ogura system, which because of certain agronomical disadvantages linked to the restorer allele is also not optimal. The further available InVigor™ hybrid canola system (Bayer CropScience) is a transgenic system and therefore linked to high regulatory costs and public concern. However, these few systems are limited in number. Thus, additional systems would be beneficial not only from economic perspective but also for agricultural reasons: Large use of a single hybrid system in a crop like rapeseed would render the population vulnerable against diseases which can more easily spread in uniform genetic populations. Utilization of several hybrid systems in parallel would allow for larger genetic variation and would reduce this vulnerability.

The problem to be solved by the present invention is therefore to provide an alternative commercially viable new nuclear male sterility hybrid system in rapeseed, which produces commercially viable rapeseed meal and oil and is a convenient and cost-efficient method to produce hybrid seed (preferably with an glucosinolate content of not more than 25 µmol per gram of air dry seed at 9% humidity), which further does not have the disadvantages of the currently used systems.

This problem is solved by the present invention.

### DESCRPTION OF THE DRAWINGS

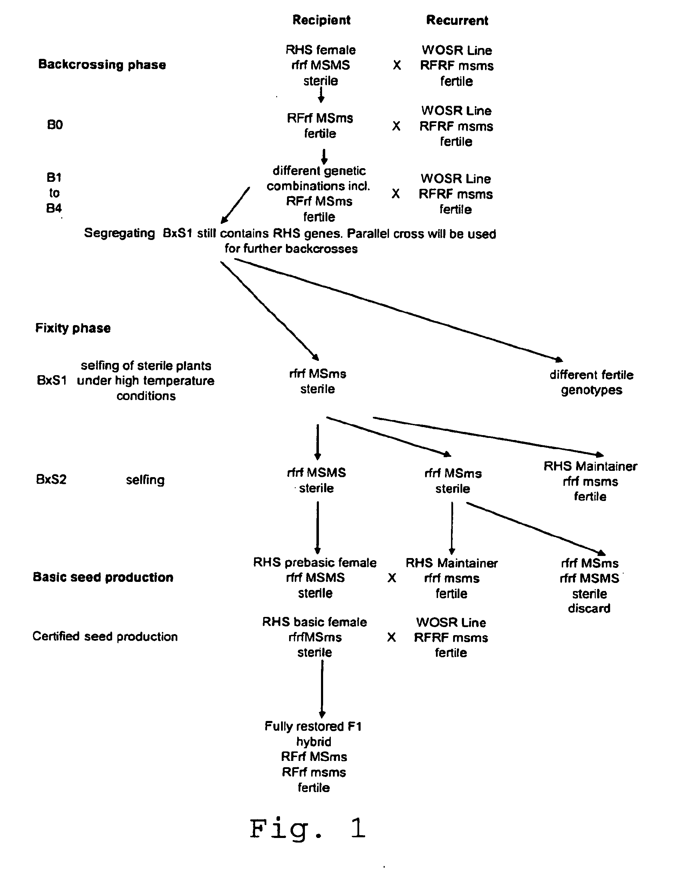

- **Fig. 1**:: Restored hybrid system (RHS) inheritance and variety development. Breeding scheme for the fixation of the Ms line and the maintainer line and the subsequent crossing scheme for the hybrid system. The upper part of the scheme describes the fixation of the MsMsrfrf genotype (prebasic female line) and the msmsrfrf genotype (maintainer line). The lower part describes the crossing scheme for providing hybrid seed by first providing a basic female line (Msmsrfrf) by crossing the prebasic fe-male line (MsMsrfrf) and the maintainer line (msmsrfrf), and then providing hybrid seed by crossing the basic female (Msmsrfrf) line with a restorer line (msmsRfRf).

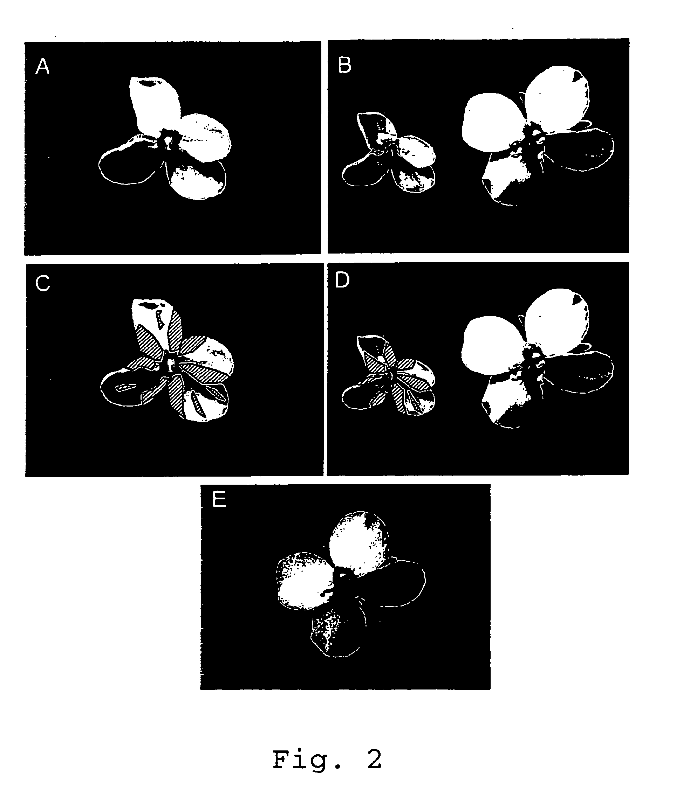

- **Fig. 2:**: Pictures demonstrating the white-striped/white-blotched phenotype of the condi-tionally male sterile plants (after temperature induced re-fertilization) in comparison to flowers of male fertile plants.
A: White-striped/white-blotched phenotype of the conditionally male sterile *Bras-sica napus* plant after high temperature Induced re-fertilization.
B: Comparison of white-striped/white-blotched flowers (male sterile *Brassica napus* plant; left flower) with normal flowers of male fertile *Brassica napus* plant (right flower).
C: Same as A, with white areas marked by overlaid hatched sections
D: Same as B, with white areas marked by overlaid hatched sections
E: Flower of male fertile *Brassica napus* plant.

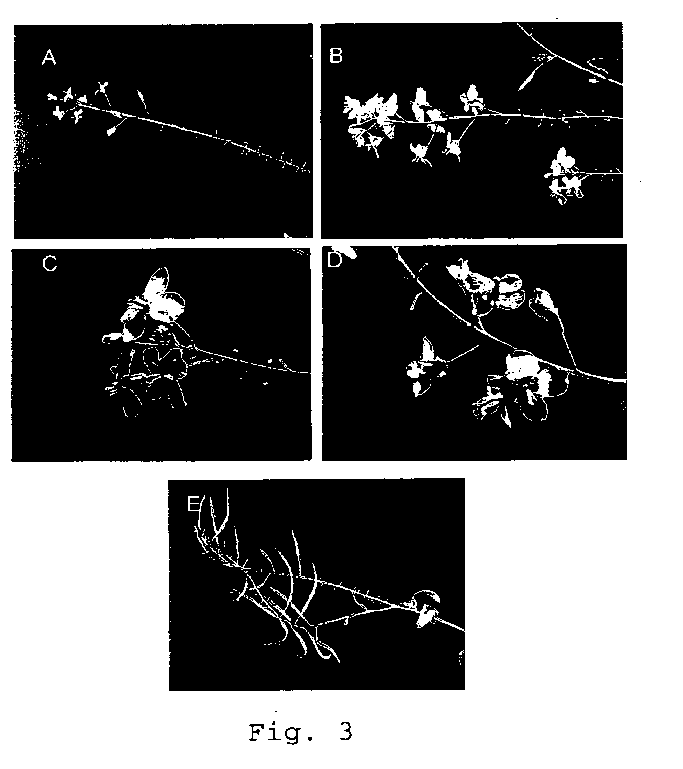

- **Fig. 3:**: Pictures demonstrating the bud abortion phenotype of the conditionally male sterile *Brassica napus* plants.
A: Sterile shoots of male sterile plants with bud abortion
B-D: Shoots of male sterile plants after heat treatment having sterile and fertile flowers on the same shoot
E: Shoots of male sterile plants with pods after heat-treatment fertilization and selfing
- **Fig. 4:**: A: Profile of a BSA candidate marker displaying a polymorphism linked to the steril-ity and then to the segregation of the Ms allele (alt: 1). The size of the fragment displayed in the male fertile bulks is the same as the one observed for the male fer-tile parent. Whereas, male sterile bulks displayed two fragments: the one observed for the male sterile parent and the one observed for the male fertile parent. This observation is consistent with the expectation, because male sterile plants can be both homozygous and heterozygous for the Ms allele.
B: Agarose profile of the amplification products obtained in male fertile (F) and male sterile (S) lines by the combination of primers HiNK6702 and 6707 (SEQ ID NOs: 13 and 14, respectively).
- **Fig. 5:**: Typical SNP plot obtained for marker SR0002A with either homozygous sterile (MsMs), heterozygous sterile (Msms), or homozygous fertile (msms) plants segre-gating in three different clouds according to the type of fluorescence transmission and its intensity.
- **Fig. 6:**: Marker Sequence NR1116. Underlined are forward and reverse primers forthe SSR amplification. The SSR itself is in bold letters. At the 5'-end of the sequence some nucleotides could not be clearly identified and are marked with "N" with may represent either A, T, C or G.
- **Fig. 7:**: Marker Sequence NR2525. Underlined are forward and reverse primers forthe SSR amplification. The SSR itself is in bold letters.

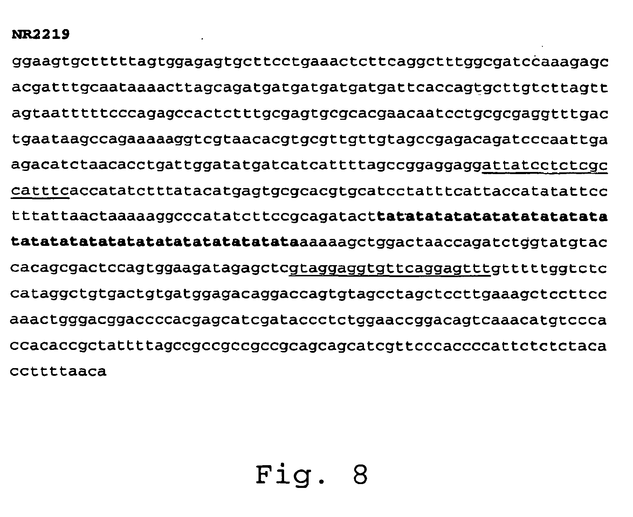

- **Fig. 8:**: Marker Sequence NR2219. Underlined are forward and reverse primers for the SSR amplification. The SSR itself is in bold letters.

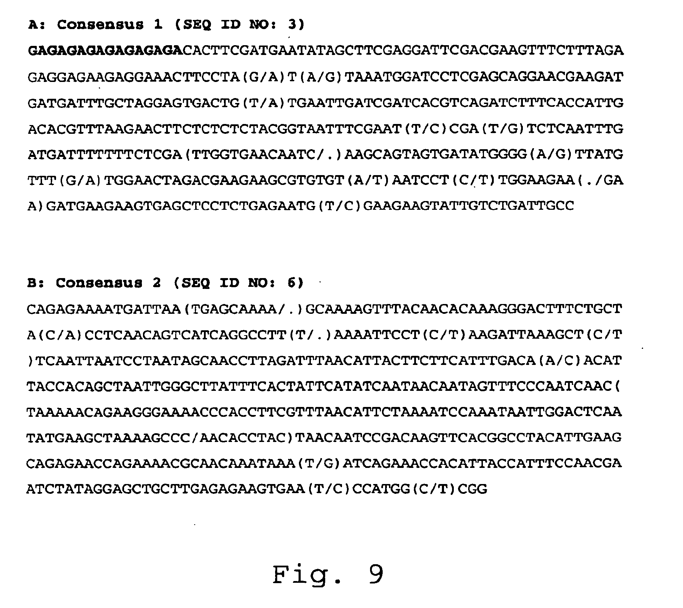

- **Fig. 9:**: A: SNP Consensus Sequence 1 (SEQ ID NO: 3)
B: SNP Consensus Sequence 2 (SEQ ID NO: 6)
Shown are the consensus sequences of the fertile and sterile alleles. Mutated areas are in (brackets) and present both the nucleotides for the fertile and the sterile haplotype (see examples for details). The bold letters at the 5'-end of Consensus Sequence 1 mark the 3'-end of the SSR repeat.

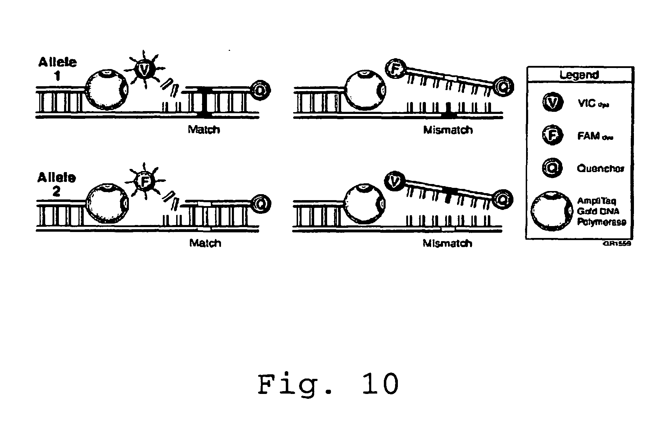

- **Fig. 10:**: TaqMan Assay basic principle (from: Pre-Developed TaqMan® Assay Reagents Allelic Discrimination Protocol; Part Number 4312214 Rev. C 5/2005; Applied Bio-systems)

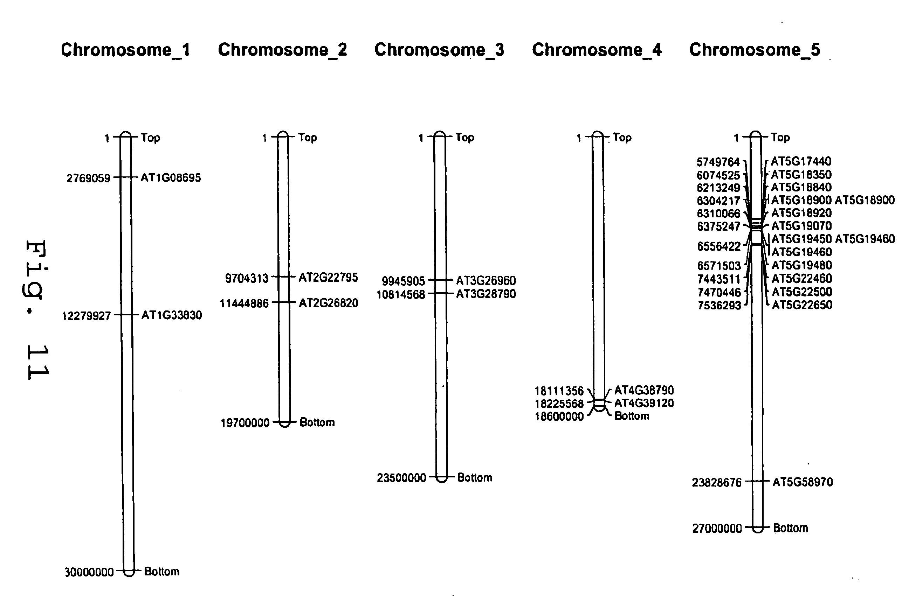

- **Fig. 11:**: Mapping position of the Arabidopsis homologues of 23 Brassica candidate genes across the five chromosomes of *Arabidopsis thaliana.* The physical distance ex-pressed in base pairs is specified on the left side of the bar, the reference ID of the *Arabidopsis thaliana* genes is specified on the right side of the bar.

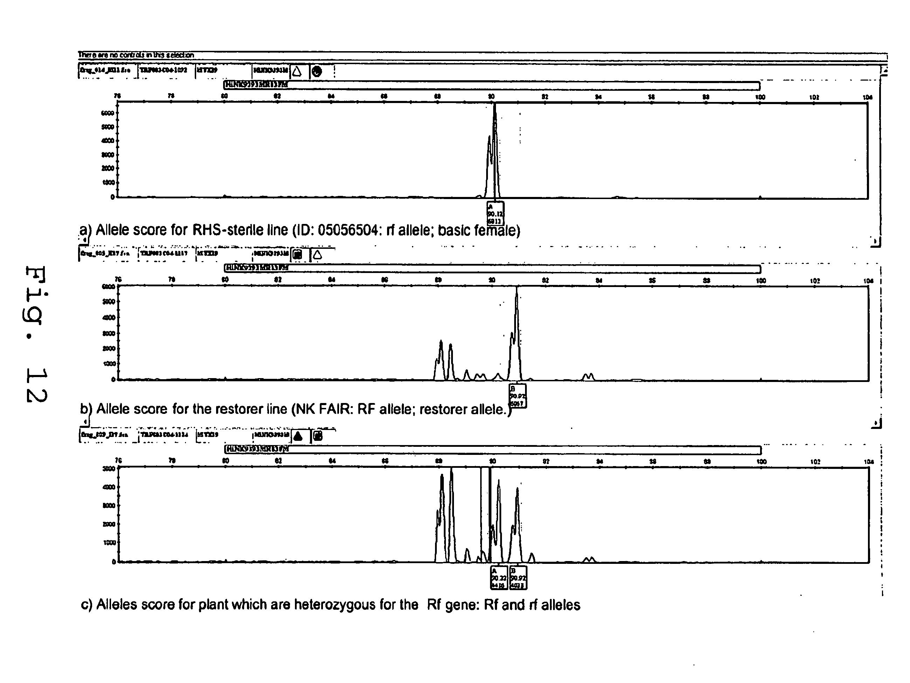

- **Fig. 12:**: GeneMapper output for the SSCP marker derived from PUT-161 a-Brassica_napus-59218 showing an example of a homozygous sterile (rfrf), homozygous fertile (RfRf) and heterozygous fertile (Rfrf) plant individual.

### DEFINITIONS

It is to be understood that this invention is not limited to the particular methodology, protocols, cell lines, plant species or genera, constructs, and reagents described herein as such. It is also to be understood that the terminology used herein is for the purpose of describing particular embodiments only, and is not intended to limit the scope of the present invention, which will be limited only by the appended claims. It must be noted that as used herein and in the appended claims, the singular forms "a," "and," and "the" include plural reference unless the context clearly dictates otherwise. Thus, for example, reference to "a plant" is a reference to one or more plants and includes equivalents thereof known to those skilled in the art, and so forth. As used herein, the word "or" means any one member of a particular list and also includes any combination of members of that list (i.e., includes also "and").
The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent, preferably 10 percent up or down (higher or lower). With regard to a temperature the term "about" means ± 1.0 °C, preferably ± 0.5°C. Where the term about is used in the context of this invention (e.g., in combinations with temperature or molecular weight values) the exact value (i.e., without "about") is preferred.
The term **"allele(s)"** means any of one or more alternative forms of a gene, all of which alleles relate to at least one trait or characteristic. In a diploid cell, the two alleles of a given gene occupy corresponding loci on a pair of homologous chromosomes. In some instances (e.g., for QTLs) it is more accurate to referto "haplotype" (i.e., an allele of a chromosomal segment) instead of "allele", however, in those instances, the term "allele" should be understood to comprise the term "haplotype". If two individuals possess the same allele at a particular locus, the alleles are termed "identical by descent" if the alleles were inherited from one common ancestor (*i.e.*, the alleles are copies of the same parental allele). The alternative is that the alleles are "identical by state" (*i.e.*, the alleles appear to be the same but are derived from two different copies of the allele). Identity by descent information is useful for linkage studies; both identity by descent and identity by state information can be used in association studies such as those described herein, although identity by descent information can be particularly useful.
The term **"backcrossing"** is understood within the scope of the invention to refer to a process in which a hybrid progeny is repeatedly crossed back to one of the parents.
The term ***"Brassica"**,* means the genus Brassica, very particularly the species *napus* (oilseed rape), *campestris* (beet), *oleracea* cv Tastie (cabbage), *oleracea* cv Snowball Y (cauliflower) and *oleracea* cv Emperor (broccoli).
The term ***"Brassica napus"*** or **"oilseed rape"** means plants, seeds, plant parts, and cells of *Brassica napus,* and comprises the annual spring type, the biannual winter type, and the biannual intermediate type oilseed rape. Annual or biannual in this context indicates whether the variety is grown over the vegetative winter period. Generally, winter-type rapeseed is grown in North Western Europe, whereas spring-types are mainly grown in Canada, China, India, Australia and South America. Oilseed rape is derived from interspecific hybridization of *B. oleracea* and *B. campestris.* In consequence the term also comprises any re-synthesis conducted from these two species. One preferred spring-type oilseed rape is canola-type rapeseed. Representative winter rape varieties that include the genetic means for the expression of low glucosinolate content and that are commercially available in Europe include, for example, CAPITOL, cv. CAMPALA, cv. CALIFORNIUM (available from Dekalb, brand of Monsanto), cv. LORENZ, cv. OASE (available from RAPOOL). Representative spring rape varieties that include the genetic means for the expression of low glucosinolate content and that are commercially available in Canada include, for example, cv. BULLET, cv. GARRISON and cv. KRISTANA (each available from Svalof Weibull). Other winter rape varieties that include the genetic means for the expression of low glucosinolate content and that are commercially available in Europe include cv. APEX, cv. NK FAIR, cv. VIKING, cv. BILLY, cv. LADOGA, and cv. CASTILLE. Such low levels of glucosinolates in the oilseed Brassica serve to impart increased commercial value to the meal.
The term ***"Brassica* nopus-specific DNA sequence"** indicates a polynucleotide sequence having a nucleotide sequence homology of more than 80%, preferably more than 85%, more preferably more than 90%, even more preferably more than 95%, still more preferably more than 97%, most preferably more than 99% with a sequence of the genome of the species *Brassica napus* (or any of the two species *Brassica napus* was generated (synthesized) from, namely *Brassica* rapa and *Brassica oleracea)* that shows the greatest similarity to it.
The term **"Canola"** means a *Brassica* napusyielding oil that contains less than 2% erucic acid, and the solid component of the seed must contain less than 30 micromoles of any one or any mixture of 3-butenyl glucosinolate, 4-pentenyl glucosinolate, 2-hydroxy-3 butenyl glucosinolate, and 2-hydroxy-4-pentenyl glucosinolate per gram of air-dry, oil-free solid.
The term **"chromosome"** is used herein as recognized in the art as meaning the self-replicating genetic structure in the cellular nucleus containing the cellular DNA and bearing in its nucleotide sequence the linear array of genes.
The term **"conditionally male sterile"** means a phenotype of male sterility (i.e., an incapability to produce fertile pollen), which can be induced and/or repressed by certain conditions. In consequence, a plant can be "switched" from a male sterile to a male fertile phenotype by applying said certain conditions. Male sterility can be caused by various factors and can be expressed for example as a complete lack of male organs (anthers), degenerated pollen, infertile pollen etc. Based on the intensity of the condition the "switch" from male sterility to male fertility may be complete or incomplete. Most preferably, in the context of the present invention the term "conditionally male sterile" means a temperature-dependent male sterility and thereby means a nuclear male sterile phenotype, wherein the sterility is temperature dependent and can be reverted to fertility at a temperature of more than 35°C (preferably between 35°C and 43°C, more preferably between 37°C and 40°C, most preferably at about 39°C; preferably with an exposure for the preferred heat treatment time as specified herein and a subsequent growing at ambient temperature, as defined herein).
A **"gene"** is defined herein as a hereditary unit consisting of a sequence of DNA that occupies a specific location on a chromosome and that contains the genetic instruction for a particular characteristic or trait in an organism.
**"Genetic engineering"**, **"transformation"** "and" **genetic modification"** are all used herein as synonyms forthe transfer of isolated and cloned genes into the DNA, usually the chromosomal DNA or genome, of another organism.
The term **"genotype"** refers to the genetic constitution of a cell or organism. An individual's "genotype for a set of genetic markers" includes the specific alleles, for one or more genetic marker loci, present in the individual. As is known in the art, a genotype can relate to a single locus or to multiple loci, whether the loci are related or unrelated and/or are linked or unlinked. In some embodiments, an individual's genotype relates to one or more genes that are related in that the one or more of the genes are involved in the expression of a phenotype of interest (e.g., a quantitative trait as defined herein). Thus, in some embodiments a genotype comprises a sum of one or more alleles present within an individual at one or more genetic loci of a quantitative trait. In some embodiments, a genotype is expressed in terms of a haplotype (defined herein below).
The term **"germplasm"** refers to the totality of the genotypes of a population or another group of individuals (e.g., a species). The term "germplasm" can also refer to plant material; e.g., a group of plants that act as a repository for various alleles. The phrase "adapted germplasm" refers to plant materials of proven genetic superiority; e.g., for a given environment or geographical area, while the phrases "non-adapted germplasm", "raw germplasm", and "exotic germplasm" refer to plant materials of unknown or unproven genetic value; e.g., for a given environment or geographical area; as such, the phrase "non-adapted germplasm" refers in some embodiments to plant materials that are not part of an established breeding population and that do not have a known relationship to a member of the established breeding population.
The term **"glucosinolates"** means sulfur-based compounds that remain in the solid component of the seed - the solid meal - after the seed has been ground and its oil has been extracted. Their structure includes glucose in combination with aliphatic hydrocarbons (3-butenyl glucosinolate, 4-pentenyl glucosinolate, 2-hydroxy-3-butenyl glucosinolate, and 2-hydroxy-4-pentenyl glucosinolate) or aromatic hydrocarbons (3-indoylmethyl glucosinolate,1-methoxy-3-indoyl methyl glucosinolate). Aliphatic glucosinolates are also known as alkenyl glucosinolates. Aromatic glucosinolates are also known as indoles. The term **"total glucosinolate content"** means the sum of all glucosinolates comprised in the indicated material e.g., in the meal or the dry seed. Total glucosinolate content can be indicated in µmol (glucosinolates) per gram of seed (or air dry seed at, for example, 9% humidity) or meal.
The term **"haplotype"** refers to the set of alleles an individual inherited from one parent. A diploid individual thus has two haplotypes. The term "haplotype" can be used in a more limited sense to refer to physically linked and/or unlinked genetic markers (e.g., sequence polymorphisms) associated with a phenotypic trait. The phrase "haplotype block" (sometimes also referred to in the literature simply as a haplotype) refers to a group of two or more genetic markers that are physically linked on a single chromosome (or a portion thereof). Typically, each block has a few common haplotypes, and a subset of the genetic markers (*i.e.*, a "haplotype tag") can be chosen that uniquely identifies each of these haplotypes.
The terms **"homology"**, **"sequence similarity"** or **"sequence identity"** of nucleotide or amino acid sequences mean a degree of identity or similarity of two or more sequences and may be determined conventionally by using known software or computer programs such as the BestFit or Gap pairwise comparison programs (GCG Wisconsin Package, Genetics Computer Group, 575 Science Drive, Madison, Wis. 53711). BestFit uses the local homology algorithm of Smith and Waterman, Advances in Applied Mathematics 2:482-489 (1981), to find the best segment of identity or similarity between two sequences. Sequence comparison between two or more polynucleotides or polyaminoacid sequences is generally performed by comparing portions of the two sequences over a comparison window to identify and compare local regions of sequence similarity. The comparison window is generally from about 20 to 200 contiguous nucleotides. Gap performs global alignments: all of one sequence with all of another similar sequence using the method of Needleman and Wunsch, J. Mol. Biol. 48:443-453 (1970). When using a sequence alignment program such as BestFit to determine the degree of DNA sequence homology, similarity or identity, the default setting may be used, or an appropriate scoring matrix may be selected to optimize identity, similarity or homology scores. Similarly, when using a program such as BestFit to determine sequence identity, similarity or homology between two different amino acid sequences, the default settings may be used, or an appropriate scoring matrix, such as blosum45 or blosum80, may be selected to optimize identity, similarity or homology scores.
**"Homologous recombination"** is the exchange ("crossing over") of DNA fragments between two DNA molecules or chromatids of paired chromosomes in a region of identical nucleotide sequences. A "recombination event" is herein understood to mean a meiotic crossing-over.
The term **"heterozygous"** means a genetic condition existing when different alleles reside at corresponding loci on homologous chromosomes.
The term **"homozygous"** means a genetic condition existing when identical alleles reside at corresponding loci on homologous chromosomes.
The terms "**hybrid"**, **"hybrid plant"**, and **"hybrid progeny"** in the context of plant breeding refer to a plant that is the offspring of genetically dissimilar parents produced by crossing plants of different lines or breeds or species, including but not limited to the cross between two inbred lines (e.g., a genetically heterozygous or mostly heterozygous individual). The phrase "single cross F₁ hybrid" refers to an F₁ hybrid produced from a cross between two inbred lines.
The term **"hybrid"** in the context of nucleic acids refers to a double-stranded nucleic acid molecule, or duplex, formed by hydrogen bonding between complementary nucleotide bases. The terms "hybridise" or "anneal" refer to the process by which single strands of nucleic acid sequences form double-helical segments through hydrogen bonding between complementary bases.
The phrase **"inbred line"** refers to a genetically homozygous or nearly homozygous population. An inbred line, for example, can be derived through several cycles of brother/sister breedings or of selfing. In some embodiments, inbred lines breed true for one or more phenotypic traits of interest. An "inbred", "inbred individual", or "inbred progeny" is an individual sampled from an inbred line. The term "inbred" means a substantially homozygous individual or line.
The terms **"introgression"**, **"introgressed"** and **"introgressing"** refer to both a natural and artificial process whereby genomic regions of one species, variety or cultivar are moved into the genome of another species, variety or cultivar, by crossing those species. The process may optionally be completed by backcrossing to the recurrent parent.
The term **"linkage"**, and grammatical variants thereof, refers to the tendency of alleles at different loci on the same chromosome to segregate together more often than would be expected by chance if their transmission were independent, in some embodiments as a consequence of their physical proximity.
The phrase **"linkage disequilibrium"** (also called "allelic association") refers to a phenomenon wherein particular alleles at two or more loci tend to remain together in linkage groups when segregating from parents to offspring with a greater frequency than expected from their individual frequencies in a given population. For example, a genetic marker allele and a QTL allele can show linkage disequilibrium when they occur together with frequencies greater than those predicted from the individual allele frequencies. Linkage disequilibrium can occur for several reasons including, but not limited to the alleles being in close proximity on a chromosome
The term **"linkage group"** refers to all of the genes or genetic traits that are located on the same chromosome. Within the linkage group, those loci that are close enough together will exhibit linkage in genetic crosses. Since the probability of crossover increases with the physical distance between genes on a chromosome, genes whose locations are far removed from each other within a linkage group may not exhibit any detectable linkage in direct genetic tests. The term "linkage group" is mostly used to refer to genetic loci that exhibit linked behavior in genetic systems where chromosomal assignments have not yet been made. Thus, in the present context, the term "linkage group" is synonymous to (the physical entity of) chromosome.
The term **"locus"** refers to a position (e.g., of a gene, a genetic marker, or the like) on a chromosome of a given species. The terms **"molecular marker"** or **"genetic marker"** refer to an indicator that is used in methods for visualizing differences in characteristics of nucleic acid sequences. It refers to a feature of an individuals genome (e.g., a nucleotide or a polynucleotide sequence that is present in an individuals genome) that Is associated with one or more loci of interest. In some embodiments, a genetic marker is polymorphic in a population of interest or the locus occupied by the polymorphism, depending on the context. Genetic markers include, for example, single nucleotide polymorphisms (SNPs), indels (i.e., insertions/deletions), simple sequence repeats (also named microsatellite markers; SSRs), restriction fragment length polymorphisms (RFLPs), random amplified polymorphic DNAs (RAPDs), cleaved amplified polymorphicsequence (CAPS) markers, Diversity Arrays Technology (DArT) markers, and amplified fragment length polymorphisms (AFLPs), among many other examples. Additional markers include insertion mutations, sequence-characterized amplified regions (SCARs), or isozyme markers or combinations of the markers described herein which defines a specific genetic and chromosomal location. Genetic markers can, for example, be used to locate genetic loci containing alleles that contribute to variability in expression of phenotypic traits on a chromosome. The phrase "genetic marker" can also refer to a polynucleotide sequence complementary to a genomic sequence, such as a sequence of a nucleic acid used as probes. A genetic marker can be physically located in a position on a chromosome that is within or outside of to the genetic locus with which it is associated (*i.e.*, is intragenic or extragenic, respectively). Stated another way, whereas genetic markers are typically employed when the location on a chromosome of the gene that corresponds to the locus of interest has not been identified and there is a non-zero rate of recombination between the genetic marker and the locus of interest, the presently disclosed subject matter can also employ genetic markers that are physically within the boundaries of a genetic locus (e.g., inside a genomic sequence that corresponds to a gene such as, but not limited to a polymorphism within an intron or an exon of a gene). In some embodiments of the present invention, the one or more genetic markers comprise between one and ten markers, and in some embodiments the one or more genetic markers comprise more than ten genetic markers.
The term **"Marker-based selection"** is understood within the scope of the invention to referto the use of genetic markers to detect one or more nucleic acids from the plant, where the nucleic acid is associated with a desired trait to identify plants that carry genes for desirable (or undesirable) traits, so that those plants can be used (or avoided) in a selective breeding program.
The term **"microsatellite or SSRs (simple sequence repeats) marker"** is understood within the scope of the invention to refer to a type of genetic marker that consists of numerous repeats of short sequences of DNA bases, which are found at loci throughout the plant's DNA and have a likelihood of being highly polymorphic.
The phrase **"nucleic acid"** refers to any physical string of monomer units that can be corresponded to a string of nucleotides, including a polymer of nucleotides (e.g., a typical DNA or RNA polymer), modified oligonucleotides (e.g., oligonucleotides comprising bases that are not typical to biological RNA or DNA, such as 2'-O-methylated oligonucleotides), and the like. In some embodiments, a nucleic acid can be single-stranded, double-stranded, multi-stranded, or combinations thereof. Unless otherwise indicated, a particular nucleic acid sequence of the present invention optionally comprises or encodes complementary sequences, in addition to any sequence explicitly indicated.
The phrase **"phenotypic trait"** refers to the appearance or other detectable characteristic of an individual, resulting from the interaction of its genome with the environment.
The term **"plurality"** refers to more than one entity. Thus, a "plurality of individuals" refers to at least two individuals. In some embodiments, the term plurality refers to more than half of the whole. For example, in some embodiments a "plurality of a population" refers to more than half the members of that population.
The term **"progeny"** refers to the descendant(s) of a particular cross. Typically, progeny result from breeding of two individuals, although some species (particularly some plants and hermaphroditic animals) can be selfed (*i.e.*, the same plant acts as the donor of both male and female gametes). The descendant(s) can be, for example, of the F₁, the F₂, or any subsequent generation.
The phrase **"qualitative trait"** refers to a phenotypic trait that is controlled by one or a few genes that exhibit major phenotypic effects. Because of this, qualitative traits are typically simply inherited. Examples in plants include, but are not limited to, flower color, cob color, and disease resistance such as for example Northern com leaf blight resistance. **"PCR (polymerase chain reaction)"** is understood within the scope of the invention to refer to a method of producing relatively large amounts of specific regions of DNA, thereby making possible various analyses that are based on those regions.
**"phenotype"** is understood within the scope of the invention to refer to a distinguishable characteristic(s) of a genetically controlled trait. A **"plant"** is any plant at any stage of development, particularly a seed plant.
A **"plant cell"** is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant.
**"Plant cell culture"** means cultures of plant units such as, for example, protoplasts, cell culture cells, cells in plant tissues, pollen, pollen tubes, ovules, embryo sacs, zygotes and embryos at various stages of development.
**"Plant material"** refers to leaves, stems, roots, flowers or flower parts, fruits, pollen, egg cells, zygotes, seeds, cuttings, cell or tissue cultures, or any other part or product of a plant.
**A "plant organ"** is a distinct and visibly structured and differentiated part of a plant such as a root, stem, leaf, flower bud, or embryo.
**"Plant tissue"** as used herein means a group of plant cells organized into a structural and functional unit. Any tissue of a plant *in planta* or in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue.
The term **"plant part"** indicates a part of a plant, including single cells and cell tissues such as plant cells that are intact in plants, cell clumps and tissue cultures from which plants can be regenerated. Examples of plant parts include, but are not limited to, single cells and tissues from pollen, ovules, leaves, embryos, roots, root tips, anthers, flowers, fruits, stems shoots, and seeds; as well as pollen, ovules, leaves, embryos, roots, root tips, anthers, flowers, fruits, stems, shoots, scions, rootstocks, seeds, protoplasts, calli, and the like.
**"polymorphism"** is understood within the scope of the invention to refer to the presence in a population of two or more different forms of a gene, genetic marker, or inherited trait.
The term **"population"** means a genetically heterogeneous collection of plants sharing a common genetic derivation.
The term **"predominately male sterile"** means that in a population of at least 100 plants not more than 10 %, preferably not more than 5 %, more preferably not more than 1 % of the flowers on all of those plants have functional male organs producing fertile pollen. It has to be understood that an individual plant can have both fertile and sterile flowers. In preferred embodiments not more than 10 %, preferably not more than 5 %, more preferably not more than 1 % of the flowers on an individual plant have functional male organs producing fertile pollen.
The term **"probe"** refers to a single-stranded oligonucleotide sequence that will form a hydrogen-bonded duplex with a complementary sequence in a target nucleic acid sequence analyte or its cDNA derivative.
The term **"primer",** as used herein, refers to an oligonucleotide which is capable of annealing to the amplification target allowing a DNA polymerase to attach, thereby serving as a point of initiation of DNA synthesis when placed under conditions In which synthesis of primer extension product is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxyribonucleotide. The primer must be sufficiently long to prime the synthesis of extension products in the presence of the agent for polymerization. The exact lengths of the primers will depend on many factors, including temperature and composition (A/T and G/C content) of primer. A pair of bi-directional primers consists of one forward and one reverse primer as commonly used in the art of DNA amplification such as in PCR amplification. It will be understood that "primer", as used herein, may refer to more than one primer, particularly in the case where there is some ambiguity in the information regarding the terminal sequence(s) of the target region to be amplified. Hence, a "primer" includes a collection of primer oligonucleotides containing sequences representing the possible variations in the sequence or includes nucleotides which allow a typical base pairing. The oligonucleotide primers may be prepared by any suitable method. Methods for preparing oligonucleotides of specific sequence are known in the art, and include, for example, cloning and restriction of appropriate sequences, and direct chemical synthesis. Chemical synthesis methods may include, for example, the phospho di- or tri-ester method, the diethylphosphoramidate method and the solid support method disclosed in, for example, US 4,458,066. The primers may be labeled, if desired, by incorporating means detectable by, for instance, spectroscopic, fluorescence, photochemical, biochemical, immunochemical, orchemical means. Template-dependent extension of the oligonucleotide primer(s) is catalyzed by a polymerizing agent in the presence of adequate amounts of the four deoxyribonucleotide triphosphates (dATP, dGTP, dCTP and dTTP, i.e. dNTPs) or analogues, in a reaction medium which is comprised of the appropriate salts, metal cations, and pH buffering system. Suitable polymerizing agents are enzymes known to catalyze primer- and template-dependent DNA synthesis. Known DNA polymerases include, for example, E. coli DNA polymerase I or its Klenow fragment, T4 DNA polymerase, and Taq DNA polymerase. The reaction conditions for catalyzing DNA synthesis with these DNA polymerases are known in the art. The products of the synthesis are duplex molecules consisting of the template strands and the primer extension strands, which include the target sequence. These products, in turn, serve as template for another round of replication. In the second round of replication, the primer extension strand of the first cycle is annealed with its complementary primer; synthesis yields a "short" product which is bound on both the 5'- and the 3'-ends by primer sequences or their complements. Repeated cycles of denaturation, primer annealing, and extension result in the exponential accumulation of the target region defined by the primers. Sufficient cycles are run to achieve the desired amount of polynucleotide containing the target region of nucleic acid. The desired amount may vary, and is determined by the function which the product polynucleotide is to serve. The PCR method is well described in handbooks and known to the skilled person. After amplification by PCR, the target polynucleotides may be detected by hybridization with a probe polynucleotide which forms a stable hybrid with that of the target sequence under stringent to moderately stringent hybridization and wash conditions. If it is expected that the probes will be essentially completely complementary (i.e., about 99% or greater) to the target sequence, stringent conditions will be used. If some mismatching is expected, for example if variant strains are expected with the result that the probe will not be completely complementary, the stringency of hybridization may be lessened. However, conditions are chosen which rule out nonspecific/adventitious binding. Conditions, which affect hybridization, and which select against nonspecific binding are known in the art, and are described in, for example, Sambrook and Russell, 2001. Generally, lower salt concentration and higher temperature increase the stringency of hybridization conditions. "PCR primer" is preferably understood within the scope of the present invention to refer to relatively short fragments of single-stranded DNA used in the PCR amplification of specific regions of DNA.
The term **"offspring"** plant refers to any plant resulting as progeny from a vegetative or sexual reproduction from one or more parent plants or descendants thereof. For instance, an offspring plant may be obtained by cloning or selfing of a parent plant or by crossing two parent plants and include selfings as well as the F₁ or F₂ or still further generations. An F₁ is a first-generation offspring produced from parents at least one of which is used for the first time as donor of a trait, while offsprings of second generation (F₂) or subsequent generations (F₃, F₄, etc.) are specimens produced from selfings of F₁'s, F₂ etc. An F₁ may thus be (and usually is) a hybrid resulting from a cross between two true breeding parents (true-breeding is homozygous for a trait), while an F₂ may be (and usually is) an offspring resulting from self-pollination of said F₁ hybrids.
**"Recombination"** is the exchange of information between two homologous chromosomes during meiosis. The frequency of double recombination is the product of the frequencies of the single recombinants. For instance, a recombinant in a 10 cM area can be found with a frequency of 10%, and double recombinants are found with a frequency of 10% x 10% = 1 % (1 centimorgan is defined as 1% recombinant progeny in a testcross).
The term **"RHS"** or **"restored hybrid system"** means the nuclear male sterility based hybrid system of this invention. The phrases **"sexually crossed"** and **"sexual reproduction"** in the context of the present invention refer to the fusion of gametes to produce progeny (e.g., by fertilization, such as to produce seed by pollination in plants). In some embodiments, a "sexual cross" or "cross-fertilization" is fertilization of one individual by another (e.g., cross-pollination in plants). In some embodiments the term "selfing" refers to the production of seed by self-fertilization or self-pollination; *i.e.*, pollen and ovule are from the same plant.
**"Selective breeding"** is understood within the scope of the present invention to refer to a program of breeding that uses plants that possess or display desirable traits as parents.
The terms **"stringent conditions"** or **"stringent hybridization conditions"** include reference to conditions under which a polynucleotide will hybridize to its target sequence to a detectably greater degree than other sequences (e.g., at least 2-fold over background). Stringent conditions are sequence-dependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lowerdegrees of similarity are detected (heterologous probing). Typically, stringent conditions will be those in which the salt concentration is less than approximately 1.5 M Na ion, typically about 0.01 to 1.0 M Na ion (or other salts) at pH 7.0 to 8.3 and the temperature is at least about 30° C for short probes (e.g., 10 to 50 nucleotides) and at least about 60° C for long probes (e.g., greater than 50 nucleotides). Stringent conditions also may be achieved with the addition of destabilizing agents such as formamide. Exemplary low stringency conditions include hybridization with a buffer solution of 30 to 35% formamide, 1 M NaCl, 1% SDS (w/v; sodium dodecyl sulphate) at 37° C, and a wash in 1 × to 2×SSC (20×SSC = 3.0 M NaCl/0.3 M trisodium citrate) at 50 to 55° C. Exemplary moderate stringency conditions include hybridization in 40 to 45% formamide, 1 M NaCl, 1% SDS at 37° C, and a wash in 0.5× to 1 ×SSC at 55 to 60° C. Exemplary high stringency conditions include hybridization in 50% formamide, 1 M NaCl, 1% SDS at 37°C, and a wash in 0.1 ×SSC at 60 to 65° C. Specificity is typically the function of post-hybridization washes, the critical factors being the ionic strength and temperature of the final wash solution. For DNA-DNA hybrids, the Tₘ can be approximated from the equation of Meinkoth and Wahl (Anal. Biochem., 138:267-284, 1984): Tₘ=81.5° C+16.6 (log M)+0.41 (% GC)-0.61 (% form)-500/L; where M is the molarity of monovalent cations, % GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1 % of mismatching; thus, Tₘ, hybridization and/or wash conditions can be adjusted to hybridize to sequences of the desired identity. For example, if sequences with approximately 90% identity are sought, the Tₘ can be decreased 10° C. Generally, stringent conditions are selected to be about 5° C lower than the thermal melting point (Tₘ) for the specific sequence and its complement at a defined ionic strength and pH. However, severely stringent conditions can utilize hybridization and/or wash at 1, 2, 3, or 4° C lower than the thermal melting point (Tₘ); moderately stringent conditions can utilize a hybridization and/or wash at 6, 7, 8, 9, or 10° C lower than the thermal melting point (Tₘ); low stringency conditions can utilize a hybridization and/or wash at 11, 12, 13, 14, 15, or 20° C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45° C (aqueous solution) or 32° C (formamide solution), it is preferred to increase the SSC concentration so that a higher temperature can be used. An extensive guide to the hybridization of nucleic acids is found in Tijssen, Laboratory Techniques in Biochemistry and Molecular Biology - Hybridization with Nucleic Acid Probes, Part 1, Chapter 2 "Overview of principles of hybridization and the strategy of nucleic acid probe assays", Elsevier, N.Y. (1993); and Current Protocols in Molecular Biology, Chapter 2, Ausubel, et al., eds., Greene Publishing and Wiley-Interscience, New York (1995). Methods of stringent hybridization are known in the art which conditions can be calculated by means known in the art. This is disclosed in Sambrook et al., Molecular Cloning: A Laboratory Manual, 2nd Ed., Cold Spring Harbor Laboratory Press, 1989, Cold Spring Harbor, N.Y. and Current Protocols in Molecular Biology, Ausebel et al, eds., John Wiley and Sons, Inc., 2000. Methods of determining percent sequence identity are known in the art, an example of which is the GCG computer sequence analysis software (GCG, Inc, Madison Wis.).
**"Tester plant"** is understood within the scope of the present invention to refer to a plant used to characterize genetically a trait in a plant to be tested. Typically, the plant to be tested is crossed with a "tester" plant and the segregation ratio of the trait in the progeny of the cross is scored.
The term **"tester"** refers to a line or individual with a standard genotype, known characteristics, and established performance. A "tester parent" is an individual from a tester line that is used as a parent in a sexual cross. Typically, the tester parent is unrelated to and genetically different from the individual to which it is crossed. A tester is typically used to generate F₁ progeny when crossed to individuals or inbred lines for phenotypic evaluation.
The phrase **"topcross combination"** refers to the process of crossing a single tester line to multiple lines. The purpose of producing such crosses is to determine phenotypic performance of hybrid progeny; that is, to evaluate the ability of each of the multiple lines to produce desirable phenotypes in hybrid progeny derived from the line by the tester cross. The terms **"variety"** or **"cultivar"** mean a group of similar plants that by structural or genetic features and/or performance can be distinguished from other varieties within the same species.

### DETAILED DESCRIPTION OF THE INVENTION

The invention provides a commercially viable system for the production of hybrid seed of *Brassica napus.* This system employs a dominant nuclear male sterility gene. Fertility can be restored by a dominant restorer allele. The inventive contributions comprise but are not limited to:
1. Providing a conditionally nuclear male sterile line, which comprises the male sterility gene in a homozygous form (genotype MsMsrfrf). The provision of this line is possible by the provision of a genetic marker for the male sterility gene as part of this invention.
2. Providing a method to propagate the conditionally male sterile line in an unchanged form by inducing fertility via a specific heat-treatment.
3. Providing a *Brassica napus* maintainer line, which neither comprises the nuclear male sterilitygene nora functional copy of the restorer allele (genotype msmsrfrf).

Since propagation of the female line in all hybrid systems is the major quantity limiting and costly step, the current system provides the female line in a 2-step procedure: First, it was found that the present system is an environmental sensitive nuclear male sterility (enms) system. Although most of these systems are commencially not viable, because sterility can be accidentally lost under field growing conditions, the current system can be switched on and off by a high temperature treatment, which Is unlikely to occur under conditions where rapeseed in normally grown. Utilization of this system provides sufficient amounts of the prebasic female by an inventive selfing procedure utilizing the enms properties.

In a second step the prebasic female is crossed with the maintainer line (msmsrfrf) to yield a male sterile basic female comprising the male sterility gene in a heterozygous form (genotype Msmsrfrf).

The term **"prebasic female",** as used herein, refers, for example, to a conditionally (e.g., high temperature modulated) male sterile *Brassica napus* line with the genotype MsMsrfrf, which genotype is obtainable for example from the *Brassica* napusseed deposited under Deposit Number NCIMB 41480 (cf. section 1, "Method of providing the prebasic female plant and seed" below) wherein said female *Brassica napus* plant is
i. homozygous for the male sterility allele (Ms allele) obtainable from the *Brassica napus* seed deposited under Deposit Number NCIMB 41480,
ii. homozygous for the maintainer allele (rf allele) obtainable from the *Brassica napus* seed deposited under Deposit Number NCIMB 41480 or 41481,
iii. predominantly male sterile when exposed before and/or during flowering to a temperature of less than 28°C, and
iv. reverting to a predominantly male fertile phenotype when exposed before and/or during flowering to a temperature of higher than 35°C.

Further, the term **"basic female",** as used herein, refers, for example, to a conditionally male sterile *Brassica napus* plant with the genotype Msmsrfrf (cf. section 2, "Production of basic seed" below), wherein said female *Brassica napus* plant is
i. heterozygous for the male sterility allele (Ms allele) obtainable from the *Brassica napus* seed deposited under Deposit Number NCIMB 41480,
ii. homozygous for the maintainer allele (rf allele) obtainable from the *Brassica napus* seed deposited under Deposit Number NCIMB 41480 or 41481,
iii. predominantly male sterile when exposed before and/or during flowering to a temperature of less than 28°C, and
iv. reverting to a predominantly male fertile phenotype when exposed before and/or during flowering to a temperature of higher than 35°C.

Both the prebasic and the basic male sterile female lines are suitable as female lines in the production of hybrid seed.

Finally, the term **"maintainer line",** as used herein, refers to a male fertile *Brassica napus* plant with the genotype msmsrfrf, which genotype is present in the *Brassica napus* seed deposited under Deposit Number NCIMB 41481 (cf. section 2.1, "Maintainer line" below),
wherein said *Brassica napus* plant is
i. homozygous for the fertility allele (ms allele) obtainable from the *Brassica napus* seed deposited under Deposit Number NCIMB 41481,
ii. homozygous for the maintainer allele (rf allele) obtainable from the *Brassica napus* seed deposited under Deposit Number NCIMB 41481, and
iii. predominantly male fertile.

It is a special advantage of the hybrid system of the present invention, that virtually every available rapeseed line are comprising a functional restorer allele and can be used as a male fertile line to be combined with the (male sterile) female line to produce hybrid seed.

One of the most hindering difficulties in providing a commercially feasible nuclear male sterility hybrid system is the propagation and multiplication of the (male sterile) female plant. In most systems only propagation starting from a plant heterozygous for the Ms allele is feasible. This leads to the need of extracting all those plants which as a consequence of the selfing lack the Ms allele entirely. It is thus advantageous to provide a conditional sterility system, wherein the fertility/sterility can be switched on/off based on a factorwhich is not present under normal growing conditions and wherein the distribution of fertility/sterility is nearly complete. No such system, which is commercially feasible, has been described so far.
It was now surprisingly found that certain components comprised in MSL hybrid seed could be utilized to obtain a commercial gms system. During the work a striking, and surprising, similarity to the Takagi system was observed. Although, the genetics of the hybrid system of the present invention were isolated from hybrid lines (in fact, the male sterile plants were initially selected from the commercially available NPZ hybrid line "Panther"; cf. Example 3), which could not be clearly linked to a Takagi germplasm (and are in fact described as belonging to a cms system), there are some phenotypic indications (such as the heat sensitivity) to assume at least a similarity of the genetics. Based on the MSL material markers have been developed (see Examples) and by use of these markers the elements of the system of the present invention have been isolated. However, seed of the original Takagi lines are not available anymore so a detailed comparison was not possible to perform. Even if the genetics in the Takagi germplasm would be identical to the one described herein, neither the original disclosure by Takagi nor the related subsequent work of Theis (1990) provide an enabling disclosure for the invention described hereunder. Without understanding the exact genetic of a hybrid system its reduction to a commercially viable process is not feasible.
Theis describes the Takagi system as follows: Male sterile plants were only present, if plants homozygous for the recessive male sterility (ms) gene also comprised the so-called modifying gene (md) in a non-homozygous recessive form. As a consequence, Theis is postulating a recessive male sterility gene and a dominant modifier gene. However, the findings by the present inventors are completely different the separation of the two genes underlying the Takagi system, which became possible by the provision of markers, demonstrated a dominant male sterility (Ms) gene and a recessive restorer allele (rf allele). Restoration of fertility is possible by a dominant restorer allele (Rf allele), which is present in all non-Takagi based lines (virtually all publicly available *Brassica napus* lines). Obviously, Theis never segregated the two genes nor did he provide lines, which were homozygous for the genes (as demonstrated by the segregation patterns in his crossing experiments). The gene which Theis wrongly named the "ms allele' is - as demonstrated in the context of the present invention - not able as such to provide a male sterile phenotype. In contrast, it is only the inactive form of a restorer allele (rf). The gene which Theis named the "modifying gene" is in fact the male sterility gene. This is not surprising based on the fact that without markers such segregation analysis can be very troublesome. Other phenotypic properties linked to the Ms allele are not suitable to differentiate between heterozygous and homozygous form. Thus, Theis is providing misleading information which would have precluded the person skilled in the art from developing a commercially viable hybrid system. Furthermore, a recessive male sterility gene is linked to many difficulties in a commercial hybrid system. Also from this angle, Theis is teaching away from a commercial application of the Takagi system.

Furthermore, providing a homozygous male sterile line would still not lead to a commercially viable system. Two important problems needed to be solved: First, the problem of propagation of the homozygous male sterile (Ms) line (referred to in the context of the present invention as prebasic female plants), which renders all other nuclear male sterility systems known until today commercially non-viable. At second, there is the problem of up-scaling to a commercial scale. The problem of propagation of the homozygous Ms line has been solved by the present invention by providing a conditionally, heat-inducible refertilization procedure. This procedure allows for an easy propagation of the male sterile prebasic line.

Although the prebasic female plants can principally be directly employed as a female for the production of hybrid seed, the heat treatment procedure would not be suitable to provide sufficient quantities of said line. Therefore, the method of the present invention employs an up-scaling step, in which the conditionally male sterile (prebasic female) line is crossed with a maintainer line to provide again male sterile seed. This seed is heterogeneous for the male sterility gene, but has still the conditionally male sterile phenotype. This propagation has only become possible by providing a maintainer line, which does have neither a functional ms allele nor a functional restorer allele. This up-scaling procedure is required for a commercially viable system.
Interestingly and being a special inventive and advantageous feature of the present invention, the fertility restorer allele is present in all non-Takagi lines, i.e. in virtually all available *Brassica napus* lines. This is beneficial because this means that virtually all lines can be used as male lines in the hybrid seed production process without the need to introgress a restorer allele. This is a significant advantage in comparison to, for example, the Ogura system and a striking similarity to the NPZ MSL system, which genetic is publicly unknown, although the NPZ MSL system is said to be a cytoplasmatic male sterility (cms) system (Frauen, 1999).

### 1. Method of providing the prebasic female plant and seed

A first embodiment of the present invention relates to a method for producing or multiplying seed of a conditionally (high temperature modulated) male sterile *Brassica napus* line with the genotype MsMsrfrf (suitable as prebasic female line for the production of *Brassica napus* hybrid seed), said method comprising the steps of
a) providing a conditionally male sterile *Brassica napus* plant with the genotype MsMsrfrf, which genotype is present in the *Brassica napus* seed deposited under Deposit Number NCIMB 41480, wherein said conditionally male sterile *Brassica napus* plant is
   i. homozygous for the male sterility gene (Ms allele) obtainable from the *Brassica napus* seed deposited under Deposit Number NCIMB 41480, and
   ii. homozygous for the maintainer allele (dysfunctional restorer allele; rf allele) obtainable from the *Brassica napus* seed deposited under Deposit Number NCIMB 41480 or 4148, and
   iii. predominantly male sterile when exposed before and/or during flowering to a temperature of less than 28°C, and
   iv. reverting to a predominantly male fertile phenotype when exposed before and/or during flowering to a temperature of higher than 35°C, and
b) exposing said conditionally male sterile *Brassica napus* plant for at least 4 hours to a temperature of higher than 35°C, and
c) exposing the heat-treated conditionally male sterile *Brassica napus* plant obtained in step (b) to a temperature of less than 33°C until development of male fertile flowers, and
d) allowing for self pollination of the *Brassica napus* plants having said male fertile flowers obtained in step (c), letting the seed develop, and harvesting the seed, wherein the harvested seeds are characterized in that they are seeds of a conditionally male sterile *Brassica napus* line with the genotype MsMsrfrf.

The term **"genotype MsMsrfrf"** means a plant homozygous for the dominant Ms allele and homozygous for the recessive maintainer allele (also referred to as dysfunctional restorer allele or rf allele). This genotype might be present in any genetic background of a Brassica plant, preferably a *Brassica napus* plant, with the provision that no copy a functional restorer allele (Rf allele) is present.

In a preferred embodiment of the present invention the conditionally male sterile *Brassica napus* plant provided in step (a) of the method for producing or multiplying seed of a conditionally male sterile *Brassica napus* line with the genotype MsMsrfrf described above is further characterized as being
iii. predominantly male sterile when exposed before and/or during flowering to a temperature of preferably less than 25°C, more preferably to a temperature of less than 20°C, but at least at a temperature, which allows for normal growing such as at least 12°C, preferably at least 14°C, more preferably at least 16°C, and
iv. reverting to a predominantly male fertile phenotype when exposed before and/or during flowering to a temperature of preferably between 35°C and 43°C, more preferably to a temperature of between 37°C and 40°C, most preferably to a temperature of about 39°C; most preferably with an exposure for the preferred heat treatment time as specified below (e.g., in step b) and a subsequent growing at ambient temperature, as defined below (e.g.. in step c)).

In a preferred embodiment of the present invention the conditionally male sterile *Brassica napus* plant is exposed in step (b) of the method for producing or multiplying seed of a conditionally male sterile *Brassica napus* line"with the genotype MsMsrfrf described above before and/or during flowering for at least 4 hours, preferably for at least 8 or t 2 hours, more preferably for at least 24 or 36 hours, even more preferably for at least 48 or 96 hours, and most preferably for at least 112 hours to a temperature of about 35°C to about 43 °C, preferably to a temperature of about 36 to about 42°C, more preferably to a temperature of about 37°C to about 41 °C. even more preferably to a temperature of about 38 to about 40°C, and most preferably to a temperature of about 39°C.
In a further preferred embodiment of the present invention the conditionally male sterile *Brassica napus* plant is exposed in step (c) of the method for producing or multiplying seed of a conditionally male sterile *Brassica napus* line with the genotype MsMsrfrf described above to a temperature of less than 30°C, preferably to a temperature of less than 28⁰C, more preferably to a temperature of between 16 and 25°C, even more preferably to a temperature of between 18 and 20°C until development of male fertile flowers.

The *Brassica napus* plant homozygous for the Ms allele represents a contribution of the present invention. Thus, another embodiment of the present invention relates to a conditionally male sterile *Brassica napus* plant with the genotype MsMsrfrf, which genotype is obtainable from the *Brassica napusseed* deposited under Deposit Number NCIMB 41480, wherein said female conditionally male sterile *Brassica napus* plant is
i. homozygous for the male sterility gene (Ms allele) obtainable from the *Brassica napus* seed deposited under Deposit Number NCIMB 41480,
ii. homozygous for the maintainer allele (dysfunctional restorer allele; rf allele) obtainable from the *Brassica napus* seed deposited under Deposit Number NCIMB 41480 or 41481,
iii. predominantly male sterile when exposed before and/or during flowering to a temperature of less than 28°C,
iv. reverting to a predominantly male fertile phenotype when exposed before and/or during flowering to a temperature of higher than 35°C.

The conditionally male sterile *Brassica napus* plant with the genotype MsMsrfrf as described above Is also referred to as **"prebasic female line"** or **"prebasic female"** in the context of the present invention.
Preferably, said conditionally male sterile *Brassica napus* plant with the genotype MsMsrfrf is obtainable from the seed produced by the method for the production of prebasic female seed of the present invention. Another embodiment of the present invention relates to seed, which grow said conditionally male sterile *Brassica napus* plant with the genotype MsMsrfrf, parts of said plant, and the use of said plant in a hybrid seed production process. Preferably, the genetic background of said plant is not a hybrid, more preferably it is an inbred *Brassica napus* line. Preferably said plant part is selected from the group comprising seeds, microspores, protoplasts, cells, ovules, pollen, vegetative parts, cotyledons, zygotes.
In a preferred embodiment of the present invention the conditionally male sterile *Brassica napus* plant with the genotype MsMsrfrf, which genotype is obtainable from the *Brassica napusseed* deposited under Deposit Number NCIMB 41480, is further characterized as being
iii. predominantly male sterile when exposed before and/or during flowering to a temperature of preferably less than 25°C, more preferably to a temperature of less than 20°C, but at least at a temperature, which allows for normal growing such as at least 12°C, preferably at least 14°C, more preferably at least 16°C, and
iv. reverting to a predominantly male fertile phenotype when exposed before and/or during flowering to a temperature of preferably between 35°C and 43°C, more preferably to a temperature of between 37°C and 40°C, most preferably to a temperature of about 39°C; preferably with an exposure for the preferred heat treatment time as specified herein and a subsequent growing at ambient temperature, as defined herein.
In a preferred embodiment of the present invention, the seed deposited under Deposit number NCIMB 41480 is used for obtaining a male sterile *Brassica napus* plant with the genotype MsMsrfrf (i.e., the prebasic female as disclosed herein). In a further preferred embodiment of the present invention, any seed produced by the Norddeutsche Pflanzenzucht Hans-Georg Lembke KG (NPZ) in Germany based on their MSL system can be used for obtaining a male sterile *Brassica napus* plant with the genotype MsMsrfrf. Examples of such seeds include, but are not limited to seeds of the lines Joker, Pronto, Panther, Artus, Baldur, Elan, Marcant, Mendel, Talent, Taurus, Tenno, Titan, Trabant, Zeppelin, Visby, Horus, and Siesta. Other examples of seeds from which the male sterile *Brassica napus* plant with the genotype MsMsrfrf can be obtained include, but are not limited to seeds like Alkido, Mika, Fangio, Elektra, and Libretto. In a further preferred embodiment of the present invention the seed deposited under Deposit number NCIMB 41480 are used in a method for producing or multiplying seed of a conditionally male sterile *Brassica napus* plant with the genotype MsMsrfrf, e.g. in the method as described above.
The prebasic male sterile line of the present invention preferably has a total glucosinolate content of not more than 50 µmol per gram of air-dry seed at 9% humidity (less than 40 µmol, more preferably between 5 and 35 µmol, most preferably between 10 and 25 µmol per gram). It has to be noted that the heat-treatment utilized in the propagation of the prebasic female (e.g. under heat-chamber conditions) increases the glucosinolate content to levels which in some cases exceeds 30 µmol per gram. Also the F₁ hybrid harvested from the sterile basic female can exceed 30 µmol GSL due to the low seed set in seed production. However, this is not a result of the genetics of the line but due to the environmental stress, whereas the glucosinolate levels in the grain yielded from the hybrid plants of the present invention (F₂ seed) are at commercial levels (having a total glucosinolate content of not more than 25 µmol per gram of air-dry seed at 9% humidity (preferably between 1 and 22 µmol, more preferably between 5 and 20 µmol, most preferably between 8 and 17 µmol per gram)

### 1.1 The Ms allele, its associated phenotype and marker

The term **"obtainable"** with regard to a deposit made under the Budapest treaty regulations and referring to the Ms, Rf, ms, rf allele or any genotype comprising a combination thereof means that these genes or genotypes can be obtained from said deposited material but can also be obtained from other material. The sequence of the genes obtained from other material may vary from the sequence of the gene in the deposited material ("variant"). Thus, the term **"Ms allele"** comprises the gene obtainable from the deposited material but also variants thereof. Accordingly, in one preferred embodiment of the present invention the Ms allele is the Ms allele present in the seed deposited under Deposit Number NCIMB 41480 or a genetic variant thereof, which confers essentially the same phenotype than the Ms allele present in the seed deposited under Deposit Number NCIMB 41480. In a preferred embodiment of the present invention the seed deposited under Deposit number NCIMB 41480 are used for obtaining the male sterility allele (Ms allele). In a further preferred embodiment of the present invention the male sterility allele (Ms allele) can also be obtained from seed produced by the Norddeutsche Pflanzenzucht Hans-Georg Lembke KG (NPZ) in Germany based on their MSL system. Examples of such seeds include, but are not limited to seeds of the lines Joker, Pronto, Panther, Artus, Baldur, Elan, Marcant, Mendel, Talent, Taurus, Tenno, Titan, Trabant, Zeppelin, Visby, Horus, and Siesta. Other examples of seeds from which the male sterility allele (Ms allele) can be obtained include, but are not limited to seeds like Alkido, Mika, Fangio, Elektra, and Libretto. Further, the male sterility allele (Ms allele) can also be obtained from seed produced by Syngenta (or one of its affiliates) such as, for example, but not restricted to seeds of the lines NK Petrol, NK Karibik, NK Speed, NK Octans, NK Kick, NK Technik, NK Picolo, NK Caravel.
**"Essentially the same phenotype",** as used herein, means the ability to confer a conditionally (preferably a high temperature modulated) nuclear male sterile phenotype.
Preferably, if obtained from other sources the origin of said other source is the mutated line provided by Takagi (1970). It has to be understood that although the Ms allele is obtainable from the *Brassica napus* seed deposited under Deposit Number NCIMB 41480, other sources may exist from which said Ms allele can be obtained (in identical or varying form). Identity and/or similarity of the genes and genotypes provided in the context of the present invention (whether obtained from the deposit or from other sources) can be demonstrated by one or more of the properties linked to said genes or genotypes described below.
The most characteristic and unique property of the Ms allele utilized in the context of the present invention is the fact that fertility can be restored by virtually any available *Brassica napus* line publicly available (other than lines resulting from a Takagi germplasm), but not by the maintainer line for which seeds are deposited under Deposit Number NCIMB 41481. The maintainer line as provided by the present invention comprises the maintainer allele (dysfunctional restorer allele) in a homozygous form (rfrf) while a "normal" *Brassica napus* plant comprises the restorer In a functional form (RfRf). Except for a germplasm derived from the Takagi germplasm there are no known lines which would not comprise at least one functional copy of the Rf allele. As a consequence thereof, virtually all available rapeseed lines at the date of the invention are found to be restorer lines. The presence of the two genes Ms and rf, which presumably present a double mutation in the Takagi germplasm, is essential for a functional hybrid system as disclosed in the present invention. This also means that by using the deposited material the presence of the Ms or the rf allele can be easily determined. A male sterile line based on the Ms allele can be "maintained" in its male sterile phenotype by crossing with the maintainer line for which seeds are deposited under Deposit Number NCIMB 41481, but can also be reverted to fertility by crossing with any restorer line (see below for examples).
Consequently, the Ms allele is linked to a male sterile phenotype which can be restored (at least in part in the following generation) to fertility by any plant comprising at least one dominant Rf allele (the so-called **"Restorer plants⁸** as referred to herein) but not by the plants derived from seed deposited under Deposit Number NCIMB 41481 (maintainer). Thus, the Ms allele is preferably characterized by conferring a conditional nuclear male sterile phenotype, which
a) is restored temporarily to fertility by an exposure to a temperature of higher than 35°C,
b) is restored to fertility in at least part of the F₁ plants obtained from crossing a male sterile plant with the genotype MsMsrfrf or Msmsrfrf with any *Brassica napus* plant comprising at least one dominant Rf allele, and
c) is maintained in the F₁ plants obtained from crossing a plant with a conditional male sterile phenotype referred by said Ms allele with the male fertile plants derived from seed deposited under Deposit Number NCIMB 41481.
In a preferred embodiment of the present invention the Ms allele is preferably characterized by conferring a conditional nuclear male sterile phenotype, which
a) is restored temporarily to fertility by an exposure to a temperature of higher than 35°C, preferably to a temperature of between 35°C and 43°C, more preferably to a temperature of between 37°C and 40°C, most preferably to a temperature of about 39°C; preferably with an exposure for the preferred heat treatment time as specified herein and a subsequent growing at ambient temperature, as defined herein,
b) is restored to fertility in at least part of the F₁ plants obtained from crossing a male sterile plant with the genotype MsMsrfrf or Msmsrfrf with any *Brassica napus* plant comprising at least one dominant Rf allele, or in all F₁ plants obtained from crossing a male sterile plant with the genotype MsMsrfrf or Msmsrfrf with a *Brassica napus* plant homozygous for the Rf allele; i.e. with the genotype RfRf, and
c) is maintained in the F₁ plants obtained from crossing a conditional male sterile plant with the genotype MsMsrfrf (e.g., a plant with a male sterile phenotype referred by said Ms allele) with the plants derived from seed deposited under Deposit Number NCIMB 41481.
A **"temporal restoration of fertility"** means that only the flowers induced during the high temperature treatment develop into male fertile flowers, whereas flowers induced either before or thereafter develop into male sterile flowers only, even if developing on the same plant. Thus, as the high temperature treatment is temporary, also the fertility restoration is temporary and correlates with the duration and length of the high temperature treatment.

Thus, by crossing a male sterile plant with
a) any male fertile, inbred *Brassica napus* plant comprising at least one Rf allele, preferably being homozygous for the Rf allele (Restorer plant), and
b) the plants derived from seed deposited under Deposit Number NCIMB 41481, the presence or absence of the Ms allele can be unambiguously identified.

**"Restorer plants"** or **"Restorer line"** means any male fertile, inbred *Brassica napus* plant comprising at least one Rf allele, preferably being homozygous for the Rf allele. Restorer plants include all fertile, inbred (open-pollinated, non-hybrid) *Brassica napus* lines commercialized as seed for growing, preferably at the priority date of this invention. No exception has been found by the inventors. Suitable Restorer lines also include those on the OECD variety list of December 2006 (OECD List of varieties eligible for certification - 2006/2007; Dec. 2006; http://www.oecd.org/dataoecd/1/44/33999447.PDF; http:/www.oecd.org/document/14/0,2340,en_2649_33909_2485070_1_1_1_1 ,00,html), preferably non-hybrid lines commercialized as seed for growing (for oil production), more preferably those, which are not marked as "d" (inbred as long as they represent hybrid parental lines) or "b" (hybrid) on said OECD list.
While this property is unique to the hybrid system of the present invention provided hereunderthere are other properties which are linked to or associated with the Ms allele. In a preferred embodiment of the present invention the conditionally male sterile phenotype and/or the Ms allele is linked to and/or associated with one or more characteristic selected from the group consisting of
I. a phenotype of bud abortion in a plant with a male sterile phenotype conferred by the Ms allele,
II. a phenotype of white-striped or white blotched petals in a plant with a male sterile phenotype conferred by the Ms allele, and
III, the presence of a Ms allele specific marker in both male fertile and male sterile plants comprising at least one copy of the Ms allele.

In another preferred embodiment of the present invention the conditionally male sterile phenotype and/or the Ms allele is linked to and/or associated with one or more marker (Ms allele marker) selected from the group ("MS gene **marker group**") consisting of
I. the markers selected from the group of polymorphisms (mutations) in the NR1116 marker region consisting of
   a) the single nucleotide polymorphism marker having an A at the position corresponding to position 85 in SEQ ID NO:3,
   b) the single nucleotide polymorphism marker having a G at the position corresponding to position 87 in SEQ ID NO:3,
   c) the single nucleotide polymorphism marker having an A at the position corresponding to position 139 in SEQ ID NO:3,
   d) the single nucleotide polymorphism marker C at the position corresponding to position 214 in SEQ ID NO:3,
   e) the single nucleotide polymorphism marker having a G at the position corresponding to position 218 in SEQ ID NO:3,
   f) the single nucleotide polymorphism marker having a G at the position corresponding to position 277 in SEQ ID NO:3,
   g) the single nucleotide polymorphism marker having an A at the position corresponding to position 286 in SEPA ID NO:3,
   h) the single nucleotide polymorphism marker having a T at the position corresponding to position 312 in SEQ ID NO:3,
   i) the single nucleotide polymorphism marker having a T at the position corresponding to position 319 in SEQ ID NO:3,
   j) the single nucleotide polymorphism marker C at the position corresponding to position 359 in SEQ ID NO:3,
   k) the deletion mutation 5'- TTGGTGAACAATC -3' at the position corresponding to 221 in SEQ ID NO:3
   l) the insertion mutation 5'- GAA -3' at the position corresponding to 328-330 in SEQ ID NO:3
II. the markers selected from the group of polymorphisms (mutations) in the NR2525 marker region consisting of
   a) the single nucleotide polymorphism marker having a A at the position corresponding to position 60 in SEQ ID NO: 6,
   b) the single nucleotide polymorphism marker having a T at the position corresponding to position 92 in SEQ ID NO:6,
   c) the single nucleotide polymorphism marker having a T at the position corresponding to position 105 in SEQ ID NO: 6,
   d) the single nucleotide polymorphism marker C at the position corresponding to position 158 in SEQ ID NO: 6,
   e) the single nucleotide polymorphism marker having a T at the position corresponding to position 431 in SEQ ID NO: 6,
   f) the single nucleotide deletion mutation at the position corresponding to position 82 in SEQ ID NO: 6,
   g) the deletion mutation 5'- TGAGCAAAA -3' at the position corresponding to position 17 to 25 in SEQ ID NO:6,
III. the markers selected from the group of SNP markers consisting of
   a) a positive signal in a SNP assay (preferably a TaqMan® based SNP assay) using a SNP-probe (SNP-Probe 1) comprising the nucleotide sequence described by SEQ ID NO: 12 (sterile allele specific probe HiNK6701) and a negative signal using a SNP-probe (SNP-Probe 2) comprising the nucleotide sequence described by SEQ ID NO: 11 (fertile allele specific probe HiNK6700),
   b) a positive signal in a SNP assay (preferably a TaqMan® based SNP assay) using a SNP-probe (SNP-Probe 3) comprising the nucleotide sequence described by SEQ ID NO: 17 (sterile allele specific probe HiNK6775) and a negative signal using a SNP-probe (SNP-Probe 4) comprising the nucleotide sequence described by SEQ ID NO: 18 (fertile allele specific probe HiNK6776),
IV. the markers selected from the group of SSR markers consisting of:
   a) a PCR fragment with an apparent molecular weight of 96.7 (+/- 1.0) bp resulting from a PCR reaction with the primers described by SEQ ID NOs: 1 and 2,
   b) a PCR fragment with an apparent molecular weight of 192.8 (+/- 0.3) bp resulting from a PCR reaction with the primers described by SEQ ID NOs: 4 and 5, and
V. the markers selected from the group of markers linked to at least one of the sequences set forth as SEQ ID NOs: 3, 6, 11 and 18,
wherein the one or more marker (Ms allele marker) also includes an isolated nucleotide sequence selected from the group consisting of sequences which
I. have a sequence identity of at least 80% to, or
II. hybridize under stringent conditions to, or
III. comprise at least 25 consecutive nucleotides of
the marker sequences defined above in I. to V.
In a preferred embodiment, the conditionally male sterile phenotype and/or the Ms allele is linked to and/or associated with markers selected from the group of polymorphisms (mutations) in the NR1116 marker region consisting of at least 1, 2, 3, 4, 5, 6, 7, 8, 9, 10, 11, or 12 of the mutations of group I., more preferably at least the mutations at the positions corresponding to position 214 (T/C) and 218 (T/G) in SEQ ID NO: 3. Preferably, there are at last 1, 2, 3, 4, 5, 6, or 7 of the mutations of group II., more preferably at least the mutations at the position corresponding to position 158 in SEQ ID NO: 6.
Further, one or more marker of group V. above linked to and/or associated with the conditionally male sterile phenotype and/or the Ms allele can be linked to 1, 2, 3, or all of the sequences set forth as SEQ ID NOs: 3, 6, 11 and 18.

Markers as described above may be used in various other aspects of the present invention. However, the aspects of the present invention are not limited to the use of the markers as disclosed in the present application. It is further emphasized that these aspects may also make use of markers not explicitly disclosed herein or markers yet to be identified.

The term "SNP" or **"single nucleotide polymorphism"** means a nucleotide (preferably a DNA) sequence variation occurring when a single nucleotide (preferablyA, T, C, orG) in preferably the genome (orothershared sequence) differs between members of a species or between paired chromosomes or alleles in an individual. For example, two sequenced DNA fragments from different individuals, AAGCCTA to AAGCTTA, contain a difference in a single nucleotide.
In this case one can say that there are two alleles: C and T. Single nucleotide polymorphisms may fall within coding sequences of genes, non-coding regions of genes, or in the intergenic regions between genes. SNPs within a coding sequence may not necessarily change the amino acid sequence of the protein that is produced, due to degeneracy of the genetic code. A SNP in which both forms lead to the same polypeptide sequence is termed *synonymous* (sometimes called a silent mutation), if a different polypeptide sequence is produced they are *non-synonymous.* SNPs that are not located in protein coding regions may still have consequences for gene splicing, transcription factor binding, or the sequence of non-coding RNA. However, a SNP as such may have no functional relevancy at all but might be only "linked" or associated with a certain phenotype and/or genotype (i.e., segregates with a certain probability with said genotype and/or phenotype). The term "SNP-probe" means a probe which is suitable to detect a SNP, more preferably a labeled probe, suitable for automated detection (as described below).
The phrase **"position corresponding to position X** In **SEQ ID NO: Y"** indicates that the sequence described by SEQ ID NO: Y is a consensus sequence. The corresponding sequence in a concrete plant (in which the presence or absence of the SNP is to be detected) will be (in most cases) different to said consensus sequence. However, in a preferred embodiment the sequence identity in an alignment of said consensus sequence to said sequence in said concrete plant can be determined. Sequence alignment is a way of arranging the primary sequences of nucleotides (e.g., DNA) to identify regions of similarity that may be a consequence of functional, structural, or evolutionary relationships between the sequences. Aligned sequences of nucleotide residues are typically represented as rows within a matrix. Gaps are inserted between the residues so that residues with identical or similar characters are aligned in successive columns. A sequence alignment can be produced for example by the ClustalW or BLAST program. If two sequences in an alignment share a common ancestor, mismatches can be interpreted as point mutations and gaps as indels (that is, insertion or deletion mutations) introduced into one or both lineages in the time since they diverged from one another. Accordingly, the sequence in said concrete plant may differ as a consequence of deletions and mutations in length to the consensus sequence. As a consequence, also the absolute position of a specific SNP (as measured from the start of the sequence) may differ. However, when aligned in a proper way those positions are still matched with each other. The phrase "position corresponding to position X in SEQ ID NO: Y" indicates this fact and thus means that - although in the sequence as obtained from a concrete plant - the absolute position might be different, in the alignment to the consensus sequence ("Y") it would still match the indicated position (X").
The term **"SNP assay"** means any of the methods known in the art which is suitable to detect or visualize a single nucleotide polymorphism. These methods can be for example based on hybridization. Several applications have been developed that interrogate SNPs by hybridizing complementary DNA probes to the SNP site (Rapley & Harbron, 2004). Such methods include Dynamic allele-specific hybridization (DASH). Other methods for SNP detection include the use of molecular beacons that make use of a specifically engineered single-stranded oligonucleotide probe comprising a fluorophore and a fluorescence quencher (Abravaya et al., 2003). SNPs can also be detected by high density oligonucleotide SNP arrays comprising hundreds of thousands of probes arrayed on a small chip, allowing for a large number of SNPs to be interrogated simultaneously (Rapley & Harbron, 2004). Furthermore, a broad range of enzymes including DNA ligase, DNA polymerase and nucleases have been employed to generate high-fidelity SNP genotyping methods. Another method is based on restriction fragment length polymorphism (RFLP). Various SNP detection methods based on PCR have been developed. Such methods include the tetra-primer ARMS-PCR, which employs two pairs of primers to amplify two alleles in one PCR reaction. Flap endonuclease (FEN) is an endonuclease that catalyzes structure-specific cleavage. This cleavage is highly sensitive to mismatches and can be used to interrogate SNPs with a high degree of specificity (Olivier, 2005). In the basic invader assay, a FEN called cleavase is combined with two specific oligonucleotide probes, which together with the target DNA can form a tripartite structure recognized by cleavase (Olivier, 2005).
Primer extension is a two step process that first involves the hybridization of a probe to the bases immediately upstream of the SNP nucleotide followed by a 'mini-sequencing' reaction, in which DNA polymerase extends the hybridized primer by adding a base that is complementary to the SNP nucleotide. This incorporated base is detected and determines the SNP allele (Syvanen, 2001; Rapley & Harbron, 2004). Illumina Incorporated's Infinium assay is an example of a whole-genome genotyping pipeline that is based on primer extension method. In the Infinium assay, over 100,000 SNPs can be genotyped. The assay uses hapten-labelled nucleotides in a primer extension reaction (Gunderson et al., 2006). Oligonucleotide ligase assay utilizes DNA ligase, which catalyzes the ligation of the 3' end of a DNA fragment to the 5' end of a directly adjacent DNA fragment. This mechanism can be used to interrogate a SNP by hybridizing two probes directly over the SNP polymorphic site (Rapley & Harbron, 2004).
Most preferred In the context of the present invention is the use of the TaqMan® assay for the detection of SNPs. The term "TaqMan® assay" in general means a quantitative real time PCR method using a dual-labelled fluorogenic probe (TaqMan probe; Heid et al., 1996). The TaqMan Real-time PCR measures accumulation of a product via the fluorophore during the exponential stages of the PCR, rather than at the end point as in conventional PCR. The exponential increase of the product is used to determine the threshold cycle, C_{T}, i.e. the numberof PCR cycles at which a significant exponential increase in fluorescence is detected, and which is directly correlated with the number of copies of DNA template present in the reaction. Different from regular PCR, in TaqMan real-time PCR a *probe* is added to the reaction, i.e., a single-stranded oligonucleotide complementary to a segment of 20-60 nucleotides within the DNA template and located between the two primers. A fluorescent reporter or fluorophore (e.g., 6-carboxyfluorescein, acronym: *FAM,* or tetrachlorofluorescein, acronym: TET) and a *quencher* (e.g., tetramethylrhodamine, acronym: TAMRA, or dihydrocyclopyrroloindole tripeptide "minor groove binder", acronym: MGB) are covalently attached to the 5' and 3' ends of the probe, respectively (Kutyavin, 2000). The close proximity between fluorophore and quencher attached to the probe inhibits fluorescence from the fluorophore. During PCR, as DNA synthesis commences, the 5' to 3' exonuclease activity of the Taq polymerase degrades that proportion of the probe that has an nealed to the template. Degradation of the probe releases the fluorophore from it and breaks the close proximity to the quencher, thus relieving the quenching effect and allowing fluorescence of the fluorophore. Hence, fluorescence detected in the real-time PCR thermal cycler is directly proportional to the fluorophore released and the amount of DNA template present in the PCR.
More specifically for SNP detection, Taq DNA polymerase's 5'-nuclease activity is used in the Taqman assay for SNP genotyping. The Taqman assay is performed concurrently with a PCR reaction and the results can be read in real-time as the PCR reaction proceeds (McGuigan & Ralston, 2002). The assay requires forward and reverse PCR primers that will amplify a region that includes the SNP polymorphic site. Allele discrimination is achieved using FRET combined with one or two allele-specific probes (the preferred SNP-probes, such as SNP-Probes 1, 2, 3 or 4 of the present invention) that hybridize to the SNP polymorphic site. The probes (e.g., the SNP-Probes 1, 2, 3 or 4 of the present invention) will preferably have a fluorophore linked to the 5' end of their nucleic acid core sequence and a quencher molecule linked to their 3' end. While the probe is intact, the quencher will remain in close proximity to the fluorophore, eliminating the fluorophore's signal. During the PCR amplification step, if the allele-specific probe is perfectly complementary to the SNP allele, it will bind to the target DNA strand and then get degraded by 5'-nuclease activity of the Taq polymerase as it extends the DNA from the PCR primers. The degradation of the probe results in the separation of the fluorophore from the quencher molecule, generating a detectable signal. If the allele-specific probe is not perfectly complementary, it will have lower melting temperature and not bind as efficiently. This prevents the nuclease from acting on the probe (McGuigan & Ralston, 2002). The Taqman assay is based on PCR, and is relatively simple to implement. The Taqman assay can be multiplexed by combining the detection of up to seven SNPs in one reaction. (Syvanen, 2001). See also Affymetrix (2007) Genome-Wide Human SNP Array 5.0. [online] Address: http://www.affymetrix.com/products/arrays/specific/genome_wide/ genome_wide snp_5.affx (Retrieved on February 27th, 2007). Reagents and detailed protocol for TaqMan based SNP detection are available and described (US 5,876,930; Livak et al., 1995; Pre-Developed TaqMan® Assay Reagents Allelic Discrimination Protocol; Part Number 4312214 Rev. C 5/2005; Applied Biosystems).
Additional SNP detection assays are well known to the person skilled in the art and are based on physical properties of DNA, single strand conformation polymorphism, employ temperature gradient gel electrophoresis or denaturing high performance liquid chromatography, high-resolution melting of the entire amplicon, or sequencing.
The term **"PCR fragment"** means a nucleic acid fragment (preferably a DNA fragment) obtained from amplification of a target DNA (e.g., a genomic DNA) by utilizing one or more primers, a DNA polymerase (preferably a heat-stable DNA polymerase) and one or more cycles of amplification. The polymerase chain reaction (PCR) is a biochemistry and molecular biology technique for exponentially amplifying DNA via enzymatic replication. As PCR is an *in vitro* technique, it can be performed without restrictions on the form of DNA, and it can be extensively modified to perform a wide array of genetic manipulations.
The term **"apparent molecular weight"** means the molecular weight of a molecule (e.g., a DNA fragment) as measured in comparison to a molecular weight standard. For example, the weight can be measure by gel (e.g., agarose or polyacrylamide gel) electrophoresis (in flatbed gels, capillaries or otherwise as known in the art). Preferably, molecularweight determination is done by using a DNA sequencer and fluorescence labeled probes as standard. More specifically, as used in the context of the present invention the apparent molecular weight means the weight determined in comparison to the GeneScan^{™} 500 ROX^{™} Size Standard (a ROX^{™} dye-labeled size standard for the reproducible sizing of fragment analysis data) by using a 3700 DNA Analyzer or equivalent. The GeneScan^{™} 500 ROX^{™} Size Standard is designed for sizing DNA fragments in the 35-500 nucleotides range and provides 16 single-stranded labeled fragments of 35, 50, 75, 100, 139, 150, 160, 200, 250, 300, 340, 350, 400, 450, 490 and 500 nucleotides. The sizing curve generated from these short fragments makes the GeneScan^{™} 500 ROX^{™} Size Standard ideal for a variety of fragment analysis applications such as Microsatellites, Fragment Length Polymorphisms and Relative Fluorescent Quantitation. Each of the DNA fragments is labeled with the ROX^{™} fluorophore which results In a single peak when run under denaturing conditions.

In another preferred embodiment, the Ms allele, the ms allele, and/or the male sterile phenotype are further characterized by being localized on the *Brassica napus* chromosome N7, preferably between the marker sequences NR1116 (e.g., SEQ ID NO: 21) and NR 2525 (e.g., SEQ ID NO: 22), more preferably with a distance of 2.8 cM to NR1116 and 6.0 cM to NR2525, even more preferably between the SNP markers SR0002A and SR0003B, most preferably with a distance of approximately 2.8 cM to SR0002A and 3.3 cM to SR0003B.
The term **"marker sequence NR1116"** means the sequence as described by SEQ ID NO: 21 and variants thereof which
i) have a sequence identity of at least 80% to, or
ii) hybridize under stringent conditions to, or
iii) comprise at least 25 consecutive nucleotides of
   the sequence as described by SEQ ID NO: 21.

The term **"marker sequence NR2525"** means the sequence as described by SEQ ID NO: 22 and variants thereof which
i) have a sequence identity of at least 80% to, or
ii) hybridize under stringent conditions to, or
iii) comprise at least 25 consecutive nucleotides of
   the sequence as described by SEQ ID NO: 22.

The term **"SNP marker SR0002A"** means the fragment comprising a SNP mutation as amplified by the primer pair described by SEQ ID NOs: 8 and 10, which - preferably - results for the fertile allele in a negative signal in a SNP assay (preferably a TaqMan® based SNP assay) using SNP-Probe 1 comprising the nucleotide sequence described by SEQ ID NO: 12 (sterile allele specific probe HiNK6701) and - preferably - a positive signal using SNP-Probe 2 comprising the nucleotide sequence described by SEQ ID NO: 11 (fertile allele specific probe HiNK6700) (and the other way round for the sterile allele).

The term **"SNP marker SR0003B"** means the fragment comprising a SNP mutation as amplified by the primer pair described by SEQ ID NOs: 15 and 16, which - preferably - results for the fertile allele in a negative signal in a SNP assay (preferably a TaqMan® based SNP assay) using SNP-Probe 3 comprising the nucleotide sequence described by SEQ ID NO: 17 (sterile allele specific probe HiNK6775) and - preferably - a negative signal using SNP-Probe 3 comprising the nucleotide sequence described by SEQ ID NO: 18 (fertile allele specific probe HiNK6776) (and the other way round for the sterile allele).

Thus, the male sterility gene (Ms allele) is linked in a homozygous form to a male sterile phenotype which
- can be restored to fertility by crossing with any plant comprising at least one dominant functional Rf allele (Restorer allele), and
- which can be maintained by crossing with plants derived from the seed deposited under Deposit Number NCIMB 41481 (Maintainer line),
wherein said Ms allele is selected from the group consisting of
a) the Ms allele as obtainable from the *Brassica napus seed* deposited under Deposit Number NCIMB 41480, and
b) variants thereof, which have the same phenotypic property (i.e., regarding the male sterility).

More preferably, said Ms allele is the Ms allele, which in the seed deposited under Deposit Number NCIMB 41480 is linked and/or associated with one or more characteristic selected from the group consisting of:
I. a phenotype of bud abortion in a plant with a conditionally male sterile phenotype conferred by the Ms allele,
II. one or more of the markers selected from the MS gene marker group (as defined above) in both male fertile and male sterile plants comprising at least one copy of the Ms allele,
III. a phenotype of white-striped or white blotched petals in a plant with a male sterile phenotype conferred by the Ms allele,
or a variant of said Ms allele, which exhibits a conditionally male sterile phenotype with the same restoring properties.

Consequently, in a preferred embodiment the male sterile *Brassica napus* plant with the genotype MsMsrfrf is homozygous for the male sterility gene (Ms allele) linked to a male sterile phenotype, which
- can be restored to fertility by crossing with any plant comprising at least one dominant functional Rf allele (Restorer), and
- which can be maintained by crossing with plants derived from the seed deposited under Deposit Number NCIMB 41481 (Maintainer),
wherein said Ms allele is selected from the group consisting of
a) the Ms allele as obtainable from the *Brassica napus* seed deposited under Deposit Number NCIMB 41480, and
b) variants thereof, which have the same phenotypic property (i.e., male sterility).
While the linkage to the Ms allele marker (characteristic II. as defined above) is present in both male fertile and male sterile plants comprising the Ms gene (i.e. irrespective of the presence or absence of the Rf allele), the phenotypic properties of bud abortion and white-striped or white blotched petals (characteristic I. and III., respectively) are only observable in a male sterile phenotype (i.e., in absence of the Rf allele). This observation thus allows for an easy discrimination of male sterile and male fertile plants.
It has to be understood that although in the deposited seed the Ms allele is linked to an Ms allele marker (characteristic II. as defined above) and/or to the phenotypic properties of bud abortion and/or white-striped or white blotched petals (characteristic I. and III., respectively), there might be circumstances under which said linkage might be lost or is broken intentionally or unintentionally during a breeding program. The likelihood of breaking said linkage depends from the distance of the marker to the Ms allele. It is well known to the person skilled in the art how to break linkage between a marker and a linked gene. However, it is possible by additional marker analysis, phenotype analysis, and or hybridization experiments to demonstrate identity of the Ms allele in such modified lines with the Ms allele in the deposited seed. For the phenotype of white-striped or white blotched petals (characteristic III.) it is so far unclear whether this is directly caused by the Ms allele or by a gene closely linked thereto. There are certain lines where this phenotype cannot be observed or is not clearly expressed. However, it is also known that such phenotypes can be masked by other phenotypes such has high-level expression of carotinoids in petals. So far no segregation of the male sterile phenotype and the bud abortion phenotype (characteristic I.) has been found. As a consequence, it is likely that this phenotype is either closely linked to or directly caused by the Ms allele.
The term **"variant",** when used with regard to the Ms or ms allele, means genetic variations which preferably do not affect the functionality of the Ms allele but which may affect its sequence. During propagation of an original line (e.g., the original Takagi line) it may happen that certain sequence polymorphisms or somaclonal variations occur in the genetic sequence of the Ms allele without affecting its function. Such variations may be in functionally non-relevant regions of the gene such as introns. Preferably, the genetic identity of a variant of the Ms allele and/or the ms allele is greater than 90%, preferably greater than 95%, more preferably greater than 98% in comparison to the Ms allele as obtainable from the seed deposited under Deposit Number NCIMB 41480 or the ms allele as obtainable from the seed deposited under Deposit Number NCIMB 41481. By other means, a variant preferably still hybridizes understringent conditions (preferably medium stringent conditions, more preferably high stringent conditions) with the Ms allele as obtainable from the seed deposited under Deposit Number NCIMB 41480. Since it can not be ruled out (in fact it is likely) that the Ms allele is a dysfunctional copy of the ms allele (which may present a functional gene involved in pollen or anther development in *Brassica napus*)*,* the term variant with regard to the Ms allele also comprises other (dysfunctional forms of the ms allele (or a variant thereof as defined above) as long as those can be maintained in sterility by the Maintainer line as deposited under Deposit Number NCIMB 41481. Such variations may vary from the Ms allele as obtainable from the seed deposited under Deposit Number NCIMB 41480 for example by different mutations, deletions, truncations etc.
The term "veriant", when used with regard to the Ms or ms allele, also refers to variants of the Ms or ms alleles that map in the same region as the Ms or ms allele described above, i.e. the variant is also being localized on the *Brassica napus* chromosome N7, preferably between the marker sequences NR1116 (e.g., SEQ ID NO: 21) and NR2525 (e.g., SEQ ID NO: 22), more preferably with a distance of 2.8 cM to NR1116 and 6.0 cM to NR2525, respectively, even more preferably between the SNP markers SR0002A and SR20003B, most preferably with a distance of approximately 2.8 cM to SR0002A and 3.3 cM to SR0003B, respectively. This type of variant also preferably hybridizes under stringent conditions (preferably medium stringent conditions, more preferably high stringent conditions) with the Ms allele as obtainable from the seed deposited under Deposit Number NCIMB 41480. Preferably, the variant of the Ms or ms allele exhibits a conditionally male sterile phenotype with the same restoring properties as described above for the Ms allele.

### 1.2 The rf allele and its phenotype

The terms **"maintainer allele" , dysfunctional restorer allele"** or **"rf allele"** mean the absence of the functional restorer allele (Rf allele) and - more specifically - a rf allele as obtainable from the *Brassica napus* seed deposited under Deposit Number NCIMB 41480 and/or 41481 and variants thereof. This rf allele is preferably linked to and characterized by the phenotypic properties of
a) not being capable of reverting to fertility the male sterile phenotype conferred by the Ms allele, and
b) being capable of maintaining the male sterile phenotype conferred by the Ms allele.
It has to be understood that although the maintainer allele (dysfunctional restorer allele; rf allele or rf) is obtainable from the *Brassica napus* seed deposited under Deposit Number NCIMB 41480 and/or 41481 other sources may exist from which said rf allele can be obtained.
In a preferred embodiment of the present invention the seed deposited under Deposit number NCIMB 41480 or the seed deposited under Deposit number NCIMB 41481 are used for obtaining the maintainer allele (rf allele).
In a further preferred embodiment of the present invention the maintainer allele (rf allele) can also be obtained from seed produced by the Norddeutsche Pflanzenzucht Hans-Georg Lembke KG (NPZ) in Germany based on their MSL system. Examples of such seeds include, but are not limited to seeds of the lines Joker, Pronto, Panther, Artus, Baldur, Elan, Marcant, Mendel, Talent, Taurus, Tenno, Titan, Trabant, Zeppelin, Visby, Horus, and Siesta. Other examples of seeds from which the maintainer allele (rf allele) can be obtained include, but are not limited to seeds like Alkido, Mika, Fangio, Elektra, and Libretto. Further, the maintainer allele (rf allele) can also be obtained from seed produced by Syngenta (or one of its affiliates) such as, for example, from seeds of the lines NK Petrol, NK Karibik, NK Speed, NK Octans, NK Kick, NK Technik, NK Picolo, NK Caravel.
The maintainer allele (rf allele) is linked to a male sterility maintaining phenotype, which in a homozygous form allows the male sterile phenotype caused by the Ms to be expressed Said male sterility maintaining phenotype can by reversed by at least one functional dominant Rf allele. Said Rf allele is obtainable from any non-Takagi based germplasm. Other than a germplasm derived from the Takagi germplasm there are no known lines which would not comprise at least one functional copy of the Rf allele. As a consequence virtually all rapeseed lines available at the date of the invention are found to be restorer lines.

In another preferred embodiment of the present invention the rf allele, the Rf allele, and/or the male sterility maintaining phenotype is further characterized by being localized on the *Brassica napus* Chromosome N19, preferably between the marker sequences NR2219 (e.g., SEQ ID NO: 23) and NR3454 (e.g., SEQ ID NO: 26), more preferably with a distance of 10.2 cM to NR2219 and 26.5 cM to NR3454, most preferably between the marker sequences NR3454 (e.g., SEQ ID NO: 26) and PUT-161a-Brassica_napus-59218 (e.g., SEQ ID NO: 31), more preferably with a distance of 26.5 cM to NR3454 and 4.1 cM to PUT-161a-Brassica_napus-59218.
The term **"marker sequence NR2219"**, as used herein, means the sequence as described by SEQ ID NO: 23 and variants thereof which
i) have a sequence identity of at least 80% to, or
ii) hybridize under stringent conditions to, or
iii) comprise at least 25 consecutive nucleotides of
   the sequence described by SEQ ID NO: 23.
   The term **"marker sequence NR3454"**, as used herein, means the sequence as described by SEQ ID NO: 26 and variants thereof which

i) have a sequence identity of at least 80% to, or
ii) hybridize under stringent conditions to, or
iii) comprise at least 25 consecutive nucleotides of
   the sequence described by SEQ ID NO: 26.
   The term **"marker sequence PUT-161 a-Brassica_napus-59218"**, as used herein, means the sequence as described by SEQ ID NO: 31 and variants thereof which

i) have a sequence identity of at least 80% to, or
ii) hybridize under stringent conditions to, or
iii) comprise at least 25 consecutive nucleotides of
   the sequence described by SEQ ID NO: 31.

Thus, the maintainer allele (rf allele) is linked in a homozygous form to a male sterility maintaining phenotype, which
a) is not capable of restoring fertility of a plant comprising at least one Ms allele,
b) is capable of maintaining sterility of a plant comprising at least one Ms allele,
wherein said rf allele is selected from the group comprising
a) the rf allele as obtainable from the *Brassica napus* seed deposited under Deposit Number NCIMB 41480 or 41481, and
b) variants thereof, which have the same phenotypic property regarding the restoring of and maintaining the male .. sterility conferred by the Ms allele.

In another preferred embodiment of the present invention the conditionally male sterility maintaining phenotype and/or the rf allele is linked and/or associated to one or more marker selected from the group (**"rf allele marker group"**) consisting of the SSR markers consisting of a PCR fragment with an apparent molecular weight of 240.8 (+/- 0.4) bp resulting from a PCR reaction with the primers having the sequences as described by SEQ ID NOs: 19 and 20. Consequently, in a preferred embodiment the *Brassica napus* plant with the genotype MsMsrfrf or msmsrfrf is homozygous for the maintainer allele (rt allele) linked in a homozygous form to a male sterility maintaining phenotype which
- is not capable of restoring fertility of a plant comprising at least one Ms allele,
- is capable of maintaining sterility of a plant comprising at least one Ms allele,
- is linked to one or more marker selected from the rf allele marker group (as defined above),
wherein said rf allele is selected from the group consisting of
a) the rf allele as obtainable from the *Brassica napus* seed deposited under Deposit Number NCIMB 41480 or 41481, and
b) variants thereof, which have the same phenotypic property.

In the context of the present invention a *Brassica napus* plant with the genotype msmsrfrf is referred to as **"maintainer"** or **"maintainer plant".**

The term "variant" when used with regard to the rf or Rf allele means genetic variations which preferably do not affect the functionality of the rf allele but which may affect its sequence. During propagation of an original line (e.g., the original Takagi line) it may happen that certain sequence polymorphism or somaclonal variations occur in the genetic sequence of the rf allele without affection its function. Such variations may be in functionally non-relevant regions of the gene such as introns. Preferably, the genetic identity of a variant of the rf allele and/or the Rf allele is greater than 90%, preferably greater than 95%, more preferably greater than 98% in comparison to the rf allele as obtainable from the seed deposited under Deposit Number NCIMB 41481 or the Rf allele as obtainable from any Restorer line. By other means a variant preferably still hybridizes under stringent conditions (preferably medium stringent conditions, more preferably high stringent conditions) with the rt allele as obtainable from the seed deposited under Deposit Number NCIMB 41480. Since it can not be ruled out (in fact it is likely) that the rf allele is a dysfunctional form of the Rf allele (which may present a functional gene involved in pollen or anther development in *Brassica napus),* the term variant with regard to the rf allele also comprises other dysfunctional forms of the Rf allele (or a variant thereof as defined above) as long as those can maintain the sterility of a male sterile Ms line as deposited under Deposit Number NCIMB 41480. Such variations may vary from the rf allele as obtainable from the seed deposited under Deposit Number NCIMB 41480 or 41481, for example, by different mutations, deletions, truncations etc.
As already stated above, the term "variant" when used with regard to the rf or Rf allele also refers to variants of the rf or Rf alleles that map in the same region as the rf or Rf allele described above, i.e. the variant is also being localized on the *Brassica napus* Chromosome N19, preferably between the marker sequences NR2219 (e.g., SEQ ID NO: 23) and NR3454 (e.g., SEQ ID NO: 26), more preferably with a distance of 10.2 cM to NR2219 and 26.5 cM to NR3454, most preferably between the marker sequences NR3454 (e.g., SEQ ID NO: 26) and PUT-161a-Brassica_napus-59218 (e.g., SEQ ID NO: 31), more preferably with a distance of 26.5 cM to NR3454 and 4.1 cM to PUT-161a-Brassica_napus-59218. This type of variant also preferably hybridizes understringentconditions (preferably medium stringent conditions, more preferably high stringent conditions) with the rf allele as obtainable from the seed deposited under Deposit Number NCIMB 41480 or 41481. Preferably, the variant of the rf or Rf allele exhibits the same phenotypic characteristics as described above for the rf or Rf allele.

### 1.3 Preferred Combination

Further, in an especially preferred embodiment the invention relates to a method of producing or multiplying seed of a conditionally male sterile *Brassica napus* line with the genotype MsMsrfrf (suitable as male sterile prebasic female for the production of *Brassica napus* hybrid seed), said method comprising the steps of
a) providing a conditionally male sterile *Brassica napus* plant with the genotype MsMsrfrf, which genotype is obtainable from the *Brassica napus* seed deposited under Deposit Number NCIMB 41480, wherein said conditionally male sterile *Brassica napus* plant is
   i. homozygous for the male sterility gene (Ms allele) linked to a male sterile phenotype which can be restored to fertility by crossing with any plant comprising at least one dominant functional Rf allele (Restorer gene), and which can be maintained by crossing with plants derived from the seed deposited under Deposit Number NCIMB 41481 (Maintainer), and wherein said Ms allele is selected from the group consisting of
      I) the Ms allele obtainable from the *Brassica napus* seed deposited under Deposit Number NCIMB 41480, and
      ii) variants thereof, which have essentially the same phenotypic property (i.e. confer a nuclear conditionally male sterile phenotype),
      and wherein said Ms allele is preferably the Ms allele, which in the seed deposited under Deposit Number NCIMB 41480 is linked and/or associated with one or more characteristic selected from the group consisting of:
      I. a phenotype of bud abortion in a plant with a male sterile phenotype conferred by the Ms allele,
      II. the presence of one or more marker selected from the MS gene marker group (as defined above) in both male fertile and male sterile plants comprising at least one copy of the Ms allele,
      III. a phenotype of white-striped or white blotched petals in a plant with a male sterile phenotype conferred by the Ms allele,
      or a variant thereof, which confers a nuclear conditionally male sterile phenotype (maintainable by the Maintainer line (as defined above), but restorable to fertility by any plant comprising an Rf allele; and thus having the same essential phenotypic property),
   ii. homozygous for the maintainer allele (dysfunctional restorer allele; rf allele), wherein said maintainer allele (rf allele) is linked in a homozygous form to a male sterility maintaining phenotype which is not capable of restoring fertility of a plant comprising at least one Ms allele, and which is capable of maintaining sterility of a plant comprising at least one Ms allele, and wherein said rf allele is selected from the group consisting of
      I) the rf allele as obtainable from the *Brassica napus* seed deposited under Deposit Number NCIMB 41480 or 41481, and
      II) variants thereof, which have the same phenotypic property,
      and wherein said rf allele preferably is the rf allele, which in the seed deposited under Deposit Number NCIMB 41480 is linked and/or associated with one or more marker selected from the rf allele marker group (as defined above),
      or a variant of said rf allele, which has essentially the same phenotypic property (i.e.; maintains the nuclear conditionally male sterile phenotype conferred by the Ms gene),
   iii. predominantly male sterile when exposed before and/or during flowering to a temperature of less than 28°C (preferably less than 25°C, more preferably less than 20°C, but at least at a temperature, which allows for normal growing such as at least 12°C, preferably at least 14°C, more preferably at least 16°C), and
   iv. reverting to a predominantly male fertile phenotype when exposed before and/or during flowering to a temperature of higher than 35°C (preferably between 35°C and 43°C, more preferably between 37°C and 40°C, most preferably at about 39°C; preferably with an exposure for the preferred heat treatment time as specified herein and a subsequent growing at ambient temperature as defined herein),
b) exposing said conditionally male sterile *Brassica napus* plant before and/or during flowering for at least 4 hours (preferably at least 8 or 12 hours, more preferably at least 24 or 36 hours, even more preferably at least 48 or 96 hours, most preferably at least 112 hours) to a temperature of about 35 to 43 °C (preferably about 36°C to about 42°C. more preferably about 37°C to about 41°C, even more preferably about 38°C to about 40°C, most preferably about 39°C), and
c) exposing the heat-treated a conditionally male sterile *Brassica napus* plant obtained in step b) to a temperature of less than 33°C (preferably less than 30°C, more preferably less than 28°C. even more preferably at a temperature between 14°C and 25°C, most preferably between 18°C and 20°C) until development of male fertile flowers, and
d) allowing for self pollination of the *Brassica napus* plants having said male fertile flowers obtained in step c), letting the seed develop, and harvesting the seed of said conditionally male sterile *Brassica napus* line with the genotype MsMsrfrf.

In the context of the present invention the conditionally male sterile *Brassica napus* plant with the genotype MsMsrfrf as described above is referred to as **"prebasic female".**

### 1.4 The heat treatment step

The propagation of the pre-basic female male sterile line (genotype MsMsrfrf) is conducted with a heat-induced fertility induction.
In a preferred embodiment, the "switch" between predominantly male sterile and predominately male fertile phenotype preferably means that not all plants and/or not all flowers at an individual plant have the same phenotype (sterility / fertility). Thus, fertile and sterile flowers can occur to a certain degree on the same plant In parallel. However, preferably the conditionally male sterile plants of the present invention (prebasic or basic female plants) have at a single plant to a degree of more than 80% (preferably more than 90%, more preferably more than 95%, even more preferably more than 98%) male sterile flowers at a temperature of less than 30°C (most preferably they do not show any fertile flowers on substantially all plants), but produce male fertile flowers to a degree of more than 20% (preferably more than 40%, more preferably more than 60%, most preferably more than 80%) at a temperature of higher than 35°C. The ratio of male fertile flowers to male sterile flowers is preferablydetermined 1 to 2 weeks after the termination of the heat exposure. If the heat is applied for about a week, the ratio is preferably determined about 2 to 3 weeks afterstart of the heat exposure. The male sterile phenotype of a plant comprising the Ms allele (and no Rf allele) can be reverted to a male fertile phenotype by an exposure to high temperatures of preferably about 35 to 43 °C (more preferably between 37°C and 40°C, most preferably at about 39°C). At a temperature of less than 28°C, preferably less than 25°C, more preferably 20°C before and during flowering there is only a male sterile phenotype. However, at a temperature of higher than 35°C before and/or during flowering fertile flowers develop. An optimal ratio between flower development and heat exposure is found at temperatures of approximately 39°C, i.e. the damage caused by heat stress induced by the elevated temperature is manageable by the plant, whereas on the other hand the development of male fertile flower is induced sufficiently to obtain the ratios of male fertile flowers to male sterile flowers on the same plant as described above. Heat exposure can be conducted in climate chambers (Redeker Kaeltetechnik; D-32791 Lage, Germany) with temperature (tolerance ± 1°C) and humidity control. In a further preferred embodiment, the heat exposure is conducted in a greenhouse compartment where a similar temperature regime could be applied. It is also preferred to conduct the heat treatment in the field in plastic tunnels during the vegetation period with the high temperature being applied by natural heating or additional heating with any kind of heater. It is even possible to maintain the male sterile plants in the field if the growing field provides a significant temperature level during the flowering period.
The term **"exposure"** in relation to the high temperature treatment means a treatment with high temperature, preferably under otherwise optimal growing conditions such as high humidity (>80%), fertilizer and crop protection treatment etc.. The exposure is preferably carried out for at least 4 hours, preferably for at least 8 or 12 hours, more preferably for at least 24 or 36 hours, even more preferably for at least 48 or 96 hours, most preferably for at least 112 hours ("heat treatment time"). The heat treatment time can be continuous or interrupted with periods at normal (ambient) temperature (preferably at about 19°C to 22°C). Preferably, the heat treatment time is applied over a period of 1 to 14 days, more preferably between about 3 and 10 days, even more preferably between 4 and 9 days, or between 5 and 8 days, most preferably for about 7 days. As described above, the heat treatment does not necessarily mean a treatment at a high temperature over the entire time. In one preferred embodiment of the present invention the heat treatment for the fertilization of the prebasic female is carried out with a day temperature / night temperature variation to avoid excessive temperature stress. This decreases the heat stress on the plants. Preferably during day time the heat is increased (as defined above) while at night time the heat is decreased to a temperature of less than 33°C, preferably less than 30°C, more preferably less than 28°C, even more preferably to a temperature between 16 and 25°C, most preferably between 19 and 22°C. Preferably, the heat treatment time is equally distributed over the above indicated period by adjusting it to a day:night regime. The ratio of day to night is between from about 0.5:1 to about 3:1, preferably from about 1:1 to about 2.5:1, more preferably from about 1.2:1 to about 2.0:1, most preferably it is about 1.8:1 (preferably with a day night temperature of 39°C (day temperature) to 21.5°C (night temperature)). In a preferred embodiment of the present invention the day:night regime is regulated by artificial light in a heat treatment / growing chamber. Preferably artificial day light of at least 10000 lux is used.
Depending on the temperature and the length of the exposure a plant may comprise both fertile and sterile flowers. However, if heat exposure is stopped prior to the end of the flowering process additional male sterile flowers can be developed in addition to the male fertile one. However, preferably the temperature and the length of exposure is adjusted to produce prebasic plants with more than 30% (preferably more than 50%) male fertile flowers.
After the heat exposure and after development of fertile flowers has been induced the plants can be transferred to "normal" conditions (in general a temperature of less than 28°C) - for example in greenhouses) and the development of these flowers will continue (although no further fertile flowers will be induced, the already induced buds will open as fertile flowers). Preferably the heat-treated *Brassica napus* plant are transferred to an environment with a temperature of less than 33°C, preferably less than 30°C, more preferably less than 28°C, even more preferably at a temperature between 16 and 25°C, most preferably between 18°C and 20°C) until development of male fertile flowers. Typically plants are kept under these conditions for about 5 to 14 days (preferably 5 to 7 days) until male fertile flowers develop. The so obtained male fertile Ms pre-basic plants are "selfed", i.e. they are allowed to self-pollinate. Such self-pollination can occur with or without human interference. It is however important that no crosspollination with other Brassica plants occurs. In consequence the plants or the fertile flowers are kept isolated from different pollinators. Selfing can be increased by e.g. brush-mediated pollen transfer or other methods known in the art. After successful pollination (selfing), the heat-treated "fertilized" plants can be used as male line (pollinator) In combination with a non-heat treated line of the same genotype as male sterile female line. Alternatively the heat treated line can be employed as a single line, i.e. representing both the female and male line. Both procedures are herein understood as self-pollination based on the identical genetic background. After pollination the pollinated plants are allowed to develop mature seeds, which are harvested as known to the person skilled in the art and stored for further use (e.g., for production of basic female seed).

It is to be noted - and it is also a preferred embodiment of the present Invention - that it is not necessary to exposure all plants of the pre-basic female male sterile line (genotype MsMsrtrf) to the heat for obtaining pollen of the pre-basic female male sterile line. In order to obtain sufficient amounts of pollen only a part of the plants of the pre-basic female male sterile line or only some or a few of these plants is/are treated with the elevated temperatures. With the pollen obtained in the treated plants the rest of the plants of the pre-basic female male sterile line will be pollinated. The number of plants that have to be exposed to the heat in order to obtain sufficient amounts of pollen are known to the person skilled in the art.

### 2. Production of basic seed

Principally the prebasic female line would already be suitable as (male sterile) female line directly for the production of hybrid seed. However, although the multiplication via the heat treatment step can be performed on father large scale (either in climatized chambers or by growing under natural conditions providing sufficient heat before or during the flowering developmental stage for male fertility induction), it is commercially less attractive because of the additional costs resulting from the specialist equipment of the heat-chambers, or - under natural conditions - the low and/or uncontrollable seed yields.
It is therefore an inventive contribution of the present invention to provide a step of multiplication of the male sterile phenotype, line and seed without heat treatment. This can be achieved by crossing the prebasic (male sterile) female line with a maintainer line, which does not comprise the Ms allele but only comprises the maintainer allele (dysfunctional restorer allele; Maintainerline; genotype msmsrfrf). Such a crossing results in a line which is still male sterile but comprises only one copy of the Ms allele. This crossing of the male sterile prebasic female and the Maintainer as a male can be used to provide male sterile basic seed (i.e., seed of the male sterile basic female) on commercial scale.

Thus, another preferred embodiment of the present invention relates to a method for producing seed of a conditionally male sterile *Brassica napus* line with the genotype Msmsrfrf suitable as (male sterile) basic female for the production of *Brassica napus* hybrid seed, said method comprising the steps of
a) providing as a female plant a conditionally male sterile (pre-basic) *Brassica napus* plant with the genotype MsMsrfrf, which genotype is obtainable from the *Brassica napus* seed deposited under Deposit Number NCIMB 41480, wherein said conditionally male sterile female *Brassica napus* plant with the genotype MsMsrfrf is
   i. homozygous for the male sterility gene (Ms allele) obtainable from the *Brassica napus* seed deposited under Deposit Number NCIMB 41480, and
   ii. homozygous for the maintainer allele (dysfunctional restorer allele; rf allele) obtainable from the *Brassica napus* seed deposited under Deposit Number NCIMB 41480 or 41481, and
   iii. predominantly male sterile when exposed before and/or during flowering to a temperature of less than 28°C, and
   iv. reverting to a predominantly male fertile phenotype when exposed before and/or during flowering to a temperature of higher than 35°C, and
b) providing as a male plant a male fertile maintainer *Brassica napus* plant with the genotype msmsrfrf, which genotype is obtainable from the *Brassica napus* seed deposited under Deposit Number NCIMB 41481, wherein said *Brassica napus* plant with the genotype msmsrfrf is
   i. homozygous for the fertility allele (dysfunctional male sterility gene; ms allele) obtainable from the *Brassica napus* seed deposited under NCIMB Deposit Number NCIMB 41481, and
   ii. homozygous for the maintainer allele (dysfunctional restorer allele; rf allele) obtainable from the *Brassica napus* seed deposited under Deposit Number NCIMB 41481, and
   iii. predominantly male fertile, and
c) allowing the male plant of step b) to pollinate the female plant of step a), letting the seed develop (preferably until maturity), and harvesting the seed, wherein the harvested seeds are characterized in that they are seeds of a conditionally male sterile *Brassica napus* line with the genotype Msmsrfrf (i.e., the basic female plant).

The Ms allele and rf allele are defined as above with the same preferences as for the pre-basic female line. The fertility allele (dysfunctional male sterility gene; ms allele) is defined below.

In a preferred embodiment of the present invention the conditionally male sterile *Brassica napus* plant provided in step (a) of the method for producing seed of a conditionally male sterile *Brassica napus* line with the genotype Msmsrfrf described above is further characterized as being
a. predominantly male sterile when exposed before and/or during flowering to a temperature of preferably less than 25°C, more preferably to a temperature of less than 20°C, but at least at a temperature, which allows for normal growing such as at least 12°C, preferably at least 14°C, more preferably at least 16°C (substep iii. of step a) above), and
b. reverting to a predominantly male fertile phenotype when exposed before and/or during flowering to a temperature of preferably between 35°C and 43°C, more preferably to a temperature of between 37°C and 40°C, most preferably to a temperature of about 39°C; most preferably with an exposure for the preferred heat treatment time as specified herein and a subsequent growing at ambient temperature, as defined herein.
In a preferred embodiment of the present invention the conditionally male sterile *Brassica napus* plant provided in step (b) of the method for producing or multiplying seed of a conditionally male sterile *Brassica napus* line with the genotype Msmsrfrf described above optionally has a total glucosinolate content of not more than 25 µmol per gram (preferably between 1 and 22 µmol, more preferably between 5 and 20 µmol, most preferably between 8 and 17 µmol per gram) of air-dry seed at 9% humidity.

In a preferred embodiment of the method for producing seed of a conditionally male sterile *Brassica napus* line with the genotype Msmsrfrf suitable as (male sterile) basic female for the production of *Brassica napus* hybrid seed, the conditionally male sterile (pre-basic) *Brassica napus* plant with the genotype MsMsrfrf used as a female plant and provided in step a) is grown from seed that has been produced using the method described above for producing or multiplying seed of a conditionally male sterile *Brassica napus* line with the genotype MsMsrfrf, i.e. by
a) providing a conditionally male sterile *Brassica napus* plant with the genotype MsMsrfrf, which genotype is present in the *Brassica napus* seed deposited under Deposit Number NCIMB 41480, wherein said conditionally male sterile *Brassica napus* plant is
   i. homozygous for the male sterility gene (Ms allele) obtainable from the *Brassica napus* seed deposited under Deposit Number NCIMB 41480, and
   ii. homozygous for the maintainer allele (dysfunctional restorer allele; rf allele) obtainable from the *Brassica napus* seed deposited under Deposit Number NCIMB 41480 or 4148, and
   iii. predominantly male sterile when exposed before and/or during flowering to a temperature of less than 28°C, and
   iv. reverting to a predominantly male fertile phenotype when exposed before and/or during flowering to a temperature of higher than 35°C, and
b) exposing said conditionally male sterile *Brassica napus* plant for at least 4 hours to a temperature of higher than 35°C, and
c) exposing the heat-treated conditionally male sterile *Brassica napus* plant obtained in step (b) to a temperature of less than 33°C until development of male fertile flowers, and
d) allowing for self pollination of the *Brassica napus* plants having said male fertile flowers obtained in step (c), letting the seed develop, and harvesting the seed, wherein the harvested seeds are characterized in that they are seeds of a conditional male sterile *Brassica napus* line with the genotype MsMsrfrf.

Preferably, the male line and the female (male sterile) line employed in the production of the basic female seed are based on an identical genetic background. More preferably, said male line and said (male sterile) female lines are provided by introgression of the respective genes for the hybrid system into an inbred *Brassica napus* line followed by at least one (preferably 2, 3 or 4) backcrossing against said line. For example, introgression can comprise one or more methods selected from a group consisting of isolation and transformation, conventional breeding, pedigree breeding, crossing, self-pollination, haploidy, doubler-haploid technology, embryo rescue, single seed descent, marker assisted breeding, induced mutagenesis, and backcrossing.
In one preferred embodiment of the present invention, the production of the basic female seed can be realized by growing the respective female (male sterile) and the male plants in alternating stripes, where the pollinator (the male fertile line) is discarded after pollination. Good pollination conditions are preferred for a sufficient yield on the female line. The relation of mother and pollinator should be preferably 2:1, 3:1, or 4:1, with 3:1 being preferred. The ratio can be set by using sowing machines with a 2/3 setup. 3 to 5 bee hives per hectare are useful if production is performed in fields.
If it is intended to produce prebasic seed with a uniform Msmsrfrf genotype; preferably, if performed in the field, the production of the basic female seed is carried out under conditions and/or at locations where the temperature does not increase beyond 33°C, preferably 30°C. For winter-type oilseed rape which is planted in early spring this is normally not critical in most locations. For spring-type oilseed rape (e.g., canola) locations with more ambient temperature conditions (Sweden, Canada) are preferred. However, a production at higher temperature is not disadvantageous at this stage. It would only cause when a temperature of 35°C is exceeded - that the prebasic female line can self-pollinate and that the produced basic seed comprises a certain level of prebasic seed. However, both prebasic seed and basic seed are equally well suited to grow a female plant for the production of hybrid seed. Consequently, a temperature control for the production of the prebasic female line is merely optional and more linked to high seed yield (which gets reduced at higher temperature) than to preventing fertilization of the prebasic female.
To avoid crosspollination with pollen from plants other than the maintainer fine, the field for the production of the basic seed is kept separate from other *Brassica napus* plants, preferably other Brassica plantings as such. The isolation distances is at least 500 m, preferably 1 km, more preferably 2 km, most preferably 5 km.

It has been found that the female (male sterile) plants are delayed in the flowering as compared to the male fertile plants employed in these production steps. To allow for optimal pollination it is therefore preferred to synchronize the flowering of the male and the (male sterile) female plant by one ore more of the methods selected from the group consisting of: ..
i. cut-back of the male fertile plant (i.e. the flowering part) to delay flowering until flowering of the female (male sterile) plant (preferably the plant is cut back immediately after start of flowering by approximately 30 cm using an electric or mechanical cutter),
ii. treatment of the flowering plant with growth and or ripening delaying chemicals (such as Folicur™, a fungicide with growth-regulating properties), and
iii. delay of sowing of the male fertile parent by up to three weeks (i.e. the male (male fertile) plants are sown up to 3 weeks later than the female (male sterile) plants).

It is preferred that the production of the basic female seed and/or hybrid seed is conducted at a temperature of less than 33°C, preferably less than 28°C, more preferably less than 25°C. The propagation effect from the prebasic to the basic seed is in general approximately 1:500 to 1:1000.

The basic female (male sterile) plant resulting from this process is preferably a conditionally male sterile *Brassica napus* plant with the genotype Msmsrfrf, wherein said female (conditionally male sterile) *Brassica napus* plant is
i. heterozygous for the male sterility allele (Ms allele) obtainable from the *Brassica napus* seed deposited under Deposit Number NCIMB 41480, and
ii. homozygous for the maintainer allele (dysfunctional restorer allele; rf allele) obtainable from the *Brassica napus* seed deposited under Deposit Number NCIMB 41480 or 41481, and
iii. predominantly male sterile when exposed before and/or during flowering to a temperature of less than 28°C, and
iv. reverting to a predominantly male fertile phenotype when exposed before and/or during flowering to a temperature of higher than 35°C.

The conditionally male sterile *Brassica napus* plant with the genotype Msmsrfrf as described above is further characterized as being
I. predominantly male sterile when exposed before and/or during flowering (see iii. above) to a temperature of preferably less than 25°C, more preferably to a temperature of less than 20°C, but at least at a temperature, which allows for normal growing such as at least 12°C, preferably at least 14°C, more preferably at least 16°C, and
II. reverting to a predominantly male fertile phenotype when exposed before and/or during flowering to a temperature of preferably between 35°C and 43°C, more preferably to a temperature of between 37°C and 40°C, most preferably to a temperature of about 39°C; preferably with an exposure for the preferred heat treatment time as specified herein and a subsequent growing at ambient temperature, as defined herein (see iv. above).

In a preferred embodiment of the present invention the conditionally male sterile *Brassica napus* plant with the genotype Msmsrfrf as described above is further characterized as having a total glucosinolate content of not more than 25 µmol per gram of air dry seed at 9% humidity, preferably between 1 and 22 µmol, more preferably between 5 and 20 µmol, most preferably between 8 and 17 µmol per gram.

The Ms allele and rf allele are as defined above with the same preferences as for the pre-basic female line. The fertility allele (dysfunctional male sterility gene; ms allele) is defined below.

Preferably, the conditionally male sterile *Brassica napus* plant with the genotype Msmsrfrf (i.e. the basic female plant) is
i. heterozygous for the male sterility allele (Ms allele) linked to a male sterile phenotype which can be restored to fertility by crossing with any plant comprising at least one dominant functional Rf allele (Restorer), and can be maintained by crossing with plants derived from the seed deposited under Deposit Number NCIMB 41481 (Maintainer), and wherein said Ms allele is selected from the group consisting of
   I) the Ms allele as obtainable from the *Brassica napus* seed deposited under Deposit Number NCIMB 41480, and
   II) variants thereof, which have the same essential phenotypic property (i.e. conferring a nuclear conditionally male sterile phenotype (maintainable by the Maintainer line as defined above, but restorable to fertility by any plant comprising an Rf allele)),
   and wherein said Ms allele is preferably linked and/or associated with one or more characteristic selected from the group consisting of:
   I. a phenotype of bud abortion in a plant with a male sterile phenotype conferred by the Ms allele, and
   II. one or more marker selected from the MS gene marker group (as defined above) in both male fertile and male sterile plants comprising at least one copy of the Ms allele, and
   III.a phenotype of white-striped or white blotched petals in a plant with a male sterile phenotype conferred by the Ms allele,
   or a variant thereof, which confers a nuclear conditionally male sterile phenotype (maintainable by the Maintainer line (as defined above), but restorable to fertility by any plant comprising an Rf allele; and thus having the same essential phenotypic property),
ii. homozygous for the maintainer allele (dysfunctional restorer allele; rf allele), wherein said maintainer allele (rf allele) is linked in a homozygous form to a male sterility maintaining phenotype which is not capable of restoring fertility of a plant comprising at least one Ms allele, and which is capable of maintaining sterility of a plant comprising at least one Ms allele, and wherein said rf allele is selected from the group consisting of
   I) the rf allele as obtainable from the *Brassica napus* seed deposited under Deposit Number NCIMB 41480 or 41481, and
   II) variants thereof, which have the same phenotypic property (i.e. maintain the nuclear conditionally male sterile phenotype conferred by the Ms allele),
   and wherein said rf allele preferably is the rf allele, which in the seed deposited under Deposit Number NCIMB 41480 is linked and/or associated with one or more marker selected from the rf allele marker group (as defined above), or a variant of said rf allele; which has essentially the same phenotypic property (i.e.; maintains the nuclear conditionally male sterile phenotype conferred by the Ms gene),
iii. predominantly male sterile when exposed before and/or during flowering to a temperature of less than 28°C (preferably less than 25°C, more preferably less than 20°C, but at least at a temperature, which allows for normal growing such as at least 12°C. preferably at least 14°C. more preferably at least 16°C), and
iv. reverting to a predominantly male fertile phenotype when exposed before and/or during flowering to a temperature of higher than 35°C (preferably between 35°C and 43°C, more preferably between 37°C and 40°C, most preferably at about 39°C; preferably with an exposure for the preferred heat treatment time as specified herein and a subsequent growing at ambient temperature as defined herein), and
v. optionally having a total glucosinolate content of not more than 25 µmol per gram of air dry seed at 9% humidity (preferably between 1 and 22 µmol, more preferably between 5 and 20 µmol, most preferably between 8 and 17 µmol per gram).

On the other hand, the plant heterozygous for the male sterility allele (Ms allele) obtainable from the *Brassica napus* seed deposited under Deposit Number NCIMB 41480 is heterozygous for the fertility allele (dysfunctional male sterility allele; ms allele). A detailed definition is provided below.

In a preferred embodiment of the present invention the seed deposited under Deposit number NCIMB 41480 are used in a method for producing a conditionally male sterile *Brassica napus* plant with the genotype Msmsrfrt, i.e. the basic mother according to the present invention. In a further preferred embodiment of the present invention seed produced by the Norddeutsche Pflanzenzucht Hans-Georg Lembke KG(NPZ) in Germany based on their MSL system can also be used in a method for producing a conditionally male sterile *Brassica napus* plant with the genotype Msmsrfrf. Examples of such seeds include, but are not limited to seeds of the lines Joker, Pronto, Panther, Artus, Baldur, Elan, Marcant, Mendel, Patent, Taurus, Tenno, Titan, Trabant, Zeppelin, Visby, Horus, and Siesta. Other examples of seeds that can also be used in a method for producing a conditionally male sterile *Brassica napus* plant with the genotype Msmsrfrf include, but are not limited to seeds like Alkido, Mika, Fangio, Elektra, and Libretto. Further, also seeds produced by Syngenta (or one of its affiliates) such as, for example, from seeds of the lines NK Petrol, NK Karibik, NK Speed, NK Octans, NK Kick, NK Technik, NK Picolo, NK Caravel, can also be used in a method for producing a conditionally male sterile *Brassica napus* plant with the genotype Msmsdrf.

Preferably, the conditionally male sterile *Brassica napus* plant with the genotype Msmsrfrf is suitable as a basic female seed in a production system for hybrid seed.

In a preferred embodiment, said basic female plant is used in a hybrid seed production process. Preferably, the genetic background of said basic female plant is not a hybrid, more Preferably it is an inbred Other preferred embodiments of the present invention relate to the plant components employed in the hybrid seed production system of the present invention; the prebasic female, the maintainer line, the basic female, the resulting hybrid plants, and the seeds growing said plants.

The production of basic seed (i.e. the seed growing basic female plants) is preferably performed in a field by using the conditionally male sterile prebasic female and the mate fertile maintainer line. Preferably the two lines are planted in stripes (as described by Sauermann & Lamp, 1997). Only the seeds obtained on the male sterile female prebasic line are harvested. The maintainer is only used a pollinator and is removed after flowering, and disposed.

### 2.1 Maintainer line

The present invention further relates to a male fertile *Brassica napus* plant with the genotype msmsrfrf (also referred to as "maintainer" in the context of the present invention), which genotype is obtainable from the *Brassica napus* seed deposited under Deposit Number NCIMB 41481, wherein said male fertile. *Brassica napus* plant is
i. homozygous for the fertility allele (dysfunctional male sterility allele; ms allele) obtainable from the *Brassica napus* seed deposited under Deposit Number NCIMB 41481, and
ii. homozygous for the maintainer allele (dysfunctional restorer allele; rf allele) obtainable from the *Brassica napus* seed deposited under Deposit Number NCIMB 41481, and
iii. predominantly male fertile, and
iv. optionally having a total glucosinolate content of not more than 25 µmol per gram (preferably between 1 and 22 µmol, more preferably between 5 and 20 µmol, most preferably between 8 and 17 µmol per gram) of air-dry seed at 9% humidity yielding said plant.

The rf allele is as defined above with the corresponding preferences.

In preferred embodiments of the present invention the seed deposited under Deposit number NCIMB 41481 is used for obtaining a male fertile *Brassica napus* plant with the genotype msmsrfrf (i.e., the maintainer line as described above) or for providing the fertility allele (ms allele as described below under 2.2) and/or the maintainer allele (rf allele as described above under 1.2) for use in any of the methods of the present invention.

A further-preferred aspect of the present invention relates to the use of the seed deposited under Deposit number NCIMB 41481 for maintaining the conditional male sterility of seeds produced in the method for producing seed of a conditionally male sterile *Brassica napus* plant with the genotype Msmsrfrf (i.e. the prebasic female of the present invention) according to the present.invention (i.e. the method as described in sections 1).

### 2.2 The ms allele, its associated phenotype and marker

The terms "ms allele", "fertility allele", or "dysfunctional male sterility allele" mean the absence of the functional male sterility allele (Ms allele or Ms gene) and - more specifically - a ms allele as obtainable from the *Brassica napus* seed deposited under Deposit Number NCIMB 41481 and variants of said allele conferring essentially the same phenotype (i.e. a male fertile phenotype). This ms allele is preferably linked to and/or characterized by the phenotypic properties of
a) not being capable of reverting to fertility the male sterile phenotype caused by the Ms allele, and
b) not being capable of conferring a male sterile phenotype in absence of a Ms allele.
While these properties are unique to the ms allele, there are other properties which are linked to or associated with the ms allele. In a preferred embodiment of the present invention the ms allele is linked to one or more marker ("ms allele marker") selected from the group ("ms allele marker group") consisting of
I, the markers selected from the group of polymorphisms (mutations) in the NR1116 marker region consisting of
   a) the single nucleotide polymorphism marker having a G at the position corresponding to position 85 in SEQ ID NO:3,
   b) the single nucleotide polymorphism marker having an A at the position corresponding to position 87 in SEQ ID NO:3,
   c) the single nucleotide polymorphism marker having a T at the position corresponding to position 139 in SEQ IO NO:3,
   d) the single nucleotide polymorphism marker having a T at the position corresponding to position 214 in SEQ ID NO:3,
   e) the single nucleotide polymorphism marker having a T at the position corresponding to position 218 in SEQ ID NO:3,
   f) the single nucleotide polymorphism marker having a A at the position corresponding to position 277 in SEQ ID NO:3,
   g) the single nucleotide polymorphism marker having a G at the position corresponding to position 286 in SEQ ID NO:3,
   h) the single nucleotide polymorphism marker having an A at the position corresponding to position 312 in SEQ ID NO:3,
   i) the single nucleotide polymorphism marker having a C at the position corresponding to position 319 in SEQ ID NO:3,
   j) the single nucleotide polymorphism marker having a T at the position corresponding to position 359 in SEQ ID NO:3,
   k) the insertion mutation 5'-TTGGTGAACAATC-3' at the position corresponding to 221 in SEQ ID NO:3,
   I) the deletion mutation 5'-GAA-3' at the position corresponding to 328-330 in SEQ ID NO:3,
II. the markers selected from the group of polymorphisms (mutations) in the NR2525 marker region consisting of
   a) the single nucleotide polymorphism marker having a C at the position corresponding to position 60 in SEQ ID NO: 6,
   b) the single nucleotide polymorphism marker having a C at the position corresponding to position 92 in SEQ ID NO:6,
   c) the single nucleotide polymorphism marker having a C at the position corresponding to position 105 in SEQ ID NO: 6,
   d) the single nucleotide polymorphism marker having an A at the position corresponding to position 158 in SEQ ID NO: 6,
   e) the single nucleotide polymorphism marker having a C at the position corresponding to position 431 in SEQ ID NO: 6,
   f) the single nucleotide polymorphism marker having a T at the position corresponding to position 82 in SEQ ID NO: 6,
   g) the insertion mutation 5'-TGAGCAAAA-3' at the position corresponding to position 17 to 25 in SEQ ID NO:6,
III. the markers selected from the group of SNP markers consisting of
   a) a positive signal in a SNP assay using a SNP-probe (SNP-Probe 2) comprising the nucleotide sequence as described by SEQ ID NO: 11 (fertile allele specific probe HiNK6700), and - preferably - a negative signal in a SNP assay (preferably a TaqMan® based SNP assay) using a SNP-probe (SNP-Probe 1) comprising the nucleotide sequence as described by SEQ ID NO: 12 (sterile allele specific probe HiNK6701), and
   b) a positive signal in a SNP assay using a SNP probe (SNP-Probe 4) comprising the nucleotide sequence as described by SEQ ID NO: 18 (fertile allele specific probe HiNK6776), and - preferably - a negative signal in a SNP assay (preferably a TaqMan® based SNP assay) using a SNP probe (SNP-Probe 3) comprising the nucleotide sequence as described by SEQ ID NO: 17 (sterile allele specific probe HiNK6775),
IV. the markers selected from the group of SSR markers consisting of
   a) a PCR fragment with an apparent molecular weight selected from the group of apparent weights consisting of 94 (+/- 0.9) bp, 110.4 (+/- 0.5) bp, 112.3 (+/- 0.4) bp, and 116.3 (+/- 0.4) bp resulting from a PCR reaction with the primers having the sequence set forth as SEQ ID NOs: 1 and 2,
   b) a PCR fragment with an apparent molecular weight of 183.8 (+/- 0.4) bp or no fertile allele associated PCR fragment resulting from a PCR reaction with the primers having the sequence set forth as SEQ ID NOs: 4 and 5,
wherein the one or more marker (Ms allele marker) also includes an isolated nucleotide sequence selected from the group consisting of sequences which
I. have a sequence identity of at least 80% to, or
II. hybridize under stringent conditions to, or
III. comprise at least 25 consecutive nucleotides of
the marker sequences defined above in I. to IV.

Preferably, there are at last 1, 2, 3, 4, 5, 6, 7, 8, 9, 10, 11, or 12 of the mutations of group I., more preferably at least the mutations at the positions corresponding to position 214 (T/C) and 218 (T/G) in SEQ ID NO: 3. Preferably, there are at last 1, 2, 3, 4, 5, 6, or 7 of the mutations of group II., more preferably at least the mutations at the position corresponding to position 158 in SEQ ID NO: 6. It has to be noted, that the markers associated to the ms allele are - in contrast to the Ms allele - much more diverse. As a consequence, there might be one or more bands with different apparent molecular weights as indicated above. The reason is rather simple: While the Ms allele resulted from one single germplasm source which was not submitted to frequent subsequent genetic modifications, the ms allele is a *Brassica napus* "natural allele" which is present in different genetic backgrounds and its genetic environment was affected by numerous breeding activities. Thus, a significantly higher variability of the associated markers is available.
As already stated above, the markers may be used in various other aspects of the present invention. However, these aspects of the present invention are not limited to the use of the markers as disclosed in the application. It is emphasized that these aspects may also make use of markers not explicitly disclosed herein or markers yet to be identified.

Further, it has to be understood that although the fertility allele (dysfunctional male sterility allele; ms allele or ms) is obtainable from the *Brassica napus* seed deposited under Deposit Number NCIMB 41481 other sources may exist from which said ms allele can be obtained. In fact, the presence of the ms allele is rather rule than exception and should be present in virtually all *Brassica napus* germplasm (otherthan germplasm derived from the Takagi germplasm).
In a preferred embodiment of the present invention the seed deposited under Deposit number NCIMB 41481 are used for obtaining the fertility allele (ms allele as defined above).

The fertility allele (dysfunctional male sterility allele; ms allele) in a homozygous form is linked to a male fertile phenotype. In a heterozygous form (i.e., in combination with the Ms allele) it allows the male sterile phenotype caused by the Ms to be expressed in the presence of a homozygous rf allele (rfrf; or - in other words - in absence of the Rf allele). Said ms allele is present in any non-Takagi based germplasm. There are no known lines other than a germplasm derived from the Takagi germplasm which would not comprise at least one function copy of the ms allele. Thus, the fertility allele (dysfunctional male sterility allele; ms allele) is linked in a homozygous form to a male fertile phenotype,
wherein said ms allele is preferably selected from the group consisting of
a) the ms allele as present in the *Brassica napus* seed deposited under Deposit Number NCIMB 41481, and
b) variants thereof, which have the same phenotypic property.

In a preferred embodiment of the present invention said male fertile *Brassica napus* plant with the genotype msmsrfrf is suitable as a maintainer line in the production process for basic female seed of the present invention. Another embodiment relates to the use of said plant in a hybrid seed production process. Preferably, the genetic background of said plant is not a hybrid, more preferably it is an inbred.

### 3. Hybrid seed production

Another embodiment of the present invention relates to a method for producing male fertile hybrid seed of *Brassica napus,* said method comprising the steps of
a) providing as a female plant a conditionally male sterile *Brassica napus* plant with the genotype Msmsrfrf (basic mother) or MsMsrfrf (prebasic female), wherein said female conditionally male sterile *Brassica napus* plant is
   i. heterozygous (basic mother) or homozygous (prebasic female) for the male sterility allele (Ms allele) obtainable from the *Brassica napus* seed deposited under Deposit Number NCIMB 41480, and
   ii. homozygous for the dysfunctional restorerallele (rf allele) obtainable from the *Brassica napus* seed deposited under Deposit Number NCIMB 41480 or 41481, and
   iii. predominantly male sterile when exposed before and/or during flowering to a temperature of less than 28°C, and
   iv. reverting to a predominantly male fertile phenotype when exposed before and/or during flowering to a temperature of higher than 35°C, and
b) providing as a male plant a male fertile *Brassica napus* plant ("Restorer line" as defined below) with the genotype RfRf, wherein said male fertile *Brassica napus* plant is
   i. homozygous for the functional restorer allele (Rf allele), which is obtainable from any fertile, inbred *Brassica napus* line commercialized as seed for growing, and
   ii. predominantly male fertile, and
c) allowing the male plant of step b) to pollinate the female conditionally male sterile plant of step a), letting the seed develop, and harvesting said male fertile hybrid seed.

In a preferred embodiment the conditionally male sterile *Brassica napus* plant provided as female plant in step a) of the method for producing male fertile hybrid seed of *Brassica napus* described above has the genotype Msmsrfrf (i.e. is the basic mother as described hereinabove). As a consequence thereof, the female plant provided in step a) is preferably heterozygous for the male sterility allele (Ms allele).
In a further preferred embodiment the conditionally male sterile *Brassica napus* plant provided as female plant in step a) of the method for producing male fertile hybrid seed of *Brassica napus* described above is further characterized as being
I. predominantly male sterile when exposed before and/or during flowering (see iii. under step a) above) to a temperature of preferably less than 25°C, more preferablyto a temperature of less than 20°C, but at least at a temperature, which allows for normal growing such as at least 12°C, preferably at least 14°C, more preferably at least 16°C, and
II. reverting to a predominantly male fertile phenotype when exposed before and/or during flowering to a temperature of preferably between 35°C and 43°C. more preferably to a temperature of between 37°C and 40°C, most preferably to a temperature of about 39°C; preferably with an exposure for the preferred heat treatment time as specified herein and a subsequent growing at ambient temperature, as defined herein (see iv. under step a) above).
In a further embodiment, the conditionally male sterile *Brassica napus* plant provided as female plant in step a) of the method for producing male fertile hybrid seed of *Brassica napus* described above is further characterized as having a total glucosinolate content of not more than 25 µmol per gram of air dry seed at 9% humidity, preferably between 1 and 22 µmol, more preferably between 5 and 20 µmol, most preferably between 8 and 17 µmol per gram.

In a preferred embodiment of the method for producing male fertile hybrid seed of *Brassica napus,* the conditionally male sterile *Brassica napus* plant with the genotype Msmsrfrf (basic mother) or MsMsrfrf (prebasic female) provided as a female plant in step a) is grown from seed that has been produced using one of the methods described above, such as the method for producing or multiplying seed of a conditionally male sterile *Brassica napus* line with the genotype MsMsrfrf for the prebasic female consisting of the steps of
a) providing a conditionally male sterile *Brassica napus* plant with the genotype MsMsrfrf, which genotype is present in the *Brassica napus* seed deposited under Deposit Number NCIMB 41480, wherein said conditionally male sterile *Brassica napus* plant is
   i. homozygous for the male sterility gene (Ms allele) obtainable from the *Brassica napus* seed deposited under Deposit Number NCIMB 41480, and
   ii. homozygous for the maintainer allele (dysfunctional restorer allele; rf allele) obtainable from the *Brassica napus* seed deposited under Deposit Number NCIMB 41480 or 4148, and
   iii. predominantly male sterile when exposed before and/or during flowering to a temperature of less than 28°C, and
   iv. reverting to a predominantly male fertile phenotype when exposed before and/or during flowering to a temperature of higher than 35°C, and
b) exposing said conditionally male sterile *Brassica napus* plant for at least 4 hours to a temperature of higher than 35°C, and
c) exposing the heat-treated conditionally male sterile *Brassica napus* plant obtained in step (b) to a temperature of less than 33°C until development of male fertile flowers, and
d) allowing for self pollination of the *Brassica napus* plants having said male fertile flowers obtained in step (c), letting the seed develop, and harvesting the seed, wherein the harvested seeds are characterized in that they are seeds of a conditionally male sterile *Brassica napus* line with the genotype MsMsrfrf,
or - for the basic mother - using the preferred embodiment of the method for producing seed of a conditionally male sterile *Brassica napus* line with the genotype Msmsrfrf described above, wherein the conditionally male sterile (pre-basic) *Brassica napus* plant with the genotype MsMsrfrf used as a female plant and provided in step a) of this method is grown from seed that has been produced using the method described above for producing or multiplying seed of a conditionally male sterile *Brassica napus* line with the genotype MsMsrfrf, i.e. by
a) providing a conditionally male sterile *Brassica napus* plant with the genotype MsMsrfrf, which genotype Is present in the *Brassica napus* seed deposited under Deposit Number NCIMB 41480, wherein said conditionally male sterile *Brassica napus* plant is
   i. homozygous for the male sterility gene (Ms allele) obtainable from the *Brassica napus* seed deposited under Deposit Number NCIMB 41480, and
   ii. homozygous for the maintainer allele (dysfunctional restorer allele; rf allele) obtainable from the *Brassica napus* seed deposited under Deposit Number NCIMB 41480 or 4148, and
   iii. predominantly male sterile when exposed before and/or during flowering to a temperature of less than 28°C, and
   iv. reverting to a predominantly male fertile phenotype when exposed before and/or during flowering to a temperature of higher than 35°C, and
b) exposing said conditionally male sterile *Brassica napus* plant for at least 4 hours to a temperature of higher than 35°C, and
c) exposing the heat-treated conditionally male sterile *Brassica napus* plant obtained in step (b) to a temperature of less than 33°C until development of male fertile flowers, and
d) allowing for self pollination of the *Brassica napus* plants having said male fertile flowers obtained in step (c), letting the seed develop, and harvesting the seed, wherein the harvested seeds are characterized in that they are seeds of a conditionally male sterile *Brassica napus* line with the genotype MsMsrfrf.

The term **"Restorer line"** means any Brassica plant (preferably any *Brassica napus* plant) which is homozygous for the Rf gene (i.e. with a genotype RfRf). Regarding the Ms gene such plant may have any combination of the ms and Ms allele, although preferably it has a msms genotype, since this is the "natural" fertile allele phenotype. As a consequence, the Restorer line may have a genotype selected from the group consisting of msmsRfRf, MsmsRfRf, and MsMsRfRf. Preferably, the Restorer line is homozygous for the fertility allele (dysfunctional male sterility allele; ms allele), which preferably is obtainable from any fertile, inbred *Brassica napus* line commercialized as seed for growing or from the seed as deposited under Deposit Number NCIMB 41481.
In another embodiment, the male fertile *Brassica napus* plant with the genotype RfRf provided in step b) of the method for producing male fertile hybrid seed of *Brassica napus* described above is further characterized as having a total glucosinolate content of not more than 25 µmol per gram of air dry seed at 9% humidity, preferably between 1 and 22 µmol, more preferably between 5 and 20 µmol, most preferably between 8 and 17 µmol per gram.
In the most preferred embodiment the seeds developed in step c) are allowed to develop until maturity before harvesting same.

In a less preferred embodiment of the present invention, the Restorer line is a male male fertile *Brassica napus* plant with the genotype Rfrf, wherein male fertile *Brassica napus* plant is
i. heterozygous for the functional restorer allele (Rf allele), which preferably is obtainable from any fertile, inbred *Brassica napus* line commercialized as seed for growing, and
ii. predominantly male fertile, and
iii. optionally having a total glucosinolate content of not more than 25 µmol per gram (preferably between 1 and 22 µmol, more preferably between 5 and 20 µmol, most preferably between 8 and 17 µmol per gram) of air-dry seed at 9% humidity.

In a preferred embodiment of the present invention the seed deposited under Deposit number NCIMB 41480 and/or NCIMB 41481, respectively, is used in a method for producing fertile hybrid seed of *Brassica napus* as described herein.

Preferably, the male fertile line (maintainer line) and the female male sterile line (basic mother or prebasic female, preferably the basic mother) employed in the production of the hybrid seed are based on genetically diverse backgrounds. Genetic distance can be measured by the use of molecular markers as described for example in Knaak (1996).
In one preferred embodiment the production of the hybrid seed according to the present invention can be realized by growing the respective female (male sterile) and the male fertile plants in alternating stripes, where the pollinator (the male fertile line) is discarded after pollination. Good pollination conditions are preferred for a sufficient yield on the female line. The relation of mother (male sterile female) plant and pollinator should be preferably 3:1, and 3 to 5 bee hives per hectare are useful if production is performed in fields. The seed production in alternating strips is preferred to get high hybridity levels. Nevertheless, it is also possible (although not yet accepted in some countries) to produce hybrid seed by mixing female (mother) line and pollinator (male line). The advantage of mixed production is a reduction of production costs.
It has been found in the course of the present invention that the male fertile plants are earlier in the flowering as compared to the male sterile female plants employed in these production steps. To allow for optimal pollination it is therefore preferred that the male fertile plant is cut-back to delay flowering until flowering of the male sterile female plant. It is preferred that the production of the basic female seed and/or hybrid seed is conducted at a temperature of less than 33°C, preferably less than 28°C, more preferably less than 25°C.

In a further preferred embodiment of the present invention the production of male fertile hybrid seed of *Brassica napus* is carried out without making use of the heat mediated selfing of the male sterile plants. In this embodiment male fertile plants with the genotype MsmsRfrf are selfed as known in the art. Plants with the genotype MSmsRFrf can be obtained by crossing of RHS female with normal rapeseed and subsequent selfing by identifying the presence of the desired genes with markers (such as the markers of the present invention). These plants could also be obtained by backcrossing and marker analysis in every generation to keep only those plants which are heterozygous for both the Ms and the Rf gene. It is even possible to select plants with the genotype MsMsrfrf from heat mediated selfings of male sterile plants (as described above for the general RHS development) and cross them with a normal msmsRfRf rapeseed line variety. These two lines should be near isogenic to obtain a pure line for the further development. In the segregating progeny male sterile plants (having the genotypes Msmsrfrt and MsMsrfrf) and male fertile plants (having the genotypes MsMsRfRf, MsmsRfRf, msmsRfRf, MsMsRfrf, MsmsRfrf, msmsRfrf, or msmsrfrf) are expected. The male fertile plants with the genotypes MsMSRfrf and msmsrfrf are obtained by selection making use of the using closely linked molecular markers as described hereinabove. These plants are selfed as known in the art.
The descendants of the male sterile MsMsRfrf plants are sown in the field as females in alternating stripes with the descendants of the male fertile msmsrfrf plants (referred to herein as maintainer). The plants in the female stripes will segregate in male fertile phenotypes having the genotypes MsMsRfRf or MsMsRfrf and male sterile phenotypes having the genotype MsMsrfrf in the segregation ratio 3 fertile : 1 sterile. As already described above, male sterile plants are phenotypically characterized by abortion of the first buds of the inflorescence and white striped or blotched petals. Further, male sterile plants start flowering significantly later than the near isogenic male fertile plants. These phenotypic differences allow the removal of male fertile (early flowering) plants before the male sterile plants start flowering. The removal of the entire male fertile plants has to be done before the male sterile plants begin flowering to avoid cross pollination between male fertile plants (e.g. the maintainer) and male sterile plant in the hybrid production both originating from the selfing of the male fertile MsmsRfrf plants. Cutting back of the male fertile plants will not be sufficient, as this only delays the flowering of the male fertile plants and still allows the undesirable cross pollination mentioned before. Once the male fertile plants are removed before flowering, the male sterile plants remaining in the field are pollinated by the male fertile restorer plant (preferably grown in alternating stripes as described above) for F₁ hybrid seed production.
Seed is only obtained from the male sterile MSMSrfrf plants grown in the female stripe and will have the genotype Msmsrfrf; plants grown from these seeds will be male sterile. This completely male sterile population is a prerequisite to produce large amounts of hybrid seed. However, this kind of production of the basic seed requires small scaled fields so that roughing of male fertile plants is possible.

Depending on the pollinator used for pollinating the plants with the genotype MsmsRfrf in the method described above it is generally possible to produce (1) seed of the basic mother (using the maintainer with the genotype msmsrfrf as pollinator) or (2) F₁ hybrid seed (using the restorer with the genotype msmsRfRf as pollinator). However, following this approach for the production of F₁ hybrid seed is less efficient since large amounts of F₁ hybrid seed are required. Without having a pure male sterile mother at hand the plants with the genotype MsmsRfrf have to be selected by discarding 75 % of the whole offsprings (male fertile plants) leading a great loss of seed producing plants. It is thus preferred to generate a pure male sterile mother line for the hybrid seed production.

In generating hybrid plants, it is preferred that both the basic female parent that is cross-bred with a restorer and the restorer itself have a glucosinolate level that is sufficiently low to ensure that the grain (or seed produced from growing) of the F₁ hybrid plants has glucosinolate levels within regulatory levels. The glucosinolate level of the seed harvested from the F₁ hybrid is roughly the average (or slightly (e.g., 10 to 20%) below the average) of the glucosinolate levels of both the female parent and the male parent. The glucosinolate level of the hybrid grain (F₂) is reflective of the genotype of the F₁ hybrid. For example, if the objective is to obtain hybrid grain (F₂) having a glucosinolate level of less than 25 µmol/gram (preferably less than 20 µmol/gram), and the male fertile parent (restorer) has a glucosinolate level of 15 µmol/gram, the female parent preferably has a glucosinolate level of less than 25 µmol/gram.

### 3.1 Restorer Lines and the Rf allele

The term "(functional) restorer allele", or "Rf allele" or "dysfunctional maintainer allele" means the allele with dominantly is capable of (i.e. the Rf allele is linked to and/or associated with one or more characteristic selected from the group consisting of)
a) restoring fertility in the F₁ plants obtained from crossing with the *Brassica napus* plant grown from seed deposited under Deposit Number NCIMB 41480, and
b) restoring fertility in the F₁ plants obtained from crossing with the *Brassica napus* plant grown from the seed obtained from crossing of the *Brassica napus* plant obtained from the seed deposited under Deposit Number NCIMB 41480 as a female male sterile plant and the *Brassica napus* plant obtained from the seed deposited under Deposit Number NCIMB 41481 as a male fertile plant.
The Rf allele is obtainable from any non-Takagi based germplasm. Other than a germplasm derived from the Takagi germplasm there are no known fertile, inbred *Brassica napus* lines, which would not comprise at least one functional copy of the Rf allele. As a consequence, virtually all available fertile, inbred *Brassica napus* lines at the date of the present invention are found to be restorer lines (i.e., comprise the Rf allele).
Thus, the restorer allele (Rf allele) is selected from the group consisting of the Rf alleles obtainable from any fertile, inbred Brassica napus line commercialized as seed for growing ('Restorer line") (excluding any hybrid lines or parental lines thereof), preferably from lines commercially available at the priority date of the present invention, more preferably a line selected from the group consisting of Bounty, Cyclone, Delta, Ebony, Garrison, Impact, Legacy, Legend, Profit, Quantum, Campala, Pollen, Grizzly, Expert, Aviso, NK Jetix, Oase, Smart, NK Fair, NK Nemax, Ladoga, Cooper, Billy, Lorenz, Aurum, Lilian, Californium, Lisek, Orkan, Winner, Licorne, Castille, Fortis, and fertile, inbred Brassica napus lines which have the before mentioned varieties in their pedigree. Suitable Restorer lines also include those on the OECD variety list of December 2006 (OECD List of varieties eligible for certification - 2006/2007; Dec. 2006; http: //www.oecd.org/ dataoecd/1/44/3399947.pdf; http://www.oecd.org/document/14/0,2340,en_2649_33909_248507-0_1_1_1_1,00.html), preferably non-hybrid lines commercialized as seed for growing (for oil production), more preferably those, which are not marked as "d" (inbred as long as they represent hybrid parental lines) or "b" (hybrid) on said OECD list

A preferred embodiment of the present invention relates to the use of any fertile inbred Brassica napus plant commercialized as seed for growing for obtaining the functional restorer allele (Rf allele) for use in the production of fertile hybrid seed of Brassica napus as disclosed herein.

The fertility restoring phenotype and/or the Rf allele is linked to and/or associated with one or more SSR marker ("Rf allele marker") which is the absence of the PCR fragment with an apparent molecular weight of 240.8 (+/- 0.4) bp resulting from a PCR reaction with the primers set forth as SEQ ID NOs: 19 and 20.
In this context it has to be noted, that it is possible for the marker associated with the Rf allele (in contrast to the rf allele) to be more diverse. As a consequence, there might be more bands with different apparent molecular weights. The reason is rather simple: While the rf allele resulted from one single germplasm source which was not submitted to frequent subsequent genetic modifications, the Rf allele is a *Brassica napus* "natural allele" which is present in different genetic backgrounds and its genetic environment was affected by numerous breeding activities. Thus, a higher variability of the associated markers is possible.

. In one preferred embodiment of the present invention the methods of multiplying the prebasic seed, production of basic female seed and the production of hybrid seed are conducted in an integrated production process. Thus, preferably, another embodiment of the present invention relates to a method for the production of *Brassica napus* hybrid seed which yields *Brassica napus* plants producing seeds (or grain (i.e., seeds for non-growing purpose); optionally, but preferably, with a total glucosinolate content of not more than 25 µmol per gram, preferably between 1 and 22 µmol, more preferably between 5 and 20 µmol, most preferably between 8 and 17 µmol per gram) of air-dry seed at 9% humidity), wherein said method comprises a method of propagating the prebasic female seed (as defined above), a method of producing the basic female seed (as defined above), and a method for the production of hybrid seed (as defined above).

A further preferred embodiment of the present invention is directed to the use of any fertile, inbred *Brassica napus* line commercialized as seed for growing ("Restorer line") carrying at least one "(functional) restorer allele", "Rf allele" or "dysfunctional maintainer allele" (i.e., "Restorer plants" or "Restorer lines" as described above) for the restoration of male fertility in the F₁ plants obtained from crossing with a conditionally male sterile *Brassica napus* plants of the present invention (see above).

### 4. Markers for the Ms, ms, rf, Rf alleles and use of marker techniques

Molecular markers can be used for the visualization of differences in nucleic acid sequences. This visualization is possible - for example - due to DNA-DNA hybridization techniques after digestion with a restriction enzyme (RFLP) and/or due to techniques using the polymerase chain reaction (e.g. STS, microsatellites, AFLP). The markers identified herein may be used in various aspects of the present invention as will be illustrated below. Aspects of the present invention are not limited to the use of the markers identified herein. It is stressed that the aspects may also make use of markers not explicitly disclosed herein or even yet to be identified.
Molecular markers, i.e. SNP and SSR, are used in the hybrid breeding program and in the development of the inbred lines used therein to follow the heritance of alleles (e.g., the Ms, ms, Rf, rf allele) or to estimate genetic distances of selected breeding lines. By marker assisted backcrossing of selected lines into the hybrid system of the present invention (or vice versa) the backcrossing steps can be reduced from five to three backcross generations.
There are several types of molecular markers that may be used in marker-based selection including restriction fragment length polymorphism (RFLP), random amplification of polymorphic DNA (RAPD), amplified restriction fragment length polymorphism (AFLP), single sequence repeats (SSR) and single nucleotide polymorphisms SNPs. RFLP involves the use of restriction enzymes to cut chromosomal DNA at specific short restriction sites, polymorphisms result from duplications or deletions between the sites or mutations at the restriction sites. RAPD utilizes low stringency polymerase chain reaction (PCR) amplification with single primers of arbitrary sequence to generate strain-specific arrays of anonymous DNA fragments. The method requires only tiny DNA samples and analyses a large number of polymorphic loci. AFLP requires digestion of cellular DNA with a restriction enzyme before using PCR and selective nucleotides in the primers to amplify specific fragments. One especially preferred method utilizes SSR marker analysis based on DNA micro-satellites (short repeated) sequences that are widely dispersed throughout the genome of eukaryotes, which are selectively amplified to detect variations in simple sequence repeats. Only tiny DNA samples are required for an SSR analysis. Also preferred are SNP markers, which use PCR extension assays that efficiently pick up point mutations. The procedure requires little DNA per sample. One or two of the above methods may be used In a typical marker-based selection breeding program.
The most preferred method for achieving amplification of nucleotide fragments that span a polymorphic region of the plant genome for marker assisted selection employs the polymerase chain reaction ("PCR") (Mullis et al., 1986), using primer pairs involving a backward primer and a forward primer that are capable of hybridizing to the proximal sequences that define a polymorphism in its double-stranded form. Alternative methods may be employed to amplify such fragments, such as the "Ligase Chain Redaction" ("LCR") (Barany, 1991), which uses two pairs of oligonucleotide probes to exponentially amplify a specific target. The sequences of each pair of oligonucleotides are selected to permit the pair to hybridize to abutting sequences of the same strand of the target. Such hybridization forms a substrate for a template-dependent ligase. As with PCR, the resulting products thus serve as a template in subsequent cycles and an exponential amplification of the desired sequence is obtained. LCR can be performed with oligonucleotides having the proximal and distal sequences of the same strand of a polymorphic site. In one embodiment, either oligonucleotide will be designed to include the actual polymorphic site of the polymorphism. In such an embodiment, the reaction conditions are selected such that the oligonucleotides can only be ligated together if the target molecule either contains or lacks the specific nucleotide that is complementary to the polymorphic site present on the oligonucleotide. Alternatively, the oligonucleotides may be selected such that they do not include the polymorphic site (see, WO 90/01069).
A further method that may alternatively be employed is the "Oligonucleotide Ligation Assay" ("OLA") (Landegren et al., 1988)). The OLA protocol uses two oligonucleotides that are designed to be capable of hybridizing to abutting sequences of a single strand of a target. OLA, like LCR, is particularly suited for the detection of point mutations. Unlike LCR, however, OLA results in "linear" rather than exponential amplification of the target sequence. Nickerson et al. have described a nucleic acid detection assay that combines attributes of PCR and OLA (Nickerson et al., 1990). In this method, PCR is used to achieve the exponential amplification of target DNA, which is then detected using OLA. In addition to requiring multiple, and separate, processing steps, one problem associated with such combinations is that they inherit all of the problems associated with PCR and OLA. Schemes based on the ligation of two (or more) oligonucleotides in the presence of a nucleic acid having the sequence of the resulting "di-oligonucleotide," thereby amplifying the di-oligonucleotide, are also known (Wu et al., 1989), and may be readily adapted to the purposes of the present invention.
In one embodiment, a molecular marker is a DNA fragment amplified by PCR, e.g. a SSR marker. In one embodiment, the presence or absence of an amplified DNA fragment is indicative of the presence or absence of the trait itself or of a particular allele of the trait. In one embodiment, a difference In the length of an amplified DNA fragment is indicative of the presence of a particular allele of a trait, and thus enables to distinguish between different alleles of a trait. In a specific embodiment of the present invention simple sequence repeat (SSR) markers are used to identify invention-relevant alleles in the parent plants and/or the ancestors thereof, as well as in the progeny plants resulting from a cross of said parent plants.

Simple sequence repeats are short repeated DNA sequences and are present in the genomes of all eukaryotes and consists of several to over a hundred repeats of 1 to 4 nucleotide motifs. Since the number of SSRs present at a particular location in the genome often differs among plants, SSRs can be analyzed to determine the absence or presence of specific alleles.
In one aspect, the present invention relates to oligonucleotide primers selected from the group of sequences described by SEO ID NOs: 1, 2, 4, 5, 7, 8, 9, 10, 11, 12, 13, 14, 15, 16, 17, 18, 19, and 20. These primers are preferably employed as either a pair of PCR oligonucleotide primers consisting of a forward primer and a reverse primer or as a SNP mutation-detecting probe. Preferably, the primer pair for the amplification of an SSR markers consists of the primers described by SEQ ID NOs: 1 and 2 (primer pair 1), SEQ ID NOs: 4 and 5 (primer pair 2), or SEQ ID NOs: 19 and 20 (primer pair 3). Preferably, the primer pair for the amplification of a SNP marker fragment (i.e., a fragment which comprises the SNP mutation) consists of the primers described by SEQ ID NOs: 8 and 10 (primer pair 4), or SEQ ID NOs: 15 and 16 (primer pair 5). Other primer pairs provided herein are also potentially suitable as marker sequences (e.g., for RFLP markers), such as the primers described by SEQ ID NOs: 13 and 14 (primer pair 6), or SEQ ID NOs: 7 and 8 (primer pair 7). A probe suitable for the detection of a single nucleotide polymorphism (SNP) may comprise as the nucleic acid part a sequence selected from the group of sequences described by SEQ ID NOs: 11, 12, 17, and 19.

The regions comprising the SSRs and the SNPs have been sequenced. There are substantial mutational differences between those sequences which are linked to the respective alleles (e.g., the MS allele, ms allele, Rf allele, rf allele) and the associated phenotypes. As a consequence thereof, those regions present an inventive feature of the present invention because they allow for identification of further markers and/or the development of primers for the sequencing of the adjacent genome, which may comprise the respective MS allele, ms allele, Rf allele, rf allele. For sequence comparison the varying regions for sterile and fertile plants have been aligned to create a consensus sequence (SEQ ID NO: 3 and 6, respectively). This sequence can be preferably employed in detecting corresponding sequences in so far not analyzed *Brassica napus* germplasm. As a consequence, another embodiment of the present invention relates to an isolated nucleotide sequence selected from the group consisting of the sequences described by SEQ ID NOs: 3, 6, and 21.

The sequences disclosed in the present invention are especially useful in marker assisted breeding and selection. However, these sequences may be used in various other aspects of the present invention which are not limited to the use of the markers as described in the present application. It is emphasized that the present invention may also make use of sequences not explicitly disclosed herein or sequences yet to be identified.

With regard to marker-assisted selection another embodiment of the present invention relates to a method of using a nucleic acid sequence of the present invention for introgressing alleles selected from the group consisting of the Ms allele, ms allele, Rf allele, and/or rf allele into a Brassica germplasm lacking said set of alleles. A further preferred embodiment of the present invention relates to the use of nucleic acid sequences according to the present invention in marker-based selection for introgressing alleles selected from the group consisting of the Ms allele, ms allele, Rf allele, and/or Rf allele into a Brassica germplasm lacking said set of alleles as described above.

The plants of the present invention conferring male sterility or maintaining same may for instance have the genotype MsMsrfrf, Msmsrfrf, or msmsrfrf, while the hybrid plants of the invention have the genotype of MsmsRfrf, or msmsRfrf, wherein Ms, ms, rf, Rf have the meaning as described above. The present invention thus also provides methods for selecting a plant of the species *Brassica napus* exhibiting a male sterility conferring or maintaining phenotype by detecting in said plant the presence of the Ms and/or rf allele as defined herein. In a preferred method of the present invention for selecting such a plant the method comprises:
i) obtaining plant material from a plant or a plant population to be tested and extracting DNA from said material;
ii) analyzing the DNA sample obtained in step i) to determine the presence / absence of the Ms allele, ms allele, rf allele and/or Rf allele by using one or more of nucleic acid sequences of the present invention.
More preferably, step ii) of the above method comprises detecting in said sample of genomic DNA at least one molecular marker linked to a Ms allele, ms allele, rf allele, or Rf allele, more preferably detecting at least two molecular markers from said group wherein one marker detects the Ms allele or ms allele and the other marker detects the rf allele or Rf allele. The analysis may be conducted in various ways and may comprise for example the following steps:
a) identifying the at least one marker locus using a pair of PCR oligonucleotide primers consisting of a forward primer and a reverse primer exhibiting a nucleotide sequence set forth in any of SEQ ID NOs: 1, 2, 4, 5, 7, 8, 9, 10, 11, 12, 13, 14, 15, 16, 17, 8, 19, or 20, and
b) identifying the marker allele by determining the molecular weight of the PCR amplification product obtained in step a), and
c) identifying a plant or plants with the desired profile using the data of the marker analysis.
DNA samples can be obtained from suitable plant material such as leaf tissue by extracting DNA using known techniques. In a preferred embodiment the step of detecting a molecular marker (step b) may comprise the use of a set of bi-diredional primers that were used in the SSR method to produce the amplification product that later proved to be a suitable marker for the Ms, ms, Rf, or rf allele. Such a set of primers is herein referred to as the primers that define the SSR marker or marker-specific primers. "Bi-directional" means that the orientation of the primers is such that one functions as the forward and one as the reverse primer in an amplification reaction of nucleic acid. The step of detecting a molecular marker (step b) may also comprise the performance of a nucleic acid amplification reaction on said genomic DNA to detect one or more of the Ms, ms, Rf, or rf allele. This can suitably be done by performing a PCR reaction using a set of marker-specific primers. In a preferred embodiment, said step b) comprises the use of at least one set of primers defining an SSR marker for said alleles, or a set of primers which specifically hybridize under stringent conditions with a nucleic acid sequence of an SSR marker for said alleles.
Primers that flank a region containing SSRs or SNPs linked to an allele of the invention disclosed herein are then used to amplify the DNA sample using the polymerase chain reaction (PCR) method well-known to those skilled in the art. Basically, the method of PCR amplification involves use of a pair of primers comprising two short oligonucleotide primer sequences flanking the DNA segment to be amplified. Repeated cycles of heating and denaturation of the DNA are followed by annealing of the primers to their complementary sequences at low temperatures, and extension of the annealed primers with DNA polymerase. The primers hybridize to opposite strands of the DNA target sequences. Hybridization refers to annealing of complementary DNA strands, wherein complementary refers to the sequence of the nucleotides such that the nucleotides of one strand can bond with the nucleotides on the opposite strand to form double stranded structures. The primers are oriented so that DNA synthesis by the polymerase proceeds bidirectionally across the nucleotide sequence between the primers. This procedure effectively doubles the amount of that DNA segment in one cycle. Because the PCR products are complementary to, and capable of binding to, the primers, each successive cycle doubles the amount of DNA synthesized in the previous cycle. The result of this procedure is exponential accumulation of a specific target fragment, the factor of which is approximately 2n, wherein n is the number ofcycles. Through PCR amplification millions of copies of the DNA segment flanked by the primers are made.
For SSRs, differences in the number of repeated sequences between the flanking primers in different alleles are reflected in length variations of the amplified DNA fragments. These variations can be detected by electrophoretically separating the amplified DNA fragments on gels. By analyzing the gel it can be determined whether the plant contains the desired allele in a homozygous or heterozygous state or whether the desired allele is absent from the plant genome.
Marker analysis can be done early in plant development using DNA samples extracted from leaf tissue of very young plants. This allows to identify plants with a desirable genetic make-up early in the breeding cycle and to discard plants that do not contain the desired, invention-relevant alleles prior to pollination, thus reducing the size of the breeding population. Further, by using molecular markers, a distinction can be made between homozygous plants that carry two copies of the desired, invention-relevant allele and heterozygous plants that carry only one copy.
The step of detecting an amplified DNA fragment having the predicted length or the predicted nucleic acid sequence may be performed by standard gel-electrophoresis techniques or by using automated DNA sequencers. The methods need not be described here as they are well known to the skilled person.
The markers of the present invention can also be used to map the alleles of the present invention to certain locations in the *Brassica napus* genome. In general, the location of a certain trait gene (e.g., the Ms or rf allele) can be indicated by a contiguous string of markers that exhibit statistical correlation to the phenotypic trait. Once a marker is found outside that string (i.e. one that has a LOD-score below a certain threshold, indicating that the marker is so remote that recombination in the region between that marker and the gene (or allele) occurs so frequently that the presence of the marker does not correlate in a statistically significant manner to the presence of the phenotype) the boundaries of the gene (or allele) are set. Thus, it is also possible to indicate the location of the gene (or allele) by other markers located within that specified region.
It has to be noted (as indicated above) that, when said alleles of the present invention (e.g., the Ms allele or the rf allele) are introgressed into another genetic background (i.e. into the genome of another plant species or another *Brassica napus* variety), some markers may no longer be found in the offspring although the trait is present therein, indicating that such markers are outside the genomic region that represents the specific trait in the original parent line only and that the new genetic background has a different genomic organization. Such markers, the absence of which indicate the successful introduction of the genetic element in the offspring are called "trans markers" and may be equally suitable in MAS procedures in the present invention.
The nucleotide sequence of the Ms or rf allele of the present invention may for instance be resolved by determining the nucleotide sequence of one or more markers associated with said alleles (e.g., the nucleic acid sequences disclosed hereunder) and designing internal primers for said marker sequences that may then be used to further determine the sequence the gene outside of said marker sequences.
In embodiments of such methods for detecting the presence of a Ms allele, ms allele, Rf allele, or rf allele in a suspected male sterility referring or maintaining plant (or a hybrid plant), the method may also comprise the steps of providing an oligonucleotide or polynucleotide capable of hybridizing under stringent hybridization conditions to a nucleic acid sequence of a marker linkedto said Ms allele, ms allele, Rf allele, or rf allele, contacting said oligonucleotide or polynucleotide with digested genomic nucleic acid of said suspected plant, and determining the presence of specific hybridization of said oligonucleotide or polynucleotide to said digested genomic nucleic acid. Preferably, said method is performed on a nucleic acid sample obtained from said suspected plant, although in situ hybridization methods may also be employed. Alternatively, and in a more preferred embodiment, the skilled person may, once the nucleotide sequence of the Ms allele, ms allele, Rf allele, or rf allele has been determined, design specific hybridization probes or oligonucleotides capable of hybridizing under stringent hybridization conditions to the nucleic acid sequence of said Ms allele, ms allele, Rf allele, or rf allele and may use such hybridization probes in methods for detecting the presence of a Ms allele, ms allele, Rf allele, or rf allele of the present invention in a suspected *Brassica napus* plant.

Under certain circumstances it might be economically viable to replant seed harvested from hybrid *Brassica* plants (e.g., if those seeds yield high value specialty oils). Thus, the present invention further relates to a method of using a *Brassica napus* plant comprising the steps of harvesting seed from a *Brassica* hybrid plant of the present invention (or grown from the seed as provided by the method of producing hybrid seed of the present invention), and planting said seed to produce progeny. Preferably, said harvested seed has a glucosinolate content of not more than 25 µmol per gram (preferably between 1 and 22 µmol, more preferably between 5 and 20 µmol, most preferably between 8 and 17 µmol per gram) of air-dry seed at 9% humidity. More preferably, said replanted seed is at least heterozygous for the dysfunctional restorer allele (rt allele) or at least heterozygous for the male sterility allele (Ms allele), i.e., has a phenotype selected from group consisting of MsmsRfRf, MsMsRfrf, MsmsRfrf, msmsRfrf, and Msmsrfrf.
The method of replanting may be repeated and may thus include the step of repeating the step of planting the harvested seed of the progeny plants.
The present invention also relates to the use of a *Brassica napus* plant in a method comprising the steps of harvesting seed from a *Brassica* plant grown from the seed as provided by the methods of the present invention as provided herein and planting said seed to produce progeny. Preferably, this use further includes the step of repeating the step of planting the harvested seed of the progeny plants.

Also disclosed are oil producing processes using the hybrid seed of the present invention, especially the plants grown therefrom and/or the oil obtained thereof. Thus, one example is a method for producing rapeseed *(Brassica napus*) oil and meal (preferably meal that is essentially oil-free, i.e. has an oil content of less than 10%, preferably less than 5%, more preferably less than 2%) comprising the steps of
a) sowing a hybrid seed provided by the present invention or provided by the method of the present invention,
b) growing the hybrid *Brassica napus* plant from said seed,
c) harvesting the mature seed or grain from said plant, and
d) crushing said seed or grain and separating or extracting the oil from the meal (and preferably further comprising the step of separating the oil from the meal).
The method for producing oil and meal results in both products (oil and meal), which will be obtained as separate products from the process and have different utility. Oil is used in the food and feed industry for various purposes. The meal is used for feeding purposes or for the production of bio-gas. Representative uses of the meal include feed for livestock Representative uses of the oil include salad, frying, cooking, spraying, and viscous-food product applications. Handling and inventory considerations are greatly simplified since the endogenous vegetable meal and oil of the present invention fulfill the requirements for a wide variety of end uses. Each of these benefits is achieved in a straightforward manner in an endogenous product that inherently possesses superior health and nutritional properties.

**"Seed"** means the seed material harvested from the *Brassica napus* plants which is suitable and/or designated for further planting. **"Grain"** means the seed material harvested from the *Brassica napus* plants which is not suitable (commercially or practically) and/or not designated for further planting.

Further disclosed is the use of the hybrid seed of the present invention in such a process.
Another examples is a method for the production of *Brassica napus* (rapeseed) oil and meal comprising the steps of
a) providing rapeseed grain which is at least heterozygous for the dysfunctional restorer allele (rf allele) or at least heterozygous for the male sterility allele (Ms allele); and
b) crushing said seed and separating or extracting the oil.
In one preferred embodiment, the oil has a fatty acid profile selected from the group of preferred profiles as described below.
Yet another preferred embodiment of the present invention relates to the use of one or more of a *Brassica napus* plants of the present invention selected from the group consisting of a conditionally male sterile *Brassica napus* plant with the genotype MsMsrfrf (i.e. the prebasic female), a conditionally male sterile *Brassica napus* plant with the genotype Msmsrfrf (i.e. the basic mother) or a male fertile *Brassica napus* plant with the genotype msmsrfrf (i.e. the maintainer) in a method for producing hybrid seed. The method for producing hybrid seed is preferably one of the methods of the present invention as described above.
In a more preferred embodiment the present invention relates to the use of a conditionally male sterile *Brassica napus* plant of the present invention with the genotype MsMsrfrf (i.e. the prebasic female) and a male fertile *Brassica napus* plant of the present invention with the genotype msmsrfrt (i.e. the maintainer) in a method for producing hybrid seed. Again, the method for producing hybrid seed is preferably one of the methods of the present invention as described above.
In a further preferred embodiment the present invention relates to the use of one or more *Brassica napus* plants of the present invention selected from the group consisting of a conditionally male sterile *Brassica napus* plant with the genotype MsMsrfrf (i.e. the prebasic female) and a male fertile *Brassica napus*plant with the genotype msmsrfrf (i.e. the maintainer) in a method for producing a conditionally male sterile *Brassica napus* plant with the genotype Msmsrfrf (i.e. the basic mother) or seed thereof. The production of hybrid seed is preferably carried out as described above (e.g., as described in section 3).
In a more preferred embodiment the present invention relates to the use of a male fertile *Brassica napus* plant with the genotype msmsrfrf (i.e. the maintainer) in a method for producing a conditionally male sterile *Brassica napus* plant with the genotype Msmsrfrf. (i.e. the basic mother) or seed thereof. Again, the method for producing the conditionally male sterile *Brassica napus* plant with the genotype Msmsrfrf is preferably one of the methods of the present invention as described above.

In a preferred embodiment, the *Brassica napus* plant used in the method of using a *Brassica napus* plant of the present invention for producing hybrid seed is selected from a variety grown or derived from the *Brassica napus* seed deposited under Deposit Number NCIMB 41480 or 41481.

Another preferred embodiment the present invention relates to the use of fertile *Brassica napus* plant with the genotype RfRf in a method of providing fertile hybrid seed of *Brassica napus* as described above. Preferably, the fertile *Brassica napus* plant with the genotype RfRf is used in a method of providing fertile hybrid seed which is one of the methods of producing fertile hybrid seed of the present invention described above.

In a further preferred embodiment the present invention relates to the *Brassica napus* plants, seeds thereof or hybrid seeds as obtained by the use or in the methods of using described above.

### 6. Combination of the hybrid system with genetic backgrounds and other traits

Principally, the prebasic female, the basic female, the maintainer, and/or the hybrid plant can be combined with any genetic background of *Brassica napus.* However, preferably these plants (especially the hybrid plants) have one or more properties selected from the group consisting of yellow seed coat color, herbicide resistance, resistance against biotic stress (e.g., pathogen resistance against, for example, insects, fungi, bacteria, nematodes, virus, etc.), and resistance against abiotic stress (e.g., drought, salt, heat, frost, etc.). Additional traits which are commercially desirable are those which would reduce the cost of production of the *Brassica* crop (input traits) or which would increase the quality of the *Brassica* crop or the oil or meal derived therefrom (output traits). Input traits can be selected from the group consisting of herbicide resistance, insect resistance, disease resistance and stress resistance (such as drought, cold, heat, or salt resistance). Output traits can be preferably selected from specific desirable oil or fatty acid profiles.
Pathogen resistance traits include but are not limited to
a) monogenic Rlm7Phoma resistance *(Leptosphaeria maculans);* such trait is accessible from the *Brassica napus* varieties cv. Roxet, and cv. Caiman;
b) Clubroot resistance *(Plasmodiophora brassicae),* such trait is accessible from the *Brassica napus* varieties cv. Tosca, and cv. Mendel; and
c) TUYV virus resistance (Turnip Yellows Virus); such trait is accessible from the *Brassica napus* variety cv. Caletta
A person skilled in the art could use the *Brassica* plant of the present invention to develop a *Brassica* plant, which is a prebasic or basic female, a maintainer or a restorer of fertility for the nuclear male sterility, that produces oilseeds, which preferably have low glucosinolate content and any other desirable trait.
The meal and oil yielded from the plants and seeds may also have various properties depending on the intended use. Such use may be an industrial use or an use as feed or food. Use for food purposes may include use as frying oil, for the production of spreads, as cooking oil, or as salad oil. All these intended uses are linked to preferred fatty acid profiles. Preferably, the *Brassica* plants and/or the seed of said plants and/or the seed obtained from said plants are of canola quality.

### 6.1. Oil Profile Traits

Preferably, the prebasic, basic, maintainer, and hybrid plants yield a grain with an oil content of more than 40%, preferably of more than 42%, and most preferably of more than 44%.
The edible endogenous vegetable oil of the *Brassica* oilseeds contains fatty acids and other traits that are controlled by genetic means (WO91/15578; US 5,387,758). Preferably erucic acid of the *Brassica* oilseed intended for human or animal consumption is included in a low concentration of no more than 2 percent by weight based upon the total fatty acid content that is controlled by genetic means in combination with the other recited components as specified. The genetic means for the expression of such erucic acid trait can be derived from numerous commercially available canola varieties having good agronomic characteristics, such as, for example, Bounty, Cyclone, Delta, Ebony, Garrison, Impact, Legacy, Legend, Profit, Quantum, Campala, Pollen, Grizzly, Expert, Aviso, NK Jetix, Oase, Smart, NK Fair, NK Nemax, Ladoga, Cooper, Billy, Lorenz, Aurum, Lilian, Californium, Lisek, Orkan, Winner, Licome, Castille, Fortis.
In one preferred embodiment the hybrid *Brassica napus* plant, (or the basic, prebasic, or maintainer line of the present invention used for its breeding) yields a specialty oil profile, For a review of preferred specialty oil profiles in Brassicas see Scarth & Tang (2006) and the references cited therein.
Rapeseed oil produced by traditional *Brassica* oilseed cultivars typically has a fatty acid composition of 5% palmitic (C16:0), 1% stearic (C18:0), 15% oleic (C18:1), 14% linoleic (C18:2), 9% linolenic (C18:3), and 45% erucic acid (C22: 1) (Ackman, 1990). C22:1 is a nutritionally undesirable fatty acid and has been reduced to very low levels in *Brassica* oil for edible uses. Low C22:1 *Brassica* oil has a nutritionally desirable fatty acid profile, with low saturated fatty acids and significant levels of C18:3, an omega-3 fatty acid (Eskin et al., 1996). C22:1 does have significant value in industrial applications and, for these uses, it is desirable to increase the 45% level in traditional rapeseed oil as high as possible to improve the economical competitiveness of the high erucic acid rapeseed (HEAR) oil and its derivatives. In addition to the common fatty adds in *Brassica* oil, members of the plant kingdom produce more than 200 unusual fatty acids, particularly in non-agronomic plants (van de Loo et al., 1993; Thelen & Ohlrogge, 2002; Jaworski &Cahoon, 2003). Many of these fatty acids have nutritional benefits or industrial uses. However, most of these plants have limited potential of domestication. Because of the relatively low cost and renewable nature of oilseed production, oilseed crops including *Brassica* oilseeds can be modified to produce the novel fatty acids as an alternative source to petroleum-derived industrial feedstock (Cahoon, 2003; Thelen & Ohlrogge, 2002).
Conventional breeding has contributed to the development of four major types of *Brassica* oils with altered fatty acid compositions aimed for different markets (Burton et al., 2004; Przybytski, 2005). These developments were the result of plant breeding using natural and artificially induced mutations within *Brassica* species.
**Low Erucic Acid**: The selection and development of *Brassica* oils low in C22:1 was initiated in 1950s, following the identification of potential human health concerns in animal feeding studies with rapeseed oil high in C22:1. Animal studies showed that diets high in C22:1 rapeseed oil were associated with myocardial damage characterized by fatty deposits around the heart and the kidneys and muscle lesions in the heart. Mutants with low levels of C22:1 in the seed oil were first identified from a German spring-type *B. napus* forage cultivar Liho in 1959 (Stefansson et al., 1961). The low C22: 1 plants were backcrossed with adapted cultivars. In North America and Europe, there has been a complete conversion of the commercial production from traditional rapeseed and low erucic acid varieties to canola quality varieties.
**High Erucic Acid and Super High Erucic Acid**: Although being nutritionally undesirable, high C22:1 oils and C22:1 derivatives have more than 200 potential industrial applications, e.g., as an additive in lubricants and solvents, as a softener in textiles, and the amide derivative is used in the manufacture of polymers, high temperature fluidity lubricants, surfactants, plasticizers, surface coatings, and pharmaceuticals (Scarth & McVetty, 2006), and more than 1000 patents for applications of C22:1 have been issued (Mietkiewska et al., 2004). To compete with petroleum-based products, it is desirable to increase the C22:1 level to as high a level as possible to reduce the cost of purification. The first High Erucic Acid Rapeseed (HEAR) cultivars with a low glucosinolate content are available, such as cv. Hero (Scarth et al., 1981, 1992), cv. Mercury (54% C22:1; Scarth et al., 1995a), cv. Castor and MilleniUM01 (C22:1 55%; McVetty et al. 1998, 1999). Further suitable varieties with high erucic acid content are cv. Hearty, Maruca, Maplus. The goal of increasing the C22:1 level has been approached by resynthesizing *B. napus* by crossing selected lines of the two ancestral diploids, B. rapa and B. oleracea (Taylor et al., 1995). Resynthesized plants can accumulate levels of C22:1 to up to 60% (Lühs & Friedt, 1995). The genes and alleles involved in C22:1 synthesis are known (Scarth & McVetty, 2006), thus both directed (marker assisted breeding) end/or transgenic approaches to increase the C22:1 content are possible. Super high erucic acid rapeseed (SHEAR) oil with a greater than 80% C22:1 level is desired to reduce the cost of producing this fatty acid and its derivatives as a renewable, environment friendly industrial feedstock. The potential of using FAE genes from high-C22:1 accumulating species to increase C22:1 accumulation in Brassica seed oil was demonstrated by a 90% increase in the C22:1 level with the expression of a nasturtium (Tropaeolum majus L) FAE gene (Mietkiewska et al., 2004).
Low Linolenic Acid C18:3 and C18:2, together with their longer-chain and more unsaturated derivatives, are the two series of essential polyunsaturated fatty acids (PUFA) within the n-3 and n-6 fatty acids, respectively, required for human development and health. However, the increased number of double bonds in the chemical structures of the PUFA makes them susceptible to oxidation. Oxidation rates of C18:2 and C18:3 are approximately 10 and 25 times higher, respectively, than that of C18:1. Therefore, oils high in C18:2 and C18:3 deteriorate more rapidly on exposure to air, especially at high temperatures, resulting in shortened shelf life of the oil which makes the oil less healthy for human consumption. Normally, less stable oils are hydrogenated to enhance the stability, but the hydrogenation process causes the formation of trans-fatty acids, which cause concerns due to their physiological functions. In addition, hydrogenation increases the cost of processing commodity oils. The first low C18:3 canola varieties are available, such as cv. Stellar (3% C18:3), line 'M11' (Röbbelen & Nitsch, 1975), and cv. Apollo (Scarth et al., 1995b). The genes and alleles involved in C22:1 synthesis are known (Scarth & McVetty, 2006), thus both directed (marker assisted breeding) and/or transgenic approaches to increase the C22:1 content are possible.
**High and Very High Oleic Acid** Oils with high C18:1 and low C18:3 levels possess a higher oxidative stability without the requirement of partial hydrogenation and produce less undesirable products during deep frying. High oleic acid oils have equivalent heat stability to saturated fats and are suitable replacements for them in commercial food-service applications that require long-life stability. Lines with high oleic content of 80 to 90% C18:1 are described (Vilkki & Tanhuanpää, 1995; Rücker & Röbbelen, 1995; Schierholt & Becker, 1999; Wong et al., 1991). Commercially available are cv. Clear Valley 75 and MONOLA with 70 to 75% C18:1 and reduced C18:3 level (Scarth & McVetty, 1999). High C18:1 mutation has been associated to the fad2 gene in B. *napus* (Laga et al., 2004). In B. juncea, two OTLs together could account for 32.2% variation in the C18:1 level (Sharma et al., 2002). Considerable progress has been made in developing very high C18:1 oilseed by engineering of the Δ12-desaturase and FatB thioesterase. Transgenic *B. napus* could accumulate as high as 89% C18:1 with the PUFA fraction being reduced in the seed oils by sense or antisense Δ12-desaturase constructs (reviewed in Scarth & McVetty, 2006). Especially preferred are combinations of high oleic and low linoleic oil profiles which result in oils with very good frying quality. Such traits are present, for example, in the variety cv. Splendor.
**Low and Very Low Saturated Fatty Acids** C16:0 is the major contributor to the total saturated fatty acid level of vegetable oils including Brassica oil. C16:0, as well as the shorter chain fatty acids C8:0 to C14:0, are widely reported to raise plasma total cholesterol (TC) and low density lipoprotein cholesterol (LDL-C) levels in animals and humans. Canola oil is the only commercial vegetable oil meeting the criteria of the low saturated oils (<7%) as defined in the labeling regulations in the USA and Canada. It is desirable to further reduce the saturated fatty acid level to achieve zero saturated fat levels. Lines with further reduced levels of less than 6% are described (Raney et al., 1999). An alternative approach to "traditional" breeding for decreasing the saturation level is the regulation of the expression of a number of genes, including KAS, desaturases, and thioesterases (Dehesh, 2004; Scarth & McVetty, 2006). Transgenic Brassica with a total level of saturated fatty acids below 3.4%, compared to around 6.0% in non-transformed B. *napus* plants are described (Dehesh, 2004).

### High Levels of Short and Medium Chain Fatty Acids (High Lauric Acid, High Caprylic

**Acid and Capric Acid)** Plants oils rich in short and medium chain fatty acids (SMCFA) are useful in a number of food and nonfood industries. Current commercial sources of SMCFA are coconut and palm kernel oils. Brassica seed oil has traces of SMCFA with hardly detectable levels of C8:0, C10:0, and C12:0. By transgenic approaches employing various genes significant levels can be obtained. The transgenic plants produced seed oil with up to 56 mol% C12:0 (Voelker et al., 1996). The seed oil of the Bay-FatB transformed *B*. *napus* plants was similar to the composition of coconut and palm kernel oils in the level of SMCFA (Voelker et al., 1996), which are used in food products such as in chocolates, candy coatings, confections, nondairy creamers, low-fat margarines, soaps, detergents, and cosmetics. Up to 40% C8: 0, and C10:0 were obtained in Brassica following transformation with the Cuphea FatB2 thioesterase gene (Dehesh et al., 1996).
**High Palmitic Acid** Significant increases in the C16:0 level of seed oil were observed in transgenic plants expressing FatB thioesterase and KAS from MCFA-accumulating plantspecies. Transgenic plants expressing the Cuphea Ch FatB1 gene have a C16:0 level of up to 34 mol% (Jones et al., 1995). Similar levels of C16:0 were found in the seed oils of transgenic B. *napus* plants expressing FatB genes from elm (Ulmus americana L.) and nutmeg *(Myristica fragrans* Houtt.) (Voelker et al., 1997).
**High Stearic Acid** Vegetable oils with high saturated fatty acid levels have applications in the manufacture of solid fat food products, such as margarine and shortening, saving the cost of hydrogenation and avoiding the production of unwanted trans-fatty acid. C18:0 has an advantage over other forms of saturated fatty acids because it either reduces or has no effect on serum lipoprotein cholesterol. Canola cultivars have only 1.1 to 2.5% C18:0 In the seed oil. No natural or induced high C18:0 Brassica germplasm has been reported. However, the genes controlling the C18:0 level are described (reviewed in Scarth & Mc Vetty, 2006). B. *napus* cv. Westar producing seed oil with up to 10.1 % C18:0 is described (Hitz et al., 1995). Expression of a FatA gene from mangosteen increased the C18:0 level of *B. napus* cv. Quantum to more than 22% (Hawkins & Kridl, 1998). Expression of a mutated mangosteen FatA from site-specific mutagenesis led to a 55 to 68% increase in the C18:0 level compared to the wild-type FatA version (Facciotti et al., 1999). A second strategy is the overexpression of Δ9-desaturase, the enzyme which directs the carbon flux to C18:1-ACP production. A reduced Δ9-desaturase activity should result in higher C18:0 accumulation (Knutzon et al., 1992). Antisense suppression using *B. rapa* Δ9-desaturase gene increased the C18:0 level to greater than 32% in transgenic B. rapa and to 40% in *B. napus* (Knutzon et al., 1992), although sense suppression with a soybean Δ9-desaturase was not as effective in *B. napus* (Hitz et al., 1995). A third strategy is the simultaneous manipulation of the activities of the two enzymes. Overexpression of FatA thioesterase and downregulation of Δ9-desaturase increased the C18:0 level up to 45%, higher than separately expressing the FatA thioesterase transgene (11 % C18:0) and the Δ9-desaturase transgene (13% C18:0) (Töpfer et al., 1995). Downregulation of both FatA and FatB in *B. napus* and *B. juncea* with a dual silencing construct containing inverted repeats of the target genes was initiated (Pandian et al., 2004). An additional approach for developing high C18:0 Brassica oil could be the downregulation of the activities of both Δ9 and A12 desaturases, as shown in cottonseed engineered with a hairpin RNA silencing constructs for the two desaturases which increased cottonseed C18:0 level from 2 to 3% to 40% (Liu et al., 2002).
**Polyunsaturated Fatty Acids (PUFA)** Gamma-linolenic acid (GLA) is a PUFA in the n-6 family of essential fatty acids. GLA is one of nutritionally important polyunsaturated fatty acids in human and animal diet. Genes for expression of these fatty acids are described (reviewed in Scarth & McVetty, 2006). Very-Long-Chain Polyunsaturated Fatty Acids (VLCP-UFA) have 20 or 22 carbon atoms with fourto six interrupted double bonds, including fatty acids with important therapeutic and nutritional benefits in humans, such as arachidonic (ARA), eicosapentaenoic (EPA), and docosahexaenoic acid (DHA). Brassica species like other higher plants do not produce very long chain PUFAs such as ARA, EPA and DHA. GLA and ALA are not widely encountered in higher plants. To develop oilseeds rich in ARA and EPA, at least three genes have to be introduced (Abbadi et al., 2004). The feasibility of engineering ARA pathway was demonstrated in tobacco (Huang et al., 2004). Conjugated fatty acids are polyunsaturated fatty acids with double bonds which are not separated by a methylene unit. Oils rich in conjugated fatty acids (such as calendic acid) have superior properties as drying oils in coating applications. Expression of a gene from pot marigold (Calendula officinalis L.) encoding an enzyme that introduces conjugated double bonds into polyunsaturated fatty acids resulted In the accumulation of calendic acid to 20 to 25% of the total fatty acids in soybean oil (Cahoon et al., 2001). Epoxy fatty acids, such as vernolic acid, are produced by monooxy-genases and divergent forms of di-iron desaturases (Hatanaka et al., 2004) and are valuable raw materials for the production of resins, glues, plastics, polymers etc..
**Other Novel Fatty Acids "Novel"** or "unusual" fatty acids are defined broadly as fatty acids that have chemical structures different from those fatty acids commonly found in major oilseed crops (Jaworski & Cahoon, 2003). Unusual monoun-saturated fatty acids are produced by special desaturases which insert the double bond into an unusual position in the acryl chain, rather than between carbons 8 and 9 as seen in the common fatty acid C18:1. Plant oils rich in petroselinic or palmitoleic acid could be used as alternatives to petroleum in the production of biodegradable lubricants, surfactants, and plastic precursors.

### 7. General technologies

### 7.1 Trait and Gene Introgression

The Ms allele, ms allele, Rf allele, rf allele of the present invention (or any combination thereof) can be introgressed into any genetic background of *Brassica napus* and other *Brassica* varieties.

### Transgenic Technologies

A nucleic acid (preferably a DNA) sequence comprising the genomic sequence for the Ms and/or the rf allele may be used for the production of a transgenic hybrid system. Said nucleic acid sequence may be derived from the seed deposited under Deposit Number NCIMB 41480 or 41481. Also disclosed is a method of producing a male sterile *Brassica napus* plant with the genotype MsMsrfrf or a *Brassica napus* maintainer plant with the genotype msmsrfrt comprising the steps of performing a method for detecting the presence of the Ms allele and/or rf allele associated with male sterility and/or maintaining male sterility in a *Brassica napus* plant as described above, and transferring a nucleic acid sequence comprising at least one Ms allele or rf allele thus detected, or a male sterility conferring or maintaining part thereof, from said donor plant to a recipient plant (preferably a Brassica plant, more preferably a *Brassica napus* plant). The transfer of said nucleic acid sequence may be performed by any of the methods known in the art including Agrobacterium mediated gene transfer or microparticle mediated gene transfer.

### Non-Transgenic Technologies

A preferred embodiment of such a method comprises the transfer by introgression of said nucleic acid sequence from a basic female or maintainer *Brassica napus* plant (e.g., the plants derived from the seed deposited under Deposit Number NCIMB 41480 or 41481) by crossing said plants. This transfer may thus suitably be accomplished by using traditional breeding techniques.
In every generation, the presence or absence of the Ms allele and/or rf allele is determined. Due to the male sterile phenotype the presences or absence of the restorer allele can be easily detected in every generation, for example, by fertility scoring. After generation of the last backcross a setting step is preferred. In the following generation molecular markers are used as described in the present invention to select plants homozygous for the Ms allele and/or rf allele. These plants represent the prebasic female (MsMsrfrf) or the maintainer line (msmsrfrf), which can be used to produce hybrid seed.
Ms alleles and/or rf alleles are preferably introgressed into commercial *Brassica napus* varieties by using marker-assisted selection (MAS) or marker-assisted breeding (MAB) as described above. MAS and MAB involve the use of one or more of the molecular markers for the identification and selection of those offspring plants that contain one or more of the genes that encode for the desired trait. In the present instance, such identification and selection is based on selection of the Ms allele and/or rt allele or markers associated therewith. *Brassica napus* plants developed according to this embodiment can advantageously derive a majority of their traits from the recipient plant, and derive the male sterility conferring or maintaining property from the donor plant (i.e., the basic female or the maintainer line).
In one method, which is referred to as pedigree breeding, a donor *Brassica napus* plant that exhibits male sterility conferring or maintaining properties (e.g, a prebasic female plant or maintainer plant of the present invention) is crossed with a recipient *Brassica napus* plant lacking said characteristics but preferably exhibiting commercially desirable characteristics, such as, but not limited to, disease resistance, insect resistance, valuable oil and/or meal characteristics, etc. The resulting plant population (representing the F₁ hybrids) is then self-pollinated and allowed to set seeds (F₂ seeds). The F₂ plants grown from the F₂ seeds are then screened for male sterility conferring or maintaining properties. The population can be screened in a number of different ways. First, the population can be screened by evaluating sterility or fertility of the lines or their property to maintain sterility, respectively. Second, marker-assisted selection can be performed using one or more of the molecular markers described above to identify those progeny that comprise a Ms allele and/or rf allele. Other methods, referred to hereinabove by methods for detecting the presence of a Ms allele and/or rt allele by associated phenotypes, may be used. Also, marker-assisted selection can be used to confirm the results obtained from the quantitative bioassays and, therefore, several methods may also be used in combination.
Further disclosed is a method of introgressing the Ms allele and/or the rf allele comprising the steps of obtaining a Brassica plant containing the Ms allele and/or the rf allele, for example, the Brassica Inbred lines deposited under Deposit Number NCIMB 41480 or 41481, respectively, crossing this plant with another Brassica plant and selecting seed containing the Ms allele and/or rf allele. The resulting F₁ plants are crossed with the recurrent parent to replace more of the genome of the Brassica inbred line, particularly between 80 and 99.5% of the genome, more particularly between 90% and 99% of the genome, but especially between 95% and 98% of the genome. A plant comprising the Ms allele and/or the rf allele is at least backcrossed two times against the variety with the target genetic background. In the 2^{nd} backcrossing parallel selfing of the fertile female (comprising the Rf allele from the fertile variety with the target background) is done to obtain BC0S1 plants. Only plants which comprise the Ms and rf allele (resulting in sterile plants after selfing) are utilized in subsequent breeding steps. The process of selfing and parallel crossing is performed in each BC generation. Alternatively, the presence/absence of the Ms allele and/or rf allele can be traced with marker technology. Inbred male sterile basic female lines or maintainer lines can be developed using the techniques of recurrent selection and backcrossing, selfing and/or dihaploids or any other technique used to make parental lines. In a method of recurrent selection and backcrossing, the male sterility referring or maintaining genotype can be introgressed into a target recipient plant (the "recurrent parent") by crossing the recurrent parent with a first donor plant, which differs from the recurrent parent and is referred to herein as the "non-recurrent parent". The recurrent parent is a plant that is lacking the male sterility conferring or maintaining properties and preferably possesses commercially desirable characteristics, such as, but not limited to (additional) disease resistance, insect resistance, valuable oil or meal characteristics, etc. The non-recurrent parent exhibits male sterility conferring or maintaining properties and comprises a nucleic acid sequence that encodes the Ms allele and/or rf allele. The non-recurrent parent can be any plant variety or inbred line that is cross-fertile with the recurrent parent. The progeny resulting from a cross between the recurrent parent and non-recurrent parent are backcrossed to the recurrent parent. The resulting plant population is then screened for the desired characteristics, which screening may occur in a number of different ways. For instance, the population can be screened using phenotypic pathology screens or quantitative bioassays as known in the art. Alternatively, instead of using bioassays, marker-assisted selection (MAS) can be performed using one or more of the hereinbefore described molecular markers, hybridization probes or polynucleotides to identify those progeny that comprise a nucleic acid sequence encoding for the Ms allele and/or rt allele. Also, MAS can be used to confirm the results obtained from the quantitative bioassays. The markers defined herein are therefore ultimately suitable to select proper offspring plants by genotypic screening.
Following screening, the *Brassica napus* plants that exhibit a male sterility conferring or maintaining phenotype or, more preferably, genotype and thus comprise the requisite nucleic acid sequence encoding the Ms allele and/or rf allele are then selected and backcrossed to the recurrent parent for a number of generations in order to allow for the *Brassica napus* plant to become increasingly inbred. This process can be performed for two to five or more generations. In principle, the progeny resulting from the process of crossing the recurrent parent with the male sterility referring or maintaining non-recurrent parent are heterozygous for the Ms allele or rf allele. Homozygous plants can be obtained by selfing of this plants and assessing the genotype of the subsequent generation by either marker analysis or further selfing and monitoring of the phenotype segregation pattern.
In general, a method of introducing a desired male sterility conferring or maintaining trait into a *Brassica napus* variety comprises the steps of:
(a) crossing an inbred *Brassica napus* parent with another *Brassica napus* plant that comprises the Ms allele and/or the rf allele to produce F₁ progeny plants;
(b) selecting said F₁ progeny plants that have the desired Ms allele and/or rf allele to produce selected F₁ progeny plants, preferably using molecular markers as defined herein;
(c) backcrossing the selected progeny plants with said inbred *Brassica napus* parent plant to produce backcross progeny plants;
(d) selecting for backcross progeny plants that have the desired Ms allele and/or rf allele and morphological and physiological characteristics of said inbred *Brassica napus* plant, wherein said selection comprises the isolation of genomic DNA and testing said DNA for the presence of at least one molecular marker for the Ms allele and/or rf allele, preferably as described herein;
(e) repeating steps (c) and (d) for two or more times in succession to produce selected third or higher backcross progeny plants; and
(f) optionally selfing selected backcross progeny in order to identify homozygous plants.
As indicated, the last backcross generation may be selfed in order to provide for homozygous pure breeding (inbred) progeny for male sterility conferring or maintaining plants. Thus, the result of recurrent selection, backcrossing and selfing is the production of lines that are genetically homogenous for the Ms allele and/or rf allele as well as for other genes associated with traits of commercial interest.
In an alternative embodiment for producing a male sterile or maintainer Brassica plant protoplast fusion can be used for the transfer of the Ms allele or rf allele of the present invention to a recipient plant. By this means, the hybrid system of the present invention can be utilized in other species, preferably in other Brassica species, such as Brassica oleracea. Protoplast fusion is an induced or spontaneous union, such as a somatic hybridization, between two or more protoplasts (cells of which the cell walls are removed by enzymatic treatment) to produce a single bi- or multi-nucleate cell. The fused cell that may even be obtained from plant species that cannot be interbred in nature, is tissue cultured into a hybrid plant exhibiting the desirable combination of traits. More specifically, a first protoplast can be obtained from a *Brassica napus* plant (e.g., the plants derived from the seed deposited under Deposit Number NCIMB 41480 or 41481). A second protoplast can be obtained from a second *Brassica napus,* such as other Brassica species or other plant variety, preferably a Brassica line that comprises commercially valuable characteristics, such as, but not limited to disease resistance, insect resistance, valuable fruit characteristics, etc. The protoplasts are then fused using traditional protoplast fusion procedures which are known in the art.
Alternatively, embryo rescue may be employed in the transfer of a nucleic acid comprising the Ms and/or rf allele as described herein from a donor plant to a recipient plant. Embryo rescue can be used as a procedure to isolate embryos from crosses wherein plants fail to produce viable seed. In this process, the fertilized ovary or immature seed of a plant is tissue cultured to create new plants (Pierik, 1999).
Other techniques can be utilized additionally or alternatively in gene and trait introgressions. Such techniques can involve the use of double-haploid technology (Fletcher et al., 1998), which can significantly increase the speed of inbred generation for the prebasic and the maintainer line. For the prebasic line it is required to utilize a heat-treatment procedure because otherwise microspores become aborted.

### 7.2 Creating distinct gene pools

The utilization of a maximum heterosis effect and a long-term successful heterosis breeding requires parents with a high genetic difference. The narrow genetic basis of oilseed rape is a limiting factor of increasing hybrid performance on the long run. By introducing resynthesized genotypes (Girke, 2002) and using reciprocal recurrent selection procedures the genetic basis has to be extended step by step. Especially in winter-type canola a narrow genetic correlation (r=0,87) of the existing commercial varieties can be observed (Girke, 2002).
Parental lines with a genetic distance lower than a fixed minimum are not worthwhile to be tested in the field. The distance can be tested by isoenzyme analysis (Mündges et al., 1990) or more conveniently by markers (RFLP: Beckmann & Soller, 1983; Botstein et al., 1980; RAPD (Random Amplified Polymorphic DNA): Williams et al., 1990; Förster & Knaak, 1995). This reduces the work of yield trials.

### 7.3 Use in Breeding

The plants disclosed above can be used for various breeding activities including, but not limited to
a) Selfing Down Of Hybrids: Low glucosinolate hybrids containing the new Ms allele and/or rf allele of the present invention were grown. Fertile plants were self pollinated, some with bags, others by brushing pollen manually. F₂ seed was harvested from these F₁ plants and planted as a population. Fertile plants from this population were selected and grown as F₃ rows, thereby providing starting material for breeding approaches such as pedigree breeding, recurrent selection and others.
b) As parent In traditional breeding: Lines containing the Ms allele and/or rf allele of the present invention were crossed with other germplasm lines as part of the breeding program. The F₁ from these crosses was grown. Fertile plants were self pollinated and resultant F₂ seed harvested. Fertile plants from the F₂ population were selected, harvested and grown as F₃ rows, thereby providing starting material for breeding approaches such as pedigree breeding, recurrent selection and others.
c) As parent in doubled haploids: A source of the Ms allele and/or rt allele of the present invention was crossed to improved germplasm. The resulting hybrids underwent microspore culture to produce double haploid restorer lines. Microspore culture methods utilized were similar to those described by Chen et al (1994) and Mollers et al (1994). Thus, further disclosed is a method of producing a *Brassica napus* plant (e.g., a basic, prebasic, or maintainer line) comprising the steps of:
   i. selecting a male fertile parent with microspores comprising a allele selected from the group consisting of the Ms allele, ms allele, rf allele, arid Rf allele;
   ii. culturing selected microspores from the selected male fertile plant of step (i), forming haploids and inducing double haploids;
   iii. testing the double haploids progeny for the phenotype associated with said allele or the presence of the allele by markers; and
   iv. selecting progeny which are positive for the presence of said allele.
d) As source in backcross program: material containing the Ms allele and/or rf allele was crossed to selected inbred lines. Male fertile plants were emasculated and crossed again to the inbred line (recurrent parent). Resulting male fertile plants were backcrossed again to the inbred line. At any generation, selfing down of material could begin to produce new restorer lines. These projects exemplify a backcrossing program to bring the Ms allele and/or rt allele into superior germplasm. Marker analysis (as described above) can be employed.

These and other objects and advantages of the present invention will be apparent to those skilled in the art from a reading of the following examples and appended claims.

### Deposit

A seed sample of *Brassica napus* inbred line 07055001 (male sterile pre-basic seed line; genotype MsMsrfrf) was deposited with NCIMB, Ltd. (NCIMB Ltd; Ferguson Building, Craibstone Estate, Bucksbum, Aberdeen, AB21 9YA, UK; Tel: +44 (0) 1224 711100 Fax: +44 (0) 1224 711299), on 16th May 2007, Deposit Number NCIMB 41480. A seed sample of *Brassica napus* inbred line 04058001 ("Maintainer Line"; genotype msmsrtrt) was deposited with NCIMB, Ltd. (NCIMB Ltd; Ferguson Building, Craibstone Estate, Bucksbum, Aberdeen, AB21 9YA, UK; Tel: +44 (0) 1224 711100 Fax: +44 (0) 1224 711299) on 16th May 2007, Deposit Number NCIMB 41481.

### Declaration regarding the source of biological material

The Ms and rf alleles were obtained from *Brassica napus* material commercially available on the market of the European Union (Germany and other countries) in 1998.

### Examples

### Example 1: GSL Analysis

The glucosinolate (GSL) content of the Brassica seeds is monitored throughout the breeding program. Glucosinolate content is given in µlmol/g of seed at 9% humidity. The glucosinolate analysis can be performed using state of the art technology such as, for example, HPLC or near-infrared reflectance spectroscopy (NIRS). Using the NIRS method, it is possible to analyze samples of undestroyed Brassica seed for their quality components oil, protein and glucosinolate.
The glucosinolate levels discussed herein are determined in accordance with two standard procedures, namely (1) high performance liquid chromatography (HPLC) as described in ISO 9167-1:1992(E) for quantification of total intact glucosinolates ("Rapeseed-Determination of glucosinolates content--Part 1: Method using high-performance liquid chromatography, International Organization for Standardization", Geneva), and (2) gas-liquid chromatography for quantification of trimethylsilyl (TMS) derivatives of extracted and purified desulfoglucosinolates as described by Sosulski and Dabrowski (1984). Both the HPLC and TMS methods for determining the glucosinolate levels discussed herein involve analysis of the solid component of the seed after crushing and oil extraction (i.e., the de-fatted or oil-free meal). More preferred, the glucosinolate analysis is performed using the near-infrared reflectance spectroscopy. The analyses are performed on a FOSS NIR Systems Model 5000-c. Glucosinolate analysis is described in Williams & Sobering (1992).

### Example 2: Method for Determining Fatty Acid Profile

The fatty acid concentrations discussed herein are determined in accordance with a standard procedure wherein the oil is removed from the Brassica oilseeds by crushing and is extracted as fatty acid methyl esters following reaction with methanol and sodium methoxide. Next, the resulting ester is analyzed for fatty acid content by gas liquid chromatography using a capillary column which allows separation on the basis of the degree of unsaturation and chain length. This analysis procedure is described in the work of Daun et al. (1983), which is herein incorporated by reference.

### Example 3: Development of homozygous prebasic male sterile lines

Male sterile Takagi germplasm *Brassica napus* plants were pollinated with pollen of the *Brassica napus* variety Zenith. The F₁ progeny of this cross was male fertile. Plants of those were crossed in the greenhouse with male fertile plants of the variety Smart after hand emasculation. F₁ plants were raised out of each plant's crossing seeds in the greenhouse. All plants showed a male fertile phenotype. Individual plants out of this population were selfed by isolation of single plants with plastic bags (Cryovac Crispac Beutel Super Micro Lochung 360 x 830 mm, Supplier. Baumann Saatzuchtbedarf D-74638 Waldenburg). F₂ selfing descendants were tested in the greenhouse on male sterility/fertility. Eight of ten populations were fertile, two of ten populations showed segregation for sterility/fertility. Sterile plants from these populations were selfed under high temperature conditions as described below in Example 10. F₃ populations of those male sterile plants were again grown in the greenhouse and selfed. Three populations showed only male sterile plants, 10 populations segregated into male sterile and male fertile plants. The male sterile plants from the non segregating populations were selfed under heat treatment. According to the inheritance scheme given in Figure 1 and confirmed by marker analysis these plants exhibit the genotype MSMSrfrf. Two hundred and sixty-four F₄ plants were raised in the greenhouse. They all showed the male sterile phenotype. After heat treatment a total of 232 g of seeds could be harvested from all plants.

### Example 4: Development of maintainer lines

The male fertile plants out of the segregating F₃ populations from Example 3 were selfed as they are exhibiting rfrfmsms (Fig. 1). Testcrosses with plants of the completely male sterile F₃ populations were carried out. Five different crosses were evaluated with 16 plants each. All plants showed a male sterile phenotype, proving that the crossing was rfrfMSMS x rfrfmsms as presumed. The absence of the Rf allele in the maintainer line is crucial. To secure this, it is essential to have markers for this allele. Cross pollination from restorer lines cannot be detected because there is no phenotypic difference between maintainer and restorer.

### Example 5: Identification of molecular marker linked to the RHS sterility allele (Ms allele)

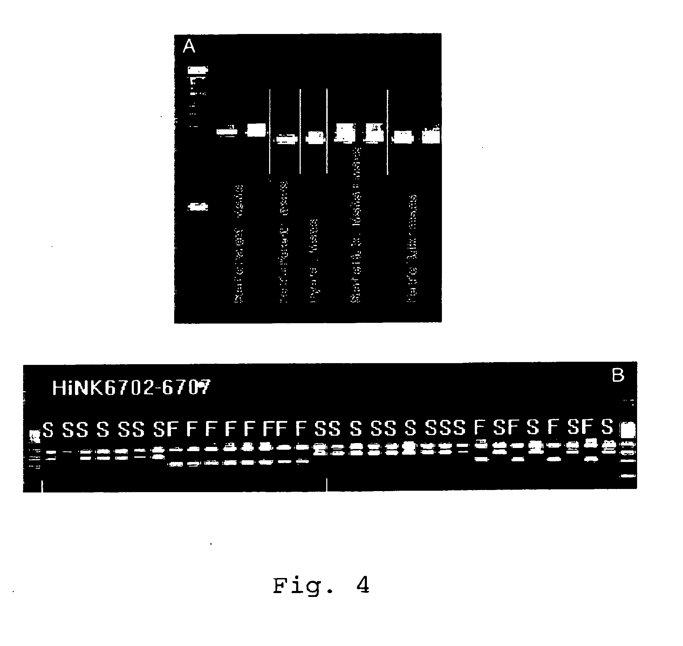

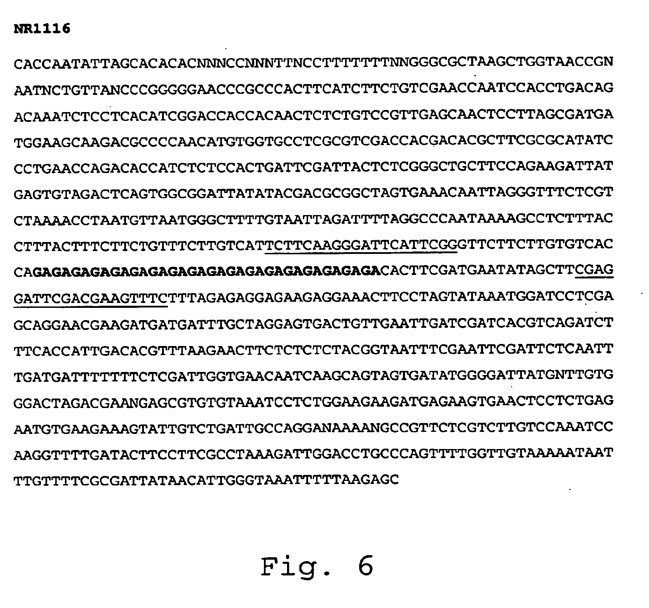

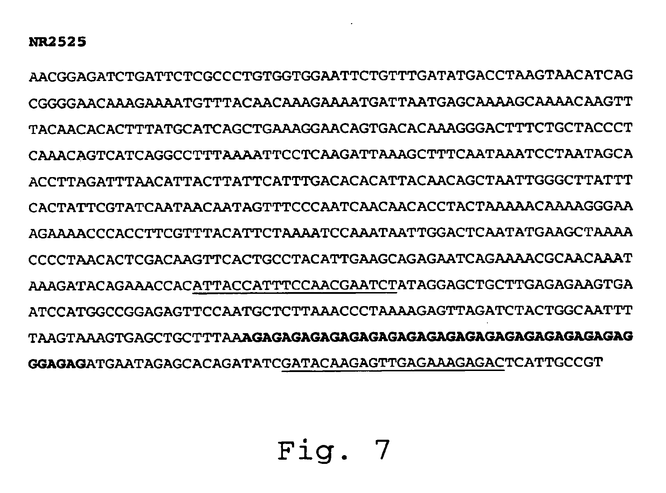

A bulk segregant analysis (BSA) (Michelmore et al., 1991) experiment was run on a F₂ population of 162 individuals segregating for the RHS sterility locus. This population is derived from the cross between an RHS-sterile line (ID: 03550015-34; as represented by the seed sample deposited under Deposit Number NCIMB 41480) and the corresponding RHS-fertile maintainer line (ID: 03560006-08; as represented by the seed sample deposited under Deposit Number NCIMB 41481). The segregation for male sterility as observed in the F₂ population fitted the 3:1 ratio expected for a single dominant gene. A set of 762 microsatellite markers (SSR) was tested for polymorphism between both parental lines as well as between bulks of male sterile and male fertile F₂ plants. Two different male fertile and male sterile bulks were analyzed. Bulks consisted of a leaf sample mix from 10 fertile or 10 sterile F₂ plants. The BSA disclosed five SSR markers showing polymorphism between the two parental lines as well as between the male sterile and male fertile bulks (Fig. 4-A).
These five SSR markers were subsequently genotyped across the whole F₂ population and mapped using Mapmaker/Exp (version 3.0b). The mapping results confirmed markers NR1116 (SSR amplified by oligonucleotide primers SEQ ID NO: 1 and 2; SSR region set forth as SEQ ID NO: 21; see also Fig. 6 for location of primers and SSR motif) and NR2525 (SSR amplified by oligonucleotide primers SEQ ID NO: 4 and 5; SSR region set forth as SEQ ID NO: 22; see also Fig. 7 for location of primers and SSR motif) to be closely linked the male sterility allele (Ms) on chromosome N7 (Table 2). NR1116 forward primer sequence:
5' -TCTTCAAGGGATTCATTCGG-3' (SEQ ID NO: 1)
NR1116 reverse primer sequence:
5'-GAAACTTCGTCGAATCCTCG-3' (SEQ ID NO: 2)
NR2525 forward primer sequence:
5'-ATTACCATTTCCAACGAATCT-3' (SEQ ID NO: 4)
NR2525 reverse primer sequence:
5'-GTCTCTTTCTCAACTCTTGTATC-3' (SEQ ID NO: 5)

SSR marker NR1116 consists of a GA/CA repeat of approximately 20 units. The observed allele size in the RHS-Ms mapping population (using an ABI 3700 sequencer) is for the RHS-sterile line (0355015-34): 96.7 (+/- 1) bp (male sterile allele) and for the RHS-maintainer line (03560006-08): 112.3 (+/- 0.4) bp (male fertile allele).
SSR marker NR2525 consists of an AG repeat of approximately 20 units. The observed allele size in RHS-Ms mapping population (using a ABI 3700 sequencer) is for the RHS-sterile line (0355015-34): 192.8 (+/- 0.3) bp (male sterile allele) and no band for the RHS-maintainer line (03560006-08) (male fertile allele). For that reason, NR2525 was scored as a dominant marker for Ms. Another fragment of 194.6 (+/- 0.5) bp was observed, but this band was not considered as it appeared persistently in all plant individuals regardless of their phenotype.
The genotype of the Ms allele was predicted according to the phenotype: male sterile plants can be 'Msms' or 'MsMs', but male fertile plant are always 'msms'. Therefore, the Ms allele was mapped as a dominant marker. Accordingly, NR1116 and NR2525 map at either side of the Ms trait at a genetic distance of 2.8 cM and 6.0 cM, respectively (see Table 2). The calculated distances are essentially the same for both the Ms and the ms allele.

**Table 2: Mapping distances calculated between Ms and the flanking markers located on Chromosome N7 using Mapmaker (version 3.0b). The genetic distance between markers is estimated in centimorgan (cM) and is directly proportional to the number of recombinants found in the population.**

| **Loci** | **Genetic distance between locii** |
|---|---|
| NR1116 | 2.8 cM |
| Ms allele | 6.0 cM |
| NR2525 | ---- |
| Sum of genetic distances | 8.8 cM |

The experimental conditions for the BSA or for the mapping of the SSR markers consisted of routine protocols well known to people skilled in the art of molecular markers. PCR amplifications for the BSA screening were run on a GeneAMP PCR System 9700 instrument from Applied Biosystems Inc. in a total reaction volume of 10 µl on 384 well plates using Sigma Jump start Taq polymerase. The PCR mix consisted of 1X reaction buffer from Sigma, 1.65 mM of MgCl₂, 0.25 mM of dNTPs and 400 nM of each primer. PCR conditions typically consisted of 2 min at 94°C, followed by 40 amplification cycles of 15 seconds at 94 °C, 45 seconds at 59 °C, and a final incubation of 2 min at 72 °C. The amplification products were subsequently loaded and migrated on 3 % Resophor Agarose 1000 (from Invitrogen Corp.) gels according to the supplier's instructions. For mapping purposes, the primers used for the PCR amplification comprised at least one primer labeled with HEX (5'-Hexachloro-fluorescein), NED (Benzofluorotrichlorocarboxy-fluorescein) or FAM (carboxy fluorescein) in order to allow for fluorescent detection on an ABI 3700 sequencer to resolve and score length polymorphisms. Apart from the use of a fluorescently labeled primer the PCR conditions for the mapping of SSRs were the same as for the BSA screening.

### Example 6: Conversion of SSR markers into SNP markers.

In order to convert the SSR markers into SN P markers that are considered more versatile for marker-assisted breeding, the amplification products of the SSR markers were sequenced across a panel comprising both homozygous male sterile (MsMs) and homozygous male fertile or maintainer (msms) lines, eight each. Each combination of male sterile and maintainer line represents a different genetic background. BLAST analysis using the nucleotide sequence of NR1116 (SEQ ID NO: 21) as a query against the genomic survey sequence database (GSS) at NCBI showed strong sequence homology between NR1116 and a GSS fragment from Brassica oleracea with accession number BH708933. The homology extends over a length of approximately 0.4 Kb immediately downstream of the microsatellite motif and allowed for the design of putatively A genome (Brassica rapa)-specific primers HiNK6440 and 6442 (SEQ ID NOs: 7 and 8, respectively).
Oligonucleotide primer HiNK6440:
5'-GTTCACTTCTCATCTTCTTCCAG-3' (SEQ ID NO: 7)
Oligonucleotide primer HiNK6442:
5' -TCCTGGCAATCAGACAATACTT-3' (SEQ ID NO: 8)

Sequence analysis of the amplification products obtained in MsMs and msms lines revealed two haplotypes that showed correlation with the male sterility trait. Table 3 summarizes the position of the polymorphic positions that distinguish both haplotypes; the consensus sequence is listed as SEQ ID NO: 3.

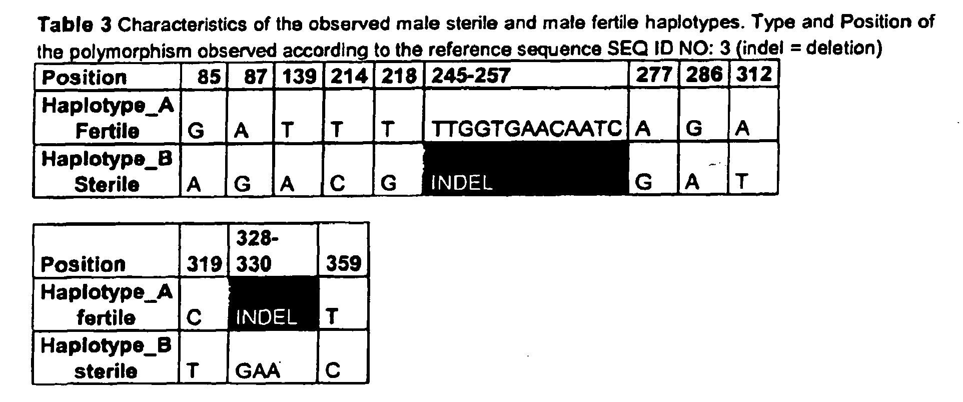

The SNPs at position 214 (T/C) and 218 (T/G) were selected to develop a TaqMan® assay using the Primer Express 2.0 software distributed by Applied Biosystems Inc. and following the corresponding instructions. The sequences of primers and probes corresponding to this assay referred to as SR0002A are listed in Table 4.

**Table4: Nucleotide sequencesofthe primers and probes for Taqman assaySR0002A. Fluoro-chromes: FAM (carboxy fluorescein); VIC (Applied Biosystems proprietary abbreviation). MGB: minor groove binder. NFQ: non fluorescent quencher**

| **NR1116** | **SR0002A TaqMan®** | |
|---|---|---|
| HiNK6441 SEQ ID: 8 | | PCR primer |
| HiNK6697 SEQ ID: 10 | 5'-ACACACGC77CTTCGTCTAGT-3' | PCR primer |
| HiNK6700 SEQ ID: 11 | VIC-CGAATCGATTCTC-MGB-NGQ | Fertile allele specific probe (ms) |
| HiNK6701 SEQ ID: 12 | FAM-CGAATCCGAGTCTC-MGB-NFQ | Sterile allele specific probe (Ms) |

The sequence of microsatellite marker NR2525 is published under accession number BZ061557 (SEQ ID NO: 22). In order to survey this sequence fragment for allelic variability correlated to the male sterility trait, PCR primers were designed to the sequence flanking the microsatellite motif.
Oligonucleotide primer HiNK6702:
5' -AGTAACATCAGCGGGGAAC-3' (SEQ ID NO: 13)
Oligonucleotide primer HiNK6707:
5'-TTTAAGAGCATTGGAACTCTCC-3' (SEQ ID NO: 14)

The combination of primers HiNK6702 and 6707 (SEQ ID NO:13 and 14, respectively) showed two amplification products that probably correspond to the A and C genome; the larger fragment of 0.6 Kb turned out to be monomorphic, but the smaller fragment showed a length polymorphism correlated to the male sterility trait in a number of different genetic backgrounds, 0.5 Kb for the male fertile allele versus 0.4 Kb for the male sterile allele (Fig. 4B). Sequence analysis of this polymorphic fragment across the panel of MsMs and msms lines revealed three haplotypes (Table 5), one single haplotype as expected for the male sterile Ms allele (haplotype B), and two haplotypes for the male fertile ms allele (haplotypes A and C).

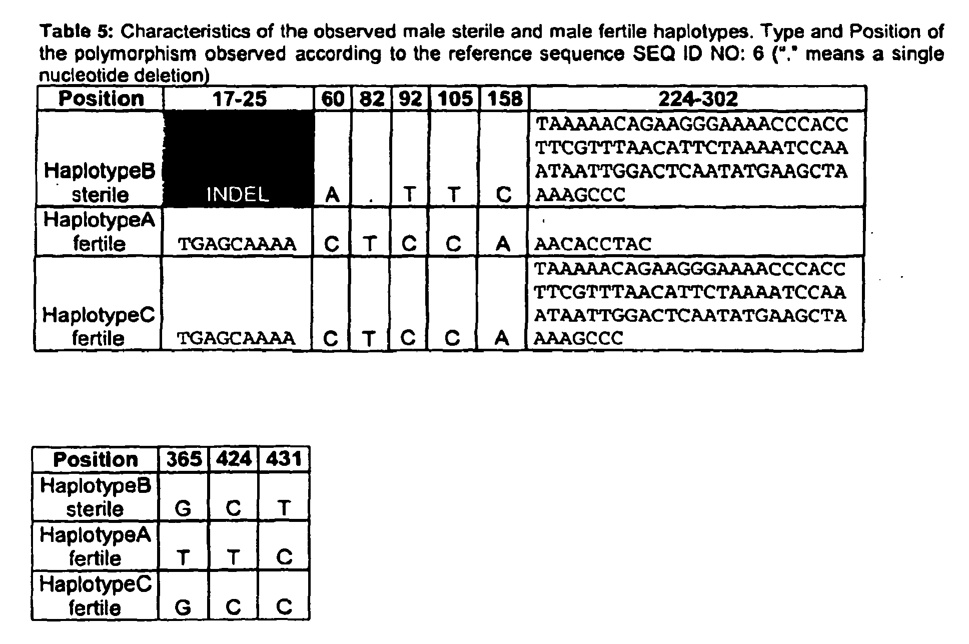

The consensus sequence for the three haplotypes is set forth as SEQ ID NO: 6. As illustrated by the alignment of the haplotypes, the male sterile allele can be distinguished from the male fertile allele at a number of positions including the SNP at position 158 that was targeted for the design of a TaqMan® assay as described above. The sequences of the primers and probes used in this assay that is referred to as SR0003B are listed in Table 6.

**Table 6: Nucleotide sequences of the primers and probes forTaqman assay SR0003B. Fluoro-chromes: FAM (carboxy fluorescein); VIC (Applied Biosystems proprietary abbreviation). MGB: minor groove binder. NFQ: non fluorescent quencher**

| **NR2525** | **SR0003B TaqMan®** | |
|---|---|---|
| HiNK6771 SEQ ID: 15 | 5'-TTTACAACACAAAGGGCTTTCTGC-3' | PCR primer |
| HiNK6772 SEQ ID: 16 | 5'-TGTAGGCCGTGAACTTGTCGGATTG -3' | PCR primer |
| HiNK6775 SEQ ID: 17 | FAM-ATTTGACACACATTACC-MGB-NFQ | Sterile allele specific probe (Ms) |
| HiNK6776 SEQ ID: 18 | VIC - ATTTGACAAACATTACC- MGB - NFQ | Fertile allele specific probe (ms) |

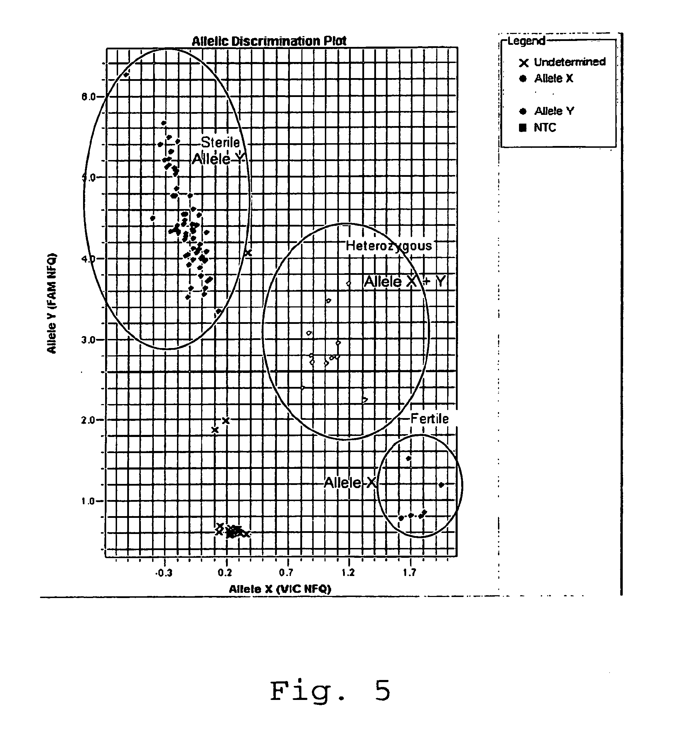

The TaqMan® assays derived from both NR1116 and NR2525 were typically run In reaction volumes of 10 µl in 384 well plates on GeneAMP PCR System 9700 instruments from Applied Biosystems Inc. using Platinum Taq polymerase and the corresponding enzyme mix from invitrogen Corp. PCR conditions typically consisted of a primary denaturation step of 2 minutes at 94 °C, followed by 40 amplification cycles of 15 seconds at 94 °C and 60 seconds at 62 °C. The fluorescent FAM and VIC signals were subsequently quantified at a 7900HT Sequence Detection System from Applied Biosystems Inc using the SDS 2.1 software package. Fig. 5 shows a typical plot obtained for marker SR0002A with the homozygous male sterile (MsMs), heterozygous male sterile (Msms) and homozygous male fertile (msms) plants segregating in three different clouds. Using the above experimental protocol, both markers were mapped in the F₂ population segregating for the male sterility trait as described in Example 1. As expected based on the mapping position of the original SSR markers, the SNP assays SR0003B and SR0002A map at either side of the Ms trait at a distance of 2.8 cM and 3.3 cM, respectively, relative to the Ms trait (Table 7).

**Table 7: Mapping distances calculated between Ms and the flanking markers located on Chromosome N7 using Mapmaker (version 3.0b). The genetic distance between markers is estimated in centimorgan (cM) and is directly proportional to the number of recombinants found in the population.**

| **Loci** | **Genetic distance between loci** |
|---|---|
| NR1116 | 0.0 cM |
| SR0002A | 2.8 cM |
| Ms allele | 3.3 cM |
| SR0003B | 2.2 cM |
| NR2525 | ------ |
| Sum of genetic distances | 8.3 cM |
| Ms and ms alleles show essentially the same distances. | |

It will be evident to people skilled in the art that the combined use of both markers will allow for the reliable genotyping of the Ms trait in oilseed rape as described hereinabove.

### Example 7: Identification of molecular markers linked to the RHS restorer allele (Rf allele)

In orderto develop markers for the Rf locus, a BSA (Michelmore et al., 1991) was performed on a F₂ population of 190 individuals segregating for the RHS fertility. This population is derived from the cross between a RHS-sterile line (MsMsrfrf) (ID: 05056504, as represented by the seed sample deposited under Deposit Number NCIMB 41480) and a restorer line (msmsRfRf) (ID: NK FAIR). Since the Ms locus for male sterility is segregating as well in this cross, fertile msms plant individuals were removed from the population prior to the BSA screening based on the genotype obtained for SSRs markers NR1116 and NR2525 as described in Example 5. In the subpopulation of 190 individuals comprising MsMs and Msms plants only, the segregation for fertility fitted the 3:1 ratio expected for single dominant gene. A set of 1225 microsatellite markers (SSR) was tested for polymorphism between both parental lines, as well as between bulks of male sterile and male fertile F2 plants. Three different male fertile and male sterile bulks were analyzed. Bulks consisted of a leaf sample mix from 10 fertile or 10 sterile F2 plants. The BSA disclosed 37 SSRs polymorphic between both parental lines and between the bulks of male sterile and male fertile F2 plants. These 37 SSRs were subsequently genotyped on the whole F2 population and mapped using Mapmaker/Exp (version 3.0b). The genotype of the Rf allele was predicted according to the phenotype observed: male fertile plants can be 'Rfrf' or 'RfRf', but male sterile plants are always 'rfrf'. Therefore, the Rf allele was mapped as a dominant marker. The Mapping results revealed two SSRs, NR2219 (SEQ ID NO: 23) and NR3454 (SEQ ID NO: 26) that are closely linked to the male fertility gene (Rf) on chromosome N19 (Table 8). In fact, NR2219 and NR3454 map at a genetic distance of 10.2 cM and 26.5 cM, respectively, at either side of Rf. The calculated distances are essentially the same for both the rf and the Rf allele.

The experimental conditions for the BSA or for the mapping of the SSR markers consisted of routine protocols well known to people skilled in the art of molecular markers as already described in example 5.

The primers used for the amplification of the SSR NR2219 were as follows:
NR2219 forward primer sequence:
5' -ATTATCCTCTCGCCATTTC-3' (SEQ ID NO: 19)
NR2219 reverse primer sequence:
5'-AAACTCCTGAACACCTCCTAC-3' (SEQ ID NO: 20)

The primers used for the amplification of the SSR NR3454 were as follows:
NR3454 forward primer sequence:
5' -GATGGTGATGGTGATAGGTC-3' (SEQ ID NO: 24)
NR3454 reverse primer sequence:
5'- GAAGAGAAGGAGTCAGAGATG-3' (SEQ ID NO: 25)

SSR NR2219 consists of a TA repeat of approximately 27 units. The observed allele size on the ABI 3700 sequencer is 240.8 (+/- 0.4) bp for the rf allele of the female parental line (ID: 05056504, as represented by the seed sample deposited under Deposit Number NCIMB 41480) whereas no band was obtained for the restorer line (NK FAIR: Rf allele; restorer allele). Accordingly, NR2219 behaved as a dominant marker in the segregating population: the presence of the allele 240.8 (+/- 0.4) corresponds to both the homozygous and heterozygous status of the rf allele, whereas the absence of the allele 240.8 (+/- 0.4) corresponds to the homozygous status of the Rf allele. SSR NR3454 consists of a TCA repeat of approximately 4 units. The observed allele size on the AB13700 sequencer is 282 (+/-0.38) bp for the rf allele of the female parental line (ID: 05056504, as represented by the seed sample deposited under Deposit Number NCIMB 41480) and 290 (+/-0.38) bp for the restorer line (NK FAIR: Rf allele; restorer allele).

**Table 8: Mapping distances calculated between Rf and the flanking markers located on Chromosome N19 using Mapmaker (version 3.0b). The genetic distance between markers is estimated in centimorgan (cM) and is directly proportional to the number of recombinants found in the population**

| Loci | Genetic distance between loci |
|---|---|
| NR3454 | 26.5 cM |
| Rf allele | 10.2 cM |
| NR2219 | ----- |
| Sum of genetic distances | 36.7 cM |

### Example 8: Fine mapping of the RHS restorer gene (Rf gene) through Single Feature polymorphism (SFP) detection on a GeneChlp.

The fine mapping of the RHS restorer gene (Rf gene) was achieved through the hybridization of Near Isogenic Lines (NILs) for the Rf gene on Syngenta's proprietary Brassica Affymetrix® GeneChip. The design of this Brassica GeneChip is a custom design, realized by Affy-metrix®. The Genechip contains 2.56 million probes derived from 152,362 Brassica unigenes representing *Brassica napus, Brassica rapa* or *Brassica oleracea.* The unigenes consisted of the consolidated consensus sequences derived from the Brassica EST assemblies at PlantGDB (www.plantgbd.org, version 161 A for *Brassica napus* and *rapa,* 157a for *Brassica oleracea*). The consolidated consensus sequences were obtained by merging the 3 assemblies using the cd-hit program (http://bioinformatics.ljcrf.edu/cd-hi/) with a threshold of 98% sequence identity. Each consensus sequence was divided into probe selection regions (PSR) of 150 bases long. On average, 4 probes per PSR, and 16 probes per transcript (perfect match probes only) were designed along the entire transcript, avoiding intron/exon boundaries. The constraints applied for the probe design ensured uniqueness of the probe sequence, comparable hybridization efficiencies and removal of cross-hybridizing probes as recommended by Affymetrix. In order to enable estimating background signals, 17.000 antigenomic probes were included on the chip.

Eight couples of Near Isogenic Lines were used for this experiment. These eight couples consist of 8 different oilseed rape lines (Restorer lines; Rf allele; restorer allele) and their respective Near isogenic lines in which the maintainer allele (rf allele) was introgressed through one backcross and then fixed by five successive selfings (Table 9). The level of isogenicity of the lines was assessed through the genotyping of fifty five polymorphic SSR markers well distributed along the nineteen chromosomes of *Brassica napus.*

**Table 9: List and characteristics of the 16 NILs used for the fine mapping of Rf (see Example 8)**

| **NIL type:** | **Restorer lines** | **Maintainer lines** | **Percentage of Isogenicity between lines** |
|---|---|---|---|
| **Rf Genotype:** | **RfRf** | **Rfrf** | |
| NIL Couple NO:1 | ID: ROXET | ID: 06558782 | 87% |
| NIL Couple NO:2 | ID: NK NEMAX | ID: 06558815 | 82% |
| NIL Couple NO:3 | ID: RNX1208 | ID: 06558736 | 77% |
| NIL Couple NO:4 | ID: NK BEAMER | ID: 06558524 | 56% |
| NIL Couple NO:5 | ID: RNX1302 | ID: 06558592 | 75% |
| NIL Couple NO:6 | ID: NK PASSION | ID: 06558614 | 71% |
| NIL Couple NO:7 | ID: NK FAIR | ID: 06558591 | 63% |
| NIL Couple NO:8 | ID: SMART | ID: 06558721 | 67% |

Each individual line was hybridized twice onto the gene chip, except for the lines of NIL couple NO:1 for which two times 6 hybridizations were performed in order to obtain sufficient statistical significance. This adds up to a total number of 36 chips used for this experiment. The DNA preparation and labeling, hybridization and scanning of chip, and data normalization has been performed as described in the published International patent application WO2007/005305, hereby incorporated herein in its entirety by reference.
For data analysis, a one-way analysis of variance (ANOVA) was performed by comparing hybridization results of all Restorer lines (RfRf lines) to all maintainer lines (rfrf lines), so that the 36 individual hybridizations were analyzed as 18 replications of RfRf genotypes and 18 replications of rfrf genotypes. This strategy leads to an increased statistical power. The alpha-value threshold chosen to declare an SFP as significant, meaning that the hybridization signal between RfRf and rfrf lines is statistically different, was calculated with the Bon-feronni test. The Bonferroni correction is a multiple-comparison correction used when several dependent or independent statistical tests are being performed simultaneously (while a given alpha value may be appropriate for each individual comparison, it is not for the set of all comparisons). In order to avoid spurious positives, the alpha value needs to be lowered to account for the number of comparisons being performed. Therefore, the theoretical alpha-value of 0.05 was divided by the number of unigenes on the GeneChip: 0.05 / 152362 = 3.3 10⁻⁷. This threshold leads to 55 significant SFP representing 30 Brassica unigenes, referred to as the Brassica candidate genes.
The 30 Brassica candidate genes were further validated by exploiting the synteny between the genomes of *Brassica* and *Arabidopsis thaliana.* A TBLASTX analysis of the nucleotide sequences of the Brassica unigenes to the TAIR *Arabidopsis thaliana* protein database allowed identifying the *Arabidopsis thaliana* homologues for 23 Brassica candidate genes. Subsequently, the 23 Brassica candidate genes were projected on the Arabidopsis genome based on the physical position of the Arabidopsis homologues (Figure 11), which resulted in the identification of a cluster of 14 Arabidopsis genes on chromosome 5. Since the Brassica unigenes are predicted to map around the Rf locus, one may assume that the Rf gene is localized within this syntenic region of 1.77 Million base pairs in Arabidopsis. The correspondence between the 14 Arabidopsis genes and the 14 Brassica candidate genes is specified in Table 10.

**Table 10: Correspondence between the 14 Arabidopsis genes mapped into a cluster on chromosome 5 and their Brassica candidate genes homolog.**

| **Arabidopsis gene ID** | **Physical position (bp)** | **Brassica unigene ID (Plant GBD source)** | **Arabidopsis Gene annotation** |
|---|---|---|---|
| AT5G17440 | 5751705 | PUT-161a-Brassica_ napus-59218 | LUC7N_terminus domain-containing protein |
| AT5G18350 | 6076548 | PUT-161a-Brassica_ napus-113367 | Disease resistance protein (TIR-NBS-LRR class), putative |
| AT5G18840 | 6214517 | PUT-161a-Brassica_ napus-61265 | Sugar transporter, putative |
| AT5G18900 | 6305255 | PUT-161a-Brassica_ napus-98270 | Oxidoreductase, 20G-Fe (II) oxygenase family protein |
| AT5G18900 | 6305255 | PUT-161a-Brassica_ napus-64108 | Oxidoreductase, 20G-Fe (II) oxygenase family protein |
| AT5G18920 | 6310457 | PUT-161a-Brassica_ napus-116958 | Unknown protein |
| AT5G19070 | 6376455 | PUT-161a-Brassica_ napus-212137960 | Unknown protein |
| AT5G19460 | 6557383 | PUT-161a-Brassica_ napus-93955 | ATNUDT20 (Arabidopsis thaliana Nudix hydrolase homolog 20) |
| AT5G19460 | 6557383 | PUT-161a-Brassica_ napus-20713 | ATNUDT20 (Arabidopsis thaliana Nudix hydrolase homolog 20) |
| AT5G19450 | 6557383 | PUT-161a-Brassica_ napus-98091 | CPK7 (CALMODULIN-DOMAIN PROTEIN KINASE 7) |
| AT5G19480 | 6572515 | PUT-161a-Brassica_ napus-112386 | Unknown protein |
| AT5G22460 | 7444530 | PUT-161a-Brassica_rapa-6777 | Esterase/lipase/ thioesterasefamily protein |
| AT5G22500 | 7472285 | PUT-161 a-Brassica_ napus-59425 | Acyl CoA reductase, putative/ male-sterility protein, putative |
| AT5G22650 | 7537143 | PUT-161a-Brassica_ napus-116246 | HD2B (HISTONE DEACETYLASE 2B) |

The 14 Brassica candidate genes listed in Table 10 were subsequently explored as targets for marker development. Since the exact nature of the polymorphism detected on the chip is not known, the Single Strand Conformation Polymorphism (SSCP) technology was adopted for genotyping and subsequent mapping. The population segregating for Rf that was used for this purpose is the same as described in Example 7. For each Brassica candidate gene, primers were designed embracing the probe region for which the SFP was identified. Forward primers were synthesized with a M13F tail (5' CACGACGTTGTAAAACGAC 3'; SEO ID NO: 27) added to the 5' end, reverse primers carried a M13R tail (5' CAGGAAACAGCTATGACC 3'; SEQ ID NO: 28) at the 5' end. The primary PCR reactions were run in a final volume of 15 µl comprising 5 µl of genomic DNA at a concentration of 5 ng/µl, 1.5 µl of 10X reaction buffer, 1.2 µl of 10mM dNTPs, 0.5 µl of 50mM MgCl₂, 0.3 µl of each primer at a concentration of 10 µM, 0.12 µl of invitrogen Taq platinium (5U/µl). All PCR reactions were performed at an ABI GeneAmp PCR System 9700. Thermal cycling conditions for the primary PCR consisted of an initial incubation of 2 minutes at 94°C to activate the Taq polymerase followed by 35 amplification cycles of denaturation during 30 sec at 94°C, annealing during 30 sec at 55°C and elongation during 30 sec at 72°C. The PCR reaction was completed with a final extension of 5 minutes at 72°C.
PCR products were diluted 100 fold and a second PCR was performed using 5 µl of the diluted product as template and 0.4 µl of the M13F labelled tail as forward primer PET-5'-CACGACGTTGTAAAACGAC-3' (SEQ ID NO: 27) and 0.4 µl of the M13R labelled tail: FAM-5'-CAGGAAACAGCTATGACC-3' (SEQ ID NO: 28) as reverse primer, each at a concentration of 10 µM. Apart from the primer concentration and the annealing temperature that was set at 50°C, the experimental conditions for the secondary PCR reactions were the same as for the primary reactions.
SSCP analysis was performed on a ABI 3130xl Genetic Analyser. Before loading and running the samples, 0.2 µl of Genescan 500LIZ size standard and 9 µl Hi-Di formamide both from Applied Biosystems were added to 2ul of a 40-fold dilution of the final PCR product. The mix was denatured for 5 min at 95°C and cooled on ice for 3 minutes to avoid re-annealing. The electrophoresis polymer consisted of the POP conformation analysis polymer (CAP) from ABI at a concentration of 7.2% prepared as recommended by the supplier. The samples were loaded and migrated on a 36cm capillary array while applying the following parameters: oven temperature: 25°C, Poly_Fill_Vol: 6500 steps, Current stability: 5 µA, Pre run voltage 15 kV, Pre run time 180 sec, Injection voltage: 1.2 kV, Injection time: 24 sec, Voltage number of steps 40nk, Voltage step interval 15 sec, Data delay time 1 sec, Run voltage 15 kV and Run time: 3000 sec. Data collected during electrophoresis were analyzed with the GeneMapper software v4.0 from ABI.
Amongst the 14 Brassica candidate genes tested, unigene ID: PUT-1 61a-Brassica_napus-59218 (SEQ ID NO: 31) was mapped closest to the Rfgene, at a distance of 4.1 cM below Rf. Figure 12 displays the type of polymorphism observed for this locus in the segregating population. The corresponding SSCP marker thus allowed to narrow the mapping interval for Rf and represents the closest marker currently available (Table 11).
PUT-161 a-Brassica_napus-59218 forward primer sequence:
5' -ACAGAGACAGAGGAGGTAGC-3' (SEQ ID NO: 29)
PUT-1 61 a-Brassica_napus-59218 reverse primer sequence:
5'-ATCATAATCCCTCGTTCTTT-3' (SEQ ID NO: 30)

**Table 11: Mapping distances calculated between Rf and the flanking markers located on Chromosome N19 using Mapmaker (version 3.0b). The genetic distance between markers is estimated in centimorgan (cM) and is directly proportional to the number of recombinants found in the population. The distance is essentially the same for both the rf and the Rf allele**

| **Loci** | **Genetic distance between loci** |
|---|---|
| N R3454 | 26.5 cM |
| Rf allele | 4.1 cM |
| PUT-161 a-Brassica_napus-59218 | ---- |
| Sum of genetic distances | 30.6 cM |

### Example 9: Marker selection for male sterile and male fertile plants in RHS system segregating population.

One possibility to determine if a plant, for example, a F₂ plant resulting from a cross between an RHS-sterile line and a restorer line is male sterile or male fertile, would be to test this plant with markers linked to Ms (as described in Examples 1 and 2) and Rf alleles (as described in Example 3).

### Example 9.1: Prediction of the Ms genotype

The genotype of N R 1116, NR2525, SR0002A and SR0003B markers allowed the prediction of the Ms-genotype (Table 12; Fig. 5).

**Table 12: Characteristics of alleles underscored for NR1116 and NR2525, and assignment of Ms and ms allele. 'Ms' is the male sterile allele of the sterility gene and 'ms' is the male fertile allele of the sterility gene. The allele sizes given have been scored on an ABI 3700 sequencer. Calibration of the apparent fragment weight was made against the molecular weight standard ROX 500 (Applied Biosystems).**

| Loci | Ms/ms allele | Allele size (bp) |
|---|---|---|
| NR1116 | ms | 94(+/-0.9) |
| | Ms | 96.7(+/-1) |
| | ms | 110.4 (+/- 0.5) |
| | ms | 112.3 (+/- 0.4) |
| | ms | 116.3 (+/-0.4) |
| NR2525 | ms | 183.8 (+/- 0.4) |
| | Ms | 192.8 (+/- 0.3) |
| | - | 194.6 (+/- 0.5) |

If there are only male sterile alleles, one can predict that the plant is homozygous for the male sterility. (MsMs). If there are both male sterile and male fertile alleles, one can predict that the plant is heterozygous for the male sterility (Msms). And if there are only fertile alleles, one can predict that the plant is homozygous for the male fertility (msms).

### Example 9.2: Prediction of the Rf genotype

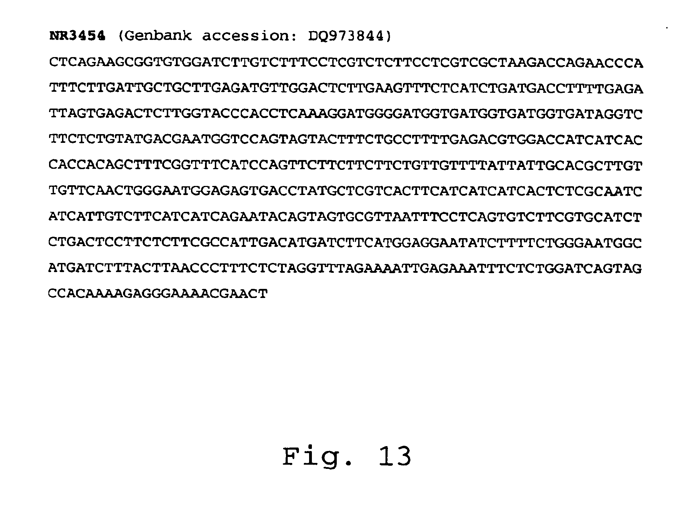

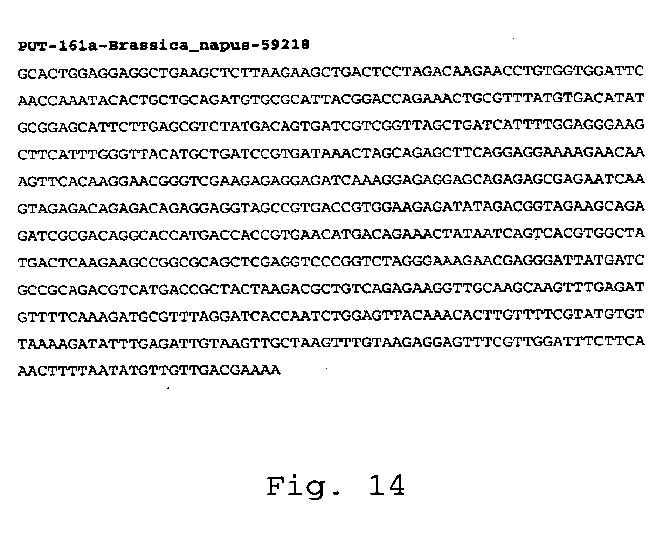

NR3454, NR2219 and PUT-161a-Brassica_napus-59218 marker allowed the prediction of the Rf-genotype (Table 12). For the SSCP marker PUT-161a-Brassica_napus-59218. because of the technology used it is not possible to give an allele size to score. One always has to refer to the polymorphism observed on referenced restorer'RfRf' and maintainer 'rfrf' lines (Figure 13).

**Table 13: Characteristics of alleles underscored for NR2219 and assignment of Rf and rf allele. 'Rf' is the fertile allele of the restorer allele and 'rf' is the sterile allele of the restorer allele. The allele sizes given have been scored on an ABI 3700 sequencer. Calibration of the apparent fragment weight was made against the molecular weight standard ROX 500 (Applied Biosystem)**

| Loci | Rf/rf allele | Allele size (bp) |
|---|---|---|
| NR2219 | Rf | Absence of 240.8 (+/- 0.4) |
| | rf | 240.8 (+/- 0.4) |
| NR3454 | Rf | 290 (+/- 0.38) |
| | rf | 282 (+/- 0.38) |

If there are only the maintainer 'rf' alleles, one can predict that the plant is homozygous for the male sterility (rfrf). If there are both maintainer and restorer alleles, one can predict that the plant is heterozygous for the male sterility (Rfrf). And if there are only restorer'Rf' alleles, one can predict that the plant is homozygous forthe male fertility (RfRf).

### Example 9.3: Prediction of male sterile and male fertile phenotype

According to the predicted Ms-genotype and Rf-genotype, one can predict the male sterility or male fertility of the plant (Table 14). As the restorer allele (Rf) is dominant on the male sterility gene (Ms), each time one has the male fertility allele at Rf the plant is male fertile. The male sterility can be achieved only if the fertility allele at Rf is absent and the male sterility allele at Ms is present.

**Table 14: Determination of male sterile and male fertile phenotype according to the genotype at the male sterility gene (Ms) and restorer allele (Rf).**

| Phenotype | Rf-genotype | Ms-genotype |
|---|---|---|
| fertile | RfRf | MsMs |
| fertile | RfRf | Msms |
| fertile | RfRf | msms |
| fertile | Rfrf | MsMs |
| fertile | Rfrf | Msms |
| fertile | Rfrf | msms |
| fertile | rfrf | msms |
| sterile | rfrf | Msms |
| sterile | rfrf | MsMs |

### Example 10: Selfing of RHS male sterile plants:

To achieve pollen production, RHS male sterile plants are preferably treated with a day temperature of approximately 38°C day (16 hours) and a night temperature of approximately 20°C (8 hours) for 7 days after opening of the first flower. This treatment was conducted in an air-conditioned room (Manufacturer of the air condition: www.redekerkaeltetechnik.de), which was equipped with eight 400 W Phillips SON-TP 400W Agro greenhouse lamps. After one week of heat treatment the plants were returned into the greenhouse and cultivated under natural conditions of at least 18°C day temperature and 14°C night temperature. 7 to 14 days after the described heat treatment the plants showed flowers with enlarged anthers. Those anthers could release pollen. The pollen was used to pollinate male sterile and male fertile flowers of the same plant. Adjacent plants were isolated using plastic bags (Cryovac Crispac Beutel Super Micro Lochung 360 x 830 mm, Supplier: Baumann Saatzuchtbedarf D-74638 Waldenburg) to prevent uncontrolled pollination. The pollinated plants showed normal pod development and seeds could be harvested after drying out of the plants as with normal fertile rapeseed plants.

### Example 11: Production of heterozygous female basic seed lines

Seeds of F₄ male fertile plants and F₄ male sterile plants from Example 10 were sown in isolation tents. Tents are 20 m long and 8 m wide. Cover material was insect prove net with a mesh size of 16 x 10. The design was one row male followed by 6 rows female, four rows male, 6 rows female and again one row male. Each row was sown with approximately 1.5 g single plant self seed. The tents were covered before flowering started. Female plants were selected for male fertile plants before flowering. Despite all care during the selfing process cross pollination by restorer pollen from the greenhouse could not be excluded completely. Male fertile plants could be detected as they did not exhibit the bud abortion phenotype of the male sterile ones. Male sterile plants start flowering later than the fertile ones due to the bud abortion. In order to synchronize flowering the male fertile plants were delayed for flowering by manually cutting back the main shoot at begin of the flowering. After flowering the two pollinator rows at the border of the tent were removed. The two plots of 6 rows of female male sterile plants and the 4 rows of male plants were separated manually to avoid mixing of seed. After ripening the male sterile female and the male fertile maintainer plants were harvested separately. In 2005 the seed yield of the basic seed female in one tent was 11.9 kg uncleaned seed. The maintainer plants in the same tent yielded 8.9 kg.
Prebasic seed production can also be done in a field production. Therefore, the minimum distance to the next rapeseed field must be 5 km. It has also to be assured that there are no fertile rapeseed or cruciferous plants in a circle of 5 km which may cross pollinate with rapeseed. It is important to check the road borders where rapeseed plants can grow often.

### Example 12: Hybrid development

Hybrids are produced in open field production. The female is a basic seed female as described in Example 4. The restorer is any conventional rapeseed line. The technique is strip-growing, with a border of at least 3m of restorer plants around the field. Within the field the ratio of male:female is between 1:3 to 1:4 with 1 stripe being between 2.5 to 4 m depending on the drilling machine of the farmer. Before flowering 50% of the restorer plants have to be topped at about 50 cm height. This can be done by every conventional grass cutter. Before flowering the male sterile plants need to be selected for male fertile plants as described above in Example 4. It has to be ensured that the amount of fertile plants in the sterile stripes does not exceed 0.2% in total to ensure a hybridity rate of above 90%. Isolation distance has to be 200m. It is essential to control the environmental temperature during flowering. If the temperature exceeds 20°C the female male sterile plants have to be checked for male fertile flowers in the following three weeks. After flowering the pollinator has to be removed to secure the purity of the harvested F₁ hybrid seed.

### Example 13: Hybrid performance

Hybrids were tested in yield trials in different countries. Trials were carried out in at least 3 replications with at least 15 m² plot size. The plots were harvested and plot yield was recalculated in dt/ha. Seeds were analyzed using NIRS technology on a FOSS NIR Systems Model 5000-c. The principle of those analyses is described in Williams & Sobering (1992). The results of those trials in Germany and Poland are given in Table 15 (RNX: Various hybrid seeds of the present invention; msl: NPZ msI-based hybrid seeds; Ogura: Inra Ogura hybrid seeds). The performance in relative seed yield is at least as good as if not better than the performance of the currently available hybrid systems.

**Table 15: Performance of RHS hybrids in 2006 (Germany and Poland) and 2007 (Germany, Poland and France).**

| **Variety** | **No. of locations** | **System** | **abs seed yield in dt/ha** | **rel seed yield** | **Oil content% in seed at 9% moisture** | **Protein content%in seed at 9% moisture** | **GSL content µmol per g seed at 9% moisture** |
|---|---|---|---|---|---|---|---|
| | | | | | | | |
| **Germany 2006** | | | | | | | |
| **TAURUS** | 7 | msl | **49.30** | **101.3** | **43.0** | **19.3** | **11.1** |
| **Elektra** | 7 | msl | **48.00** | **98.7** | **42.0** | **19.6** | **11.0** |
| RNX 3401 | 7 | RHS | 51.30 | 105.4 | 41.8 | 20.2 | 10.9 |
| RNX 3402 | 7 | RHS | 50.80 | 104.4 | 41.4 | 19.9 | 11.4 |
| RNX 3501 | 7 | RHS | 48.60 | 99.9 | 40.4 | 20.4 | 13.0 |
| RNX 3504 | 7 | RHS | 52.60 | 108.1 | 40.9 | 19.7 | 11.9 |
| RNX 3505 | 7 | RHS | 51.10 | 105.0 | 40.6 | 20.3 | 11.3 |
| RNX 3506 | 7 | RHS | 50.50 | 103.8 | 40.8 | 20.0 | 11.1 |
| **Poland 2006** | | | | | | | |
| **ES SAPHIR** | 4 | INRA Ogura | **46.09** | **99.0** | **43.1** | **18.8** | **33.4** |
| **Elektra** | 4 | msl | **46.98** | **101.0** | **44.4** | **17.9** | **12.7** |
| RNX 3401 | 4 | RHS | 47.89 | 102.9 | 44.2 | 17.7 | 11.3 |
| RNX 3402 | 4 | RHS | 48.62 | 104.5 | 44.6 | 17.5 | 13.7 |
| RNX 3403 | 4 | RHS | 48.83 | 104.9 | 44.3 | 17.8 | 14.3 |
| RNX 3404 | 4 | RHS | 49.43 | 106.2 | 44.3 | 17.9 | 13.6 |
| RNX 3405 | 4 | RHS | 45.89 | 98.6 | 41.8 | 19.0 | 12.0 |
| RNX 3407 | 4 | RHS | 44.97 | 96.6 | 43.4 | 18.6 | 16.6 |
| RNX 3501 | 4 | RHS | 48.15 | 103.5 | 42.6 | 18.3 | 12.3 |
| RNX 3502 | 4 | RHS | 47.99 | 103.1 | 43.9 | 18.8 | 14.9 |
| RNX 3504 | 4 | RHS | 49.32 | 106.0 | 43.0 | 18.0 | 12.2 |
| RNX 3505 | 4 | RHS | 49.74 | 106.9 | 43.2 | 18.6 | 11.0 |
| RNX 3506 | 4 | RHS | 48.28 | 103.7 | 43.2 | 18.1 | 9.8 |
| RNX 3507 | 4 | RHS | 48.49 | 104.2 | 42.3 | 18.4 | 12.1 |
| | | | | | | | |
| **Germany 2007** | | | | | | | |
| **Taurus** | 9 | msl | **44.95** | **103.7** | **43.9** | **18.9** | **14.6** |
| **Elektra** | 9 | msl | **41.76** | **96.3** | **42.7** | **19.4** | **21.0** |
| RNX 3401 | 9 | RHS | 49.84 | 115.0 | 42.1 | 19.8 | 14.3 |
| RNX 3404 | 9 | RHS | 49.09 | 113.2 | 42.1 | 19.4 | 16.5 |
| RNX 3504 | 9 | RHS | 49.54 | 114.3 | 42.2 | 18.7 | 13.7 |
| RNX 3621 | 9 | RHS | 50.53 | 116.5 | 41.7 | 18.9 | 13.2 |
| RNX 3622 | 9 | RHS | 49.92 | 115.1 | 42.1 | 19.7 | 14.8 |
| RNX 3623 | 9 | RHS | 50.14 | 1 15.6 | 42.7 | 19.4 | 14.7 |
| RNX 3624 | 9 | RHS | 48.63 | 112.2 | 42.9 | 18.8 | 15.5 |
| Poland 2007 | | | | | | | |
| **Elektra** | 9 | msl | **38.68** | **94.4** | **41.0** | **20.4** | **18.5** |
| **NELSON** | 9 | INRA Ogura | **43.29** | **105.6** | **39.9** | **20.2** | **22.6** |
| RNX 3401 | 9 | RHS | 42.81 | 104.5 | 40.6 | 20.7 | 15.5 |
| RNX 3402 | 9 | RHS | 45.18 | 110.2 | 41.9 | 19.4 | 14.9 |
| RNX 3403 | 9 | RHS | 46.65 | 113.8 | 42.0 | 19.3 | 14.2 |
| RNX 3404 | 9 | RHS | 44.04 | 107.5 | 41.2 | 19.9 | 16.2 |
| RNX 3504 | 9 | RHS | 45.68 | 111.4 | 40.6 | 19.5 | 15.8 |
| RNX 3505 | 9 | RHS | 43.38 | 105.8 | 40.3 | 20.4 | 14.7 |
| RNX 3507 | 9 | RHS | 44.04 | 107.5 | 39.0 | 20.4 | 16.7 |
| RNX 3621 | 9 | RHS | 44.66 | 109.0 | 40.9 | 19.5 | 13.8 |
| RNX 3622 | 9 | RHS | 45.02 | 109.9 | 41.2 | 20.3 | 14.9 |
| RNX 3623 | 9 | RHS | 44.99 | 109.8 | 41.6 | 20.0 | 15.2 |
| RNX 3624 | 9 | RHS | 44.58 | 108.8 | 42.3 | 19.0 | 15.0 |
| RNX 3625 | 9 | RHS | 44.50 | 108.6 | 41.1 | 20.0 | 14.0 |
| RNX 3726 | 9 | RHS | 45.17 | 110.2 | 40.6 | 19.4 | 14.6 |
| **France 2007** | | | | | | | |
| **EXAGON E** | 11 | INRA Ogura | **36.83** | **102.6** | **43.7** | **18.6** | **19.5** |
| **MENTIO N** | 11 | msl | **34.94** | **97.4** | **43.2** | **17.0** | **14.4** |
| RNX 3404 | 11 | RHS | 35.71 | 99.5 | 44.3 | 17.4 | 15.3 |
| RNX 3403 | 11 | RHS | 35.94 | 100.1 | 44.8 | 16.8 | 14.2 |
| RNX 3621 | 11 | RHS | 35.83 | 99.9 | 43.6 | 17.4 | 13.1 |

### REFERENCES

1. Abbadi, A., F. Domergue, J. Bauer, J.A. Napier, R. Welti, U. Zähringer, P. Cirpus, and E. Heinz. 2004. Plant Cell 16:2734-2748.
2. Abravaya K., Huff J., Marshall R., Merchant B., Mullen C., Schneider G., and Robinson J. (2003) Clin Chem Lab Med. 41:468-474.
3. Ackman, R.G.1990. Canola fatty acids- an ideal mixture for health, nutrition, and food use. p. 81-98. In F. Shahidi (ed.) Canola and rapeseed: Production, chemistry, nutrition, and processing technology. Van Nostrand Reinhold, New York.
4. Aherne FX, A.J. Lewis. The nutritive value of faba beans and low glucosinotate rapeseed meal for swine. Adv. Exp. Med. Biol. 1978, 105, pp 453-471.
5. Atanassova B. 1999. Functional male sterility (ps-2) in tomato (Lycopersicon esculentum) and its application in breeding and hybrid seed production. Euphytica 107: 13-21.
6. Barany, Proc. Natl. Acad. Sci.(U.S.A.) 88:189 193 (1991)
7. Becker H.C., Damgaard C., Karisson B. (1992) Theor. Appl. Genet. 84, 303-306.
8. Becker, H. C. and Link, W. 2000: Heterosis and hybrid breeding. Vortr. Pflanzenzüchtg. 48: 319-327.
9. Beckmann, J. S. and Soller, M. 1983. Restriction fragment length polymorphisms in genetic improvement: methodologies, mapping and costs. Theor. Appl. Genet 67: 35-43.
10. Botstein, D., White, R. L., Skolnik, M. and Davis, R. W. 1980. Construction of a Genetic Linkage Map in Man Using Restriction Fragment Length Polymorphisms. Am. J. Hum. Genet. 32: 314-331.
11. Brandle, J. E. and McVetty, P. B. E. (1990): Canadian Journal of Plant Science 70: 935-940.
12. Brandle, J.E. and McVetty, P.B.E. (1989): Crop Science, 29: 1191-1195.
13. Burton, J.W., J.F. Miller, B.A. Vick, R. Scarth, and C.C. Holbrook. 2004. Altering fatty acid composition in oil seed crops. Adv. Agron. 4:273-306.
14. Cahoon, E.B. 2003. AgBioForum, 6:11-13.
15. Cahoon, E.B., K.G. Ripp, S.E. Hall, and A.J. Kinney. 2001. J. Biol. Chem. 276:2637-2643.
16. Chen, F. X., B. C. Hu, C. Li, Q. S. Li, W. S. Chen, and M. L. Zhang, 1998: Genetic studies on GMS in Brassica napus L. I. Inheritance of recessive GMS line 9012A. Acta Agron. Sin. 24, 431-438.
17. Chen, Z.Z., S. Snyder, Z.G. Fan and W.H. Loh. 1994 Plant Breeding 113: 217-221
18. Daskalov S. 1972. Proc. Eucarpia Meet. Capsicum 7: 202-210.
19. Daun JK et al, J. Amer. Oil Chem. Soc., 60:1751-1754 (1983)
20. Dehesh, K. 2004. United States Patent Application 20040132189.
21. Dehesh, K., A. Jones, D.S. Knutzon, and T.A. Voelker. 1996. Plant J. 9:167-172.
22. Delourme R, A. Bouchereau, N. Hubert, M. Renard, B.S. Landry. Theoretical Applied Genetics, vol. 88, pp. 741-748, 1994.
23. Delourme R. and F. Eber. Theoretical and Applied Genetics vol. 85, pp222-228, 1992.
24. Delourme R., F. Eber, M. Renard. "Breeding Double Low Restorer Lines in Radish Cytoplasmic Male Sterility of Rapeseed (Brassica Napus L.)." Proc. 9th Int. Rapeseed Conf. Cambridge. England (1995).
25. Delourme R., F. Eber, M. Renard. "Radish Cytoplasmic Male Sterility in Rapeseed: Breeding Restorer Lines with a Good Female Fertility." Proc 8th Int. Rapeseed Conf., Saskatoon, Canada. 1506-1510 (1991).
26. Denis M., Delourme R., GourretJ. P., Mariani C., and Renard M. Plant Physiol. 101, 1295-1304 (1993).
27. Dickson MH. 1970. J. Amer. Soc. Hort. Sci. 95: 13-14.
28. Eskin, N.A.M., B.E. McDonald, R. Przybylski, L.J. Malcolmson, R. Scarth, T. Mag, K. Ward, and D. Adolph. 1996. Canola oil. p. 1-96. In Y. H. Hui (ed.) Edible oil and fat products: Oil and oil seeds. John Wiley & Sons Inc., New York.
29. Facciotti, M.T., P.B. Bertain, and L. Yuan. 1999. 17:593-597.
30. Fletcher,R.; Coventry,J.and Scott,L.S. (1998): Doubled Haploid Technology for Spring and Winter Brassica napus. Revised Edition. University of Guelph Toronto, Department of Plant Agriculture, Technical Bulletin, OAC Publication.
31. Förster, J. und Knaak, C. 1995: Estimation of the genetic distance of 21 winter rapeseed varieties by RAPD analyss in comparison to RFLP results. Proc. 9th Int. Rapeseed Congress, 4 - 7 July 1995, Cambridge, UK.
32. Frauen, M. (1999a) Ernährungsdienst 46:13.
33. Frauen M, J. Noack, A. Girke, W. Paulmann (2007): Ten years experience of development and cultivation of winter oilseed rape hybrids in Europe based on the MSL system Poc. 12.th. Int. Rapeseed Congress, Wuhan, China (2007)
34. Frauen, M., and A. Baer (1996) GCIRC Bulletin 12:47-51.
35. Fu TD (1981) Eucarpia Cruciferae Newsl 6 : 6D7
36. Girke A., Ph.D Thesis (Dissertation) Göttingen, May 2002 "Neue Genpools aus resynthetisiertem Raps (Brassica napus L.) für die Hybridzüchtung"
37. Grant, I. and Beversdorf, W.D. (1985): Canadian Journal of Genetics and Cytology, 27: 472-478.
38. Gunderson K.L., Steemers F.J., Ren H., et al. (2006) Methods Enzymol. 410:359-76
39. Hatanaka, T., R. Shimizu, and D. Hildebrand. 2004. Phytochemistry 65:2189-2196.
40. Hawkins, D.J., and J.C. Kridl. 1998. Plant J. 13: 743-752.
41. Heid CA, Stevens J, Livak KJ, Williams PM (1996). Genome Res 6: 986-994
42. Heyn, F.U. 1976. Transfer of restorer genes from Raphanus to cytoplasmic male sterile Brassica napus. Cruciferae Newsletter 1: 15-16.
43. Hitz, W.D., C.J. Mauvis, K.G. Ripp, and R.J. Reiter.1995. The use of cloned rapeseed genes for the cytoplasmic fatty acid desaturases and the plastid acyl-ACP thioesterases to alter relative levels of polyunsaturated and saturated fatty acids in rapeseed oil. p. 470-472. In Proc. 9th Int. Rapeseed Cong.: Rapeseed today and tomorrow, Cambridge, UK.
44. Horner HT and Palmer RG. 1995.. Crop Sci. 35:1527-1535.
45. Hou, G. Z., H. Wang, and R. M. Zhang, 1990: Genetic study on genicmale sterility (GMS) material No. 117A In Brassica napus L. Oil Crop China 2,7-10.
46. Hu SW et al. (2000) Acta Agriculturae Boreali-orientalis Sinica 9:90-94
47. Huang, Y.S., S.L. Pereira, and A,E. Leonard. 2004. Biochimie 86:793-798.
48. International Standard ISO 9167-1:1992(E). "Rapeseed - Determination of glucosinolates convent - Part 1: Method using high-performance liquid chromatography."
49. Jaworski, J., and E.B. Cahoon. 2003. Curr. Opin. Plant Biol. 6:178-184.
50. Jones, A., H.M. Davies, and T.A. Voelker. 1995. Plant Cell 7:359-371.
51. Kaul MLH.1988. Male Sterility in Higher Plants. Monographs on Theor. Appl. Genet.10, Springer-Verlag, Berlin.
52. Knaak, C. 1996. Schätzung genetischer Distanzen mittels RFLP zur Identifikation von Genpools für die Hybridzüchtung bei Winterraps. Diss. Univ. Göttingen. Cuvillier Verlag Göttingen
53. Knutzon, D.S., G.A. Thompson, S.E. Radke, W.B. Johnson, V.C. Knauf, and J.C. Kridl. 1992. Proc. Natl. Acad. Sci. USA 89:2624-2628.
54. Kumar, Sanjeet, and P. K. Singh. Journal of New Seeds; Vol. 6, No. 4, 2004, pp. 300-407. "Mechanisms for Hybrid Development in Vegetables."
55. Kutyavin IV, Afonina IA, Mills A, Gorn W, Lukhtanov EA, Belousov ES, Singer MJ, Walburger DK, Lokhov SG, Gall AA, Dempcy R, Reed MW, Meyer RB, Hedgpeth J (2000). Nucleic Acids Res 28: 655-661
56. Laga, B., J. Seurinck, T. Verhoye, and B. Lambert. 2004.. Pflanzenschutz-Nachrichten Bayer 57:87-92.
57. Landegren et al.. Science 241:1077 1080 (1988)
58. Lardy GP, and M.S. Kerley. J. Anim. Sci. (1994), 72: 1936-1942.
59. Lefort-Buson, M., Guillot-Lemoine, B. and Dattee Y. (1987): Genome, 29: 413-418
60. Li SL, Y. X. Qian, and Z. H. Wu, 1985: Acta Agriculturae Shanghai 1, 1-12.
61. Li SL, Y. X. Qian, Z. H. Wu, and B. R. Stefansson, 1988: Can. J. Plant Sci. 68, 1115-1118.
62. Li SL, Z. J. Zhou, and X. R. Zhou, 1993: Acta Agriculturae Shanghai 9. 1-7.
63. Li SL, Z. J. Zhou, and X. R. Zhou, 1995: Acta Agriculturea Shanghai 11, 21-26.
64. Liu, Q., S.P. Singh, and A.G. Green. 2002. Plant Physiol. 129:1732-1743.
65. Livak, K.J., Marmaro, J., and Todd, J.A. 1995. Nat. Genet. 9:341-342.
66. Lühs, W., and W. Friedt. 1995. Breeding high-erucic acid rapeseed by means of Brassica napus resynthesis. p. 449-451. In Proc. 9th Int. Rapeseed Cong. (GCIRC), Cambridge, UK.
67. Makaroff (1989 Journal of Biol. Chem. 264: 11706-11713
68. Mariani C et al. (1992) Nature 357:384-387
69. Mathias R (1985) Z Pflanzenzüchtg. 94:170-173
70. McGuigan F.E., Ralston S.H. (2002) Psychiatr Genet. 12(3):133-6.
71. McVetty, P.B.E., R. Scarth, and S.R. Rimmer. 1999. MillenniUM01 summer rape. Can. J. Plant Sci. 79:251-252.
72. McVetty, P.B.E., S.R. Rimmer, and R. Scarth. 1998. Can. J. Plant Sci. 78:305-306.
73. Melchinger, A. E., und Gumber, R. K.1998: Overview of Heterosis and Heterotic Groups in Agronomic Crops. In: Concepts and Breeding of Heterosis in Crop Plants. Crop Science Society of America, Madison, USA.
74. Michelmore RW, I. Paran and R.V. Kesseli. 1991. Identification of markers linked to disease resistance genes by bulked segregant analysis: A rapid method to detect markers in specific genomic regions using segregating populations. Proc Natl Acad Sci USA 88:9828-9832
75. Mietkiewska, E., E.M. Giblin, S. Wang, D.L. Barton, J. Dirpaul, J.M. Brost, V. Katavic, and D.C. Taylor. 2004. Plant Physiol. 136:2665-2675.
76. Mollers C., M.C.M. Iqbal and G. Robbelen. 1994 Euphytica 75: 95-104.
77. Mullis et al., Cold Spring Harbor Symp. Quant. Biol. 51:263 273 (1986)
78. Mündges, H., W. Köhler, W. Friedt. 1990. Identification of rapeseed cultivars (Brassica napus) by starch gel electrophoresis of enzymes. Euphytica 45: 179-187.
79. Nickerson et al., Proc. Natl. Acad. Sci.(U.S.A.) 87:8923 8927 (1990)
80. Nieuwhof M. 1968. Euphytica 17: 265-273.
81. Ogura, H. 1968. Studies on the new male sterility in Japanese radish, with special reference on the utilization of this sterility towards the practical raising of hybrid seeds. Mem Fac Agric Kagoshima Univ. 6: 39-78.
82. Olivier M. (2005) Mutat Res. 573(1-2):103-10.
83. Pandian, A., Q. Liu, C. Hurlestone, S. Singh, P. Salisbury, and A. Green. 2004. Development of nutritionally superior Brassica napus and B. juncea oils using RNAi-mediated gene silencing. 4th Int. Crop Sci. Cong. Available at http.//www.cropscience.org.au/ icsc2004/poster/5/1/3/703_pandiana.htm (verified 23 January 2006).
84. Paulmann, W. and Frauen, M. (1991): Einsatz von biotechnologischen Verfahren in derpraktischen Rapszüchtung. Bericht der Arbeitstagung Saatzuchtleiter, Gumpenstein, 173-182
85. Paulmann, W. and Frauen, M. (1999) Proceedings of the 10th international Rapeseed Congress; http://www.regional.org.aulau/gcirc/4/258.htm
86. Pellan-Delourme, R. and Renard, M. 1988. Genome 30:234-238.
87. Pellan-Delourme, R., Eber, F., Renard, M. 1987. Male fertility restoration in Brassica napus with radish cytoplasmic male sterility.Proc. 7th Int. Rapeseed Conf., Poznan, Poland: 199-203.
88. Pelletier G., C. Primard. "Molecular. Phenotypic and Genetic Characterization of Mitochondrial Recombinants in Rapeseed." Proc. 7 th Int. Rapeseed Conf. Poznau. Poland 113-118 (1987).
89. Pelletier, G., C. Primard, F. Vedel, P. Chetrit, R. Remy, P. Rousselle and M. Renard. 1983. Mol Gen Genet 191: 244-250.
90. Phatak SC and Jaworski CA. 1989. HortScience 24:1050.
91. Pinochet X et al. (2000) OCL-Leagineux Corps Gras Lipides 7:11-16
92. Pierik R (1999) In vitro culture of higher plants. Kluwer Academic Publishers, Dordrecht
93. Pleines, S., and W. Friedt. 1989. Genetic control of linolenic acid concentration in seed oil of rapeseed (Brassica napus L.). Theor. Appl. Genet 78:793-797.
94. Przybylski, R. 2005. Canola oil:physical and chemical properties.Available at Canola Council of Canada http: //www.canola-councii.org/ oii_tech.html (verified 23 January 2006). Rakow, G. 2004. Species origin and economic importance of Brassica. p. 3-11. In E.C. Pua and C.J. Douglas (ed.). Biotechnology in Agriculture and Forestry, Vol. 54 Brassica. Springer-Verlag Berlin Heidelberg, New York.
95. Rakow, G. and D.I. McGregor. J. Am. Oil Chem. Soc. 50(10): 400403, (1973).
96. Raney, J.P., G.F.W. Rakow, and T.V. Olson. 1999. Identification of Brassica napus germplasm with seed oil low in saturated fat In Proc. 10th Int. Rapeseed Cong.: New horizons for an old crop, Canberra, Australia. Available at http://www.regional.org.au/au/ gcirc/4/502.htm (verfied 23 January 2006).
97. Rapley R., Harbron S. (Eds.) (2004) Chichester. John Wiley & Sons Ltd.
98. Ratledge, Colin, Dawson, Peter and Rattray, James.1984. Biotechnology for the Oils and Fats Industry. American Oil Chemists' Society, Champaign. 328pp
99. Renard, M., Delourme, R., Vallée, P. and Pierre, J. 1997: Acta Hortculturae 459: 583-591.
100. Riaz A., U G., Quresh Z., Swati M.S., Quiros C.F. (2001) Plant Breed. 120, 411-15.
101. Rick CM. 1948. Hilgardia 18: 599-633.
102. Röbbelen, G. (1985): Züchtung von Hybridraps. Bericht der Arbeitstagung Saatzuchtleiter, Gumpenstein, 173-185
103. R6bbelen, G., and A. Nitsch. 1975. Z. Pflanzenzüchtg. 75:93-105.
104. R6bbelen, Gerhard. "Changes and Limitations of Breeding for Improved Polyenic Fatty Acids Content in Rapeseed." (Chapter 10) in "Biotechnology for the Oils and Fats Industry" edited by Colin Ratledge, Peter Dawson and James Rattray, American Oil Chemists' Society, (1984).
105. Rücker, B., and G. Röbbelen. 1995. Development of high oleic acid winter rapeseed. p. 389-391. In Proc. 9th Int. Rapeseed Cong., Cambridge, UK.
106. Rundfeldt H. 1981. Z Pflanzenzucht 44: 30-62.
107. Sambrook J and Russell DW (2001) Molecular Cloning: A Laboratory Manual. Cold Spring Harbor Laboratory Press, Cold Spring Harbor, New York.
108. Sauermann und Lamp, Landpost 7.6.1997; p.36 re.
109. Sawhney VK. 1983. J. Hered. 74: 51-54.
110. Scarth, R., and P.B. E. McVetty. 1999. Designer oil canola- a review of new food-grade Brassica oils with focus on high oleic, low linolenic types. In N. Wratten and P.A. Salisbury (ed.) Proc. 10th Int. Rapeseed Cong., Canberra, Australia. http://www.regional.org.au/ au/gcirc/4/57.htm (verified 23 January 2006).
111. Scarth, R., P.B.E. McVetty, and S.R. Rimmer. 1995a. Mercury high erucic acid low glucosinolate summer rape. Can. J. Plant Sci. 75: 205-206.
112. Scarth, R., P.B.E. McVetty, S.A. Rimmer, and B.R. Stefansson. 1991. Hero summer rape. Can. J. Plant Sci. 71:865-866.
113. Scarth, R., P.B.E. McVetty, S.R. Rimmer, and J. Daun. 1992. Breeding for special oil quality in canola/rapeseed: The University of Manitoba program, p. 171-176. In S.L. MacKenzie and D.C. Taylor (ed.) Seed oils for the future. AOCS Press, Champaign, IL
114. Scarth, R., S.R. Rimmer, and P.B.E. McVetty. 1995b. Apollo low linolenic summer rape. Can. J. Plant Sci. 75: 203-204.
115. Scarth R and Tang J.H. (2006) Modification of Brassica Oil Using Conventional and Transgenic Approaches. Crop Sci. 46:1225-1236.
116. Schierhott, A., and H.C. Becker. 1999. Genetic and environmental variability of high oleic acid content in winter oilseed rape. In Proc 10th int. Rapeseed Cong., Canberra, Australia. Available at http:// www.regional.org.au/au/ gcirc/4/363.htm (verified 23 January 2006).
117. Schuster, W. (1969): Vergleich von zwei Zuchtverfahren in der Erhaltungszüchtung von Winterraps. Zeitschrift für Pflanzenzüchtung 62: 47-62
118. Sernyk, J. L. and Stefansson, B. R. (1983): Heterosis in summer rape. Canadian Journal of Plant Science 63: 407-413.
119. Sharma, R., R.A. Aggarwal, R. Kumar, T. Mohapatra, and R.P. Sharma. 2002. Construction of an RAPD linkage map and localization of QTLs for oleic acid level using recombinant inbreds in mustard (Brassica juncea). Genome 45:467-472.
120. Shen JX et al. (2005) Plant Breed 124:111-116
121. Shifriss C. 1997. Euphytica 93: 83-88.
122. Shull, G. H., 1922: Über die Heterozygotie mit Rücksicht auf den praktischen Züchtungserfolg. Beiträge zur Pftanzenzücht., 5. Heft, 134-158.
123. Song,L.Q., et al., 2005 :Genetic verification of multiple allelic gene for dominant genic male sterility in 609 AB (Brassica napus L). Acta Agron. Sin. 31 (7):869-875.
124. Sosulski F.W. and K.J. Dabrowski. 1984. Determination of glucosinolates in canola meal and protein products by desulfatation and capillary gas-liquid chromatography. Journal of Agriculture and Food Chemistry. 32: 1172-1175.
125. Stefansson, B.R. "The Development of Improved Rapeseed Cultivars." (Chapter 6) in "High and Low Erucic Acid Rapeseed Oils" edited by John K.G. Kramer, John K.G., Frank D. Sauer. and Wallace J. Pigden. Academic Press Canada, Toronto (1983).
126. Stefansson, B.R., F.W. Hougen, and R.K. Downey. 1961. Note on the isolation of rape plants with seed oil free from erucic acid. Can. J. Plant Sci. 41:218-219.
127. Syvanen A.C. (2001) Nat Rev Genet. 2(12):930-42.
128. Takagi, Y. (1970): Monogenic recessive male sterility in oil rape (Brassica napus L.) induced by gamma irradiation. Zeitschrift für Pflanzenzüchtung 64: 242-247.
129. Taylor, D.C., D.L Barton, E.M. Giblin, S.L. MacKenzie, C.G.J. van den Berg, and P.B.E. McVetty. 1995. Microsomal lyso-phosphatidic acid acyltransferase from a Brassica oleracea cultivar incorporates erucic acid into the sn-2 position of seed triacylglcerols. Plant Physiol. 109:409-420.
130. Theis R (1990) Thesis, University of Goettingen, Germany
131. Thelen, J.J., and J.B. Ohlrogge. 2002. Metabolic engineering of fatty acid biosynthesis in plants. Metab. Eng. 4:12-21.
132. Töpfer, R., N. Martini, and J. Schell. 1995. Science 268:681-686.
133. Tu, J. X., T. D. Fu, and Y. L. Zheng,1997a: Analysis on inheritance and isolocus of the rapeseed GMS90-2441A (B. napus L.). J. Huazhong Agri. Univ. 16, 255-258.
134. Tu, J. X., Y. L. Zheng, and T. D. Fu, 1997b: RAPD markers linked to genetic male sterile gene of rapeseed. J. Huazhong Agri. Univ. 16, 112-117.
135. Tu et al.. 1999. Studies on the recessive genic male sterility and its genetic markers in Rapeseed (Brassica napus L.); http:/Avww.regional.org.au/au/gcirc/4/87.htm; 10th rapeseed congress, Canberra Australia.
136. US 5,254,802.
137. van de Loo, F.J., B.G. Fox, and C. Somerville. 1993. Unusual fatty acids. p. 91-126. In T.S. Moore Jr (ed.) Lipid metabolism in plants. CRC, Boca Raton, FL. Vilkki, J.P., and P.K. Tanhuanpää. 1995. Breeding of high oleic acid spring turnip rape in Finland. p. 386-388. In Proc. 9th Int. Rapeseed Cong., Cambridge, UK.
138. Vilkki, J.P., and P.K. Tanhuanpää. 1995. Breeding of high oleic acid spring turnip rape in Finland.In Proc. 9th Int. Rapeseed Cong., Cambridge, UK, p. 386-388.
139. Voelker, T.A., A. Jones, A.M. Cranmer, H.M. Davies, and D.S. Knutzon. 1997. Plant Physiol. 114:669-677.
140. Voelker, T.A., T.R. Hayes, A.M. Cranmer, J.C. Turner, and H.M. Davies. 1996. Plant J. 9:229-241.
141. Wang, H., X. H. Tang, and Z. X. Zhao, 2001: Genetic study on ecotype genetic male sterile of H90s in Brassica napus L. Chinese J. Oil Crop Sci. 23,11-15.
142. Weight PAL, R.K. Scougall, D.W.F. Shannon and J.W. Wells. Role of glucosinolates in the causation of liver haemorrhages in laying hens fed water-extracted or heat-treated rapeseed cakes. Res. Vet. Sci. (1987) 43, pp 313-319.
143. Williams ME, Lecmans J and Michiels F.1997. Male sterility through recombinant DNAtechnology. In: Shivanna KR and Sawhney VK (eds), Pollen Biotechnology for Crop Production and Improvement. Cambridge Univ. Press, pp. 237-257.
144. Williams ME, LeemansJ, Michiels F (1997) Male sterility through recombinant DNA technology. In KR Shivanna, VK Sawhney, eds, Pollen Biotechnology for Crop Production and Improvement. Cambridge University Press, Cambridge, UK, pp 237-257
145. Williams P., Sobering D. 1992; In: Hildrum K., Isaksson T., Naes T. and Tandberg A. (eds.) Near Infra-red Spectroscopy. Bridging the gap between Data Analysis and NIR Applications. Horwood Chichester,UK: 41-446.
146. Williams, J. G. K., Kubelik, A. R., Kenneth, J. L., Rafalski, J. A. und Tingey, S. V. 1990: Nucl. Acids Res. 18: 6531-6535.
147. Wong, R., J.D. Patel, I. Grant, J. Parker, D. Chame, M. Elhalwagy, and E. Sys. 1991. The development of high oleic canola. p. 53. In Abstracts, 8th Int. Rapeseed Cong., Saskatoon, Canada.
148. Wu et al., Genomics 4:560 569 (1989)
149. Yang G S and Fu T D. Environment effects on the cytoplasmic male sterility of rapeseed (Brassica napus and Brassica campestris). Oil Crops of China, 1987; 3:15-19

## Claims

1. A method for producing or multiplying seed of a conditionally male sterile *Brassica napus* line with the genotype MsMsrfrf, said method comprising the steps of
a) providing a conditionally male sterile *Brassica napus* plant with the genotype MsMsrfrf, wherein said conditionally male sterile *Brassica napus* plant is
i. homozygous for the male sterility allele (Ms allele) obtainable from the *Brassica napus* seed deposited under Deposit Number NCIMB 41480, and
ii. homozygous for the maintainer allele (rf allele) obtainable from the *Brassica napus* seed deposited under Deposit Number NCIMB 41480, and
iii. predominantly male sterile when exposed before and/or during flowering to a temperature of less than 28°C, and
iv. reverting to a predominantly male fertile phenotype when exposed before and/or during flowering to a temperature of higher than 35°C,
wherein said genotype MSMSrfrf is obtainable from the *Brassica napus* seed deposited under Deposit Number NCIMB 41480,
b) exposing said conditionally male sterile *Brassica napus* plant for at least 4 hours to a temperature of higher than 35°C, and
c) exposing the heat-treated conditionally male sterile *Brassica napus* plant obtained in step (b) to a temperature of less than 33°C until development of male fertile flowers, and
d) allowing for self pollination of the *Brassica napus* plants having said male fertile flowers obtained in step (c), letting the seed develop, and harvesting the seed,
wherein the harvested seeds are **characterized in that** they are seeds of a conditionally male sterile *Brassica napus* line with the genotype MsMsrfrf.

2. A method for producing seed of a conditionally male sterile *Brassica napus* line with the genotype Msmsrfrf, said method comprising the steps of
a) providing as a female plant a conditionally male sterile *Brassica napus* line with the genotype MsMsrfrf by using the method according to claim 1 to obtain seeds of a conditionally male sterile *Brassica napus* line with the genotype MsMsrfrf and by growing said plants from said seeds, wherein said conditionally males sterile female *Brassica napus* plant with the genotype MsMsrfrf is
i. homozygous for the male sterility allele (Ms) obtainable from the *Brassica napus* seed deposited under Deposit Number NCIMB 41480, and
ii. homozygous for the maintainer allele (rf allele) obtainable from the *Brassica napus* seed deposited under Deposit Number NCIMB 41480 or 41481, and
iii. predominantly male sterile at a temperature of less than 28°C, and
iv. reverting to a male fertile phenotype at a temperature of higher than 35°C,
v. obtainable from the *Brassica napus* seed deposited under Deposit Number NCIMB 41480, and
b) providing as a male plant a male fertile *Brassica napus* plant with the genotype msmsrfrf, wherein said *Brassica napus* plant with the genotype msmsrfrf is
i. homozygous for the fertility allele (ms allele) obtainable from the *Brassica napus* seed deposited under Deposit Number NCIMB 41481, and
ii. homozygous for the maintainer allele (rf allele) obtainable from the *Brassica napus* seed deposited under Deposit Number NCIMB 41480 or 41481, and
iii. predominantly male fertile,
iv. obtainable from the *Brassica napus* seed deposited under Deposit Number NCIMB 41481, and
c) allowing the male plant of step b) to pollinate the female plant of step a), letting the seed develop, and harvesting the seed, wherein the harvested seeds are **characterized in that** they are seeds of a conditionally male sterile *Brassica napus* line with the genotype Msmsrfrf.

3. The method for producing or multiplying seed of a conditionally male sterile *Brassica napus* line with the genotype Msmsrfrf according to claim 2, wherein said male plant line and said female plant line are provided by introgression of the Ms, ms, and/or rf allele into an inbred *Brassica napus* line followed by at least one backcrossing against said inbred *Brassica napus* line.

4. A method for producing male fertile hybrid seed of *Brassica napus,* said method comprising the steps of
a) providing as a female plant a conditionally male sterile *Brassica napus* plant with the genotype Msmsrfrf or MsMsrfrf by using the method according to any of claims 1 to 3 to obtain seeds of a conditionally male sterile *Brassica napus* line with the genotype Msmsrfrf or MsMsrfrf and by growing said plants from said seeds, wherein said female conditionally male sterile *Brassica napus* plant is
i. heterozygous or homozygous for the male sterility allele (Ms allele) obtainable from the *Brassica napus* seed deposited under Deposit Number NCIMB 41480, and
ii. homozygous for the maintainer allele (rf allele) obtainable from the *Brassica napus* seed deposited under Deposit Number NCIMB 41480 or 41481, and
iii. predominantly male sterile at a temperature of less than 28°C, and
iv. reverting to a male fertile phenotype at a temperature of higher than 35°C, and,
b) providing as a male plant a male fertile *Brassica napus* plant with the genotype RfRf, wherein said male fertile *Brassica napus* plant is
i. homozygous for the functional restorer allele (Rf allele), which is obtainable from any fertile, inbred *Brassica napus* line commercialized as seed for growing, and
ii. predominantly male fertile, and
c) allowing the male plant of step b) to pollinate the female conditionally male sterile plant of step a), letting the seed develop, and harvesting said fertile hybrid seed.

5. The method for producing male fertile hybrid seed of *Brassica napus* according to claim 4, wherein said male (male fertile) plant line and said female (male sterile) plant line are based on genetically diverse background and/or wherein said female (male sterile) line is heterozygous for the Ms allele.

6. The method according to any one of claims 2 to 5, wherein the female (male sterile) and the male (male fertile) plants are grown in alternating stripes and/or wherein flowering of the male plants is delayed by cutting-back or treatment with growth-delaying chemicals or by sowing the male (male fertile) plants up to 3 weeks later than female (male sterile) plants.

7. A method for the production of *Brassica napus* hybrid seed which yields *Brassica napus* plants producing grain optionally with a total glucosinolate content of not more than 25 µmol per gram of air-dry seed at 9% humidity, wherein said method comprises one or more of the methods as claimed in
a) claim 1, and
b) any one of claim 2 and 3, and
c) any one of claim 4 to 6.

8. The method of any one of claims 1 to 7, wherein the Ms allele is **characterized by** conferring a conditional nuclear male sterile phenotype, which
a) is restored temporarily to fertility by an exposure to a temperature of higher than 35°C,
b) is restored to fertility in at least part of the F₁ plants obtained from crossing a male sterile plant with the genotype MsMsrfrf or Msmsrfrf with any *Brassica napus* plant comprising at least one dominant Rf allele, and
c) is maintained in the F₁ plants obtained from crossing a plant with a conditional male sterile phenotype referred by said Ms allele with the male fertile plants derived from seed deposited under Deposit Number NCIMB 41481.

9. The method of any one of claims 1 to 8, wherein the Ms allele is the Ms allele present in the seed deposited under Deposit Number NCIMB 41480 or a genetic variant thereof, which is conferring a conditionally male sterile phenotype.

10. The method of any one of claims 1 to 9, wherein the conditionally male sterile phenotype and/or the Ms allele is linked to and/or associated with one or more characteristic selected from the group consisting of
I. a phenotype of bud abortion in a plant with a male sterile phenotype conferred by the Ms allele,
II. a phenotype of white-striped or white blotched petals in a plant with a male sterile phenotype conferred by the Ms allele, and
III. the presence of a Ms allele specific marker in both male fertile and male sterile plants comprising at least one copy of the Ms allele.

11. The method of any one of claims 1 to 10, wherein the conditionally male sterile phenotype and/or the Ms allele is linked to and/or associated with one or more marker selected from the group consisting of
I. the markers selected from the group of polymorphisms in the NR1116 marker region consisting of
a) the single nucleotide polymorphism marker having a A at the position corresponding to position 85 in SEQ ID NO:3,
b) the single nucleotide polymorphism marker having a G at the position corresponding to position 87 in SEQ ID NO:3,
c) the single nucleotide polymorphism marker having an A at the position corresponding to position 139 in SEQ ID NO:3,
d) the single nucleotide polymorphism marker having a C at the position corresponding to position 214 in SEQ ID NO:3,
e) the single nucleotide polymorphism marker having a G at the position corresponding to position 218 in SEQ ID NO:3,
f) the single nucleotide polymorphism marker having a G at the position corresponding to position 277 in SEQ ID NO:3,
g) the single nucleotide polymorphism marker having an A at the position corresponding to position 286 in SEQ ID NO:3,
h) the single nucleotide polymorphism marker having a T at the position corresponding to position 312 in SEQ ID NO:3,
i) the single nucleotide polymorphism marker having a T at the position corresponding to position 319 in SEQ ID NO:3,
j) the single nucleotide polymorphism marker having a C at the position corresponding to position 359 in SEQ ID NO:3,
k) the deletion mutation 5'- TTGGTGAACAATC -3' at the position corresponding to 221 in SEQ ID NO:3, and
l) the insertion mutation 5'- GAA -3' at the position corresponding to 328-330 in SEQ ID NO:3
II. the markers selected from the group of polymorphisms in the NR2525 marker region consisting of
a) the single nucleotide polymorphism marker having a A at the position corresponding to position 60 in SEQ ID NO: 6,
b) the single nucleotide polymorphism marker having a T at the position corresponding to position 92 in SEQ ID NO:6,
c) the single nucleotide polymorphism marker having a T at the position corresponding to position 105 in SEQ ID NO: 6,
d) the single nucleotide polymorphism marker having a C at the position corresponding to position 158 in SEQ ID NO: 6,
e) the single nucleotide polymorphism marker having a T at the position corresponding to position 431 in SEQ ID NO: 6,
f) the single nucleotide deletion mutation at the position corresponding to position 82 in SEQ ID NO: 6, and
g) the deletion mutation 5'-TGAGCAAAA-3' at the position corresponding to position 17 to 25 in SEQ ID NO:6,
III. the markers selected from the group of SNP markers consisting of
a) a positive signal in a SNP assay using a SNP-probe comprising the nucleotide sequence described by SEQ ID NO: 12 and a negative signal using a SNP-probe comprising the nucleotide sequence described by SEQ ID NO: 11, and
b) a positive signal in a SNP assay using a SNP-probe comprising the nucleotide sequence described by SEQ ID NO: 17 and a negative signal using a SNP-probe comprising the nucleotide sequence described by SEQ ID NO: 18,
IV. the markers selected from the group of SSR markers consisting of:
a) a PCR fragment with an apparent molecular weight of 96.7 (+/- 1.0) bp resulting from a PCR reaction with the primers having the sequences set forth as SEQ ID NOs: 1 and 2, and
b) a PCR fragment with an apparent molecular weight of 192.8 (+/- 0.3) bp resulting from a PCR reaction with the primers having the sequences set forth as SEQ ID NOs: 4 and 5, and
V. the markers selected from the group of markers linked to one at least one of the sequences set forth as SEQ ID NOs: 3, 6, 11 and 18,
wherein the one or more marker (Ms allele marker) also includes an isolated nucleotide sequence selected from the group consisting of sequences which
I. have a sequence identity of at least 80% to, or
II. hybridize under stringent conditions to, or
III. comprise at least 25 consecutive nucleotides of
the marker sequences defined above in sections I. to V..

12. The method of any one of claims 1 to 7, wherein the ms allele is **characterized by** the phenotypic properties of
a) not being capable of reverting to fertility the male sterile phenotype conferred by the Ms allele, and
b) not being capable of conferring a male sterile phenotype in absence of a Ms allele.

13. The method of any one of claims 1 to 7 and 12, wherein the ms allele is linked to and/or associated with one or more marker selected from the group consisting of
I. the markers selected from the group of polymorphisms in the NR1116 marker region consisting of
a) the single nucleotide polymorphism marker having a G at the position corresponding to position 85 in SEQ ID NO:3,
b) the single nucleotide polymorphism marker having an A at the position corresponding to position 87 in SEQ ID NO:3,
c) the single nucleotide polymorphism marker having a T at the position corresponding to position 139 in SEQ ID NO:3,
d) the single nucleotide polymorphism marker having a T at the position corresponding to position 214 in SEQ ID NO:3,
e) the single nucleotide polymorphism marker having a T at the position corresponding to position 218 in SEQ ID NO:3,
f) the single nucleotide polymorphism marker having a A at the position corresponding to position 277 in SEQ ID NO:3,
g) the single nucleotide polymorphism marker having a G at the position corresponding to position 286 in SEQ ID NO:3,
h) the single nucleotide polymorphism marker having an A at the position corresponding to position 312 in SEQ ID NO:3,
i) the single nucleotide polymorphism marker having a C at the position corresponding to position 319 in SEQ ID NO:3,
j) the single nucleotide polymorphism marker having a T at the position corresponding to position 359 in SEQ ID NO:3,
k) the insertion mutation 5'-TTGGTGAACAATC-3' at the position corresponding to 221 in SEQ ID NO:3, and
l) the deletion mutation 5'-GAA-3' at the position corresponding to 328-330 in SEQ ID NO:3
II. the markers selected from the group of polymorphisms in the NR2525 marker region consisting of
a) the single nucleotide polymorphism marker having a C at the position corresponding to position 60 in SEQ ID NO: 6,
b) the single nucleotide polymorphism marker having a C at the position corresponding to position 92 in SEQ ID NO:6,
c) the single nucleotide polymorphism marker having a C at the position corresponding to position 105 in SEQ ID NO: 6,
d) the single nucleotide polymorphism marker having an A at the position corresponding to position 158 in SEQ ID NO: 6,
e) the single nucleotide polymorphism marker having a C at the position corresponding to position 431 in SEQ ID NO: 6,
f) the single nucleotide polymorphism marker having a T at the position corresponding to position 82 in SEQ ID NO: 6, and
g) the insertion mutation 5'-TGAGCAAAA-3' at the position corresponding to position 17 to 25 in SEQ ID NO:6,
III. the markers selected from the group of SNP markers consisting of
a) a positive signal in a SNP assay using a SNP-probe comprising the nucleotide sequence described by SEQ ID NO: 11 and a negative signal using a SNP-probe comprising the nucleotide sequence described by SEQ ID NO: 12, and
b) a positive signal in a SNP assay using a SNP-probe comprising the nucleotide sequence described by SEQ ID NO: 18 and a negative signal using a SNP-probe comprising the nucleotide sequence described by SEQ ID NO: 17,
IV. the markers selected from the group of SSR markers consisting of:
a) a PCR fragment with an apparent molecular weight selected from the group of apparent weights consisting of 94 (+/- 0.9) bp, 110.4 (+/- 0.5) bp, 112.3 (+/- 0.4) bp, and 116.3 (+/- 0.4) bp resulting from a PCR reaction with the primers having the sequences set forth as SEQ ID NOs: 1 and 2, and
b) a PCR fragment with an apparent molecular weight of 183.8 (+/- 0.4) bp or no fertile allele associated PCR fragment resulting from a PCR reaction with the primers having the sequences set forth as SEQ ID NOs: 4 and 5.
wherein the one or more marker also includes an isolated nucleotide sequence selected from the group consisting of sequences which
a) have a sequence identity of at least 80% to, or
b) hybridize under stringent conditions to, or
c) comprise at least 25 consecutive nucleotides of the marker sequences defined above in sections I. to IV.

14. The method of any one of claims 1 to 7, wherein the rf allele is **characterized by** the phenotypic properties of
a) not being capable of reverting to fertility the male sterile phenotype conferred by the Ms allele, and
b) being capable of maintaining the male sterile phenotype conferred by the Ms allele.

15. The method of any one of claims 1 to 7 and 14, wherein the rf allele is selected from the group consisting of
a) the rf allele obtainable from the *Brassica napus* seed deposited under Deposit Number NCIMB 41480 or 41481, and
b) variants thereof, which are in a homozygous form capable of maintaining the male sterility phenotype conferred by the Ms allele.

16. The method of any one of claims 1 to 7, 14 and 15, wherein the rf allele is linked to and/or associated with the SSR markers consisting of a PCR fragment with an apparent molecular weight of 240.8 (+/- 0.4) bp resulting from a PCR reaction with the primers having the sequences set forth as SEQ ID NOs: 19 and 20.

17. The method of any one of claims 1 to 7, wherein the fertility restoring phenotype and/or the Rf allele is linked to and/or associated with one or more characteristic selected from the group consisting of
a) restore fertility in the F₁ plants obtained from crossing with the *Brassica napus* plant grown from seed deposited under Deposit Number NCIMB 41480, and
b) restore fertility in the F₁ plants obtained from crossing with the *Brassica napus* plant grown from the seed obtained from crossing of the *Brassica napus* plant obtained from the seed deposited under Deposit Number NCIMB 41480 as a female male sterile plant and the *Brassica napus* plant obtained from the seed deposited under Deposit Number NCIMB 41481 as a male fertile plant.

18. The method of any one of claims 1 to 17, wherein the Rf allele and/or the ms allele is obtained from a commercially available fertile inbred *Brassica napus* line selected from the group consisting of the non-hybrid *Brassica napus* varieties commercialized as seed for growing purpose included in the OECD List of varieties eligible for certification of December 2006.

19. The method of any one of claims 1 to 7 and 17 to 18, wherein the fertility restoring phenotype and/or the Rf allele is linked and/or associated with a SSR marker consisting of the absence of a PCR fragment with an apparent molecular weight of 240.8 (+/-0.4) bp resulting from a PCR reaction with the primers having the sequences set forth as SEQ ID NOs: 19 and 20.

20. An oligonucleotide primer selected from the group of sequences described by SEQ ID NOs: 1, 2, 4, 5, 7, 8, 9, 10, 11, 12, 13, 14, 15, 16, 17, 18, 19, and 20.

21. A probe comprising as the nucleic acid part a sequence selected from the group of sequences described by SEQ ID NOs: 11, 12, 17, and 19, wherein the probe is suitable for the detection of a single nucleotide polymorphism.

22. An isolated nucleotide sequence selected from the group consisting of the sequences set forth as SEQ ID NOs: 3, 6, and 21, wherein said isolated nucleotide sequences are markers useful for detecting the Ms allele, ms allel, Rf allele and/or rf allele in *Brassica* germplasm and the associated phenotypes.

23. Use of a nucleic acid sequence according to claims 20 or 22 in marker-based selection for introgressing alleles selected from the group consisting of the Ms allele, ms allele, Rf allele, and/or rf allele into a Brassica germplasm lacking said set of alleles.

24. Use of a male fertile *Brassica napus* plant with the genotype RfRf in a method of producing fertile hybrid seed of *Brassica napus* according to any one of claims 4 to 6.

25. Use according to claim 23, wherein the method is a method as claimed in any one of claims 4 to 19.

26. Use of one or more *Brassica napus* plants selected from conditionally male sterile *Brassica napus* plants with the genotype MsMsrfrf and/or male fertile *Brassica napus* plants with the genotype msmsrfrf in the method according to any of claims 2, 3, and 6 to 19 for producing a conditionally male sterile *Brassica napus* plant with the genotype Msmsrfrf or seed thereof.

## Patentansprüche

1. Verfahren zur Produktion oder Vermehrung von Saatgut einer bedingt männlich sterilen *Brassica napus*-Linie mit dem Genotyp MsMsrfrf, wobei das Verfahren die folgenden Schritte umfaßt:
a) Bereitstellung einer bedingt männlich sterilen *Brassica napus*-Pflanze mit dem Genotyp MsMsrfrf, wobei diese bedingt männlich sterile *Brassica napus-*Pflanze
i. für das Gen für männliche Sterilität (Ms-Allel) homozygot ist, das aus dem mit der Hinterlegungsnummer NCIMB 41480 hinterlegten Brassica *napus*-Saatgut erhältlich ist, und
ii. für das Maintainer-Allel (rf-Allel) homozygot ist, das aus dem mit der Hinterlegungsnummer NCIMB 41480 hinterlegten *Brassica napus-*Saatgut erhältlich ist, und
iii. überwiegend männlich steril ist, wenn sie vor und/oder während der Blüte einer Temperatur von unter 28°C ausgesetzt ist, und
iv. zu einem überwiegend männlich fertilen Phänotyp zurückkehrt, wenn sie vor und/oder während der Blüte einer Temperatur von mehr als 35°C ausgesetzt ist,
wobei der Genotyp MsMsrfrf aus dem mit der Hinterlegungsnummer NCIMB 41480 hinterlegten *Brassica napus*-Saatgut erhältlich ist, und
b) Aussetzen dieser bedingt männlich sterilen *Brassica napus*-Pflanze füer mindestens vier Stunden einer Temperatur von mehr als 35°C, und
c) Aussetzen der hitzebehandelten bedingt männlich sterilen *Brassica napus-*Pflanze, die in Schritt b) erhalten wurde, einer Temperatur von unter 33°C bis zur Entwicklung von männlich fertilen Blüten, und
d) Ermöglichen der Selbstbefruchtung der *Brassica napus*-Pflanzen mit diesen männlich fertilen Blüten, die in Schritt c) erhalten wurden, Ermöglichen der Entwicklung des Saatguts und Ernten des Saatguts, wobei das geerntete Saatgut **dadurch gekennzeichnet sind, daß** es sich um Saatgut einer bedingt männlich sterilen *Brassica napus*-Linie mit dem Genotyp MsMsrfrf handelt.

2. Verfahren zur Produktion von Saatgut einer bedingt männlich sterilen *Brassica napus*-Linie mit dem Genotyp Msmsrfrf, wobei das Verfahren die folgenden Schritte umfaßt:
a) Bereitstellen einer bedingt männlich sterilen *Brassica napus*-Linie mit dem Genotyp MsMsrfrf als weibliche Pflanze, indem das Verfahren gemäss Anspruch 1 zur Gewinnung von Saatgut einer bedingt männlich sterilen *Brassica napus*-Linie mit dem Genotyp MsMsrfrf eingesetzt wird und Pflanzen aus diesem Saatgut angezogen werden, wobei diese bedingt männlich sterile *Brassica napus*-Pflanze mit dem Genotyp MsMsrfrf
i. für das Gen für männliche Sterilität (Ms-Allel) heterozygot ist, das aus dem mit der Hinterlegungsnummer NCIMB 41480 hinterlegten *Brassica napus*-Saatgut erhältlich ist, und
ii. für das Maintainer-Allel (rf-Allel) homozygot ist, das aus dem mit der Hinterlegungsnummer NCIMB 41480 hinterlegten *Brassica napus-*Saatgut erhältlich ist, und
iii. bei einer Temperatur von unter 28°C überwiegend männlich steril ist, und
iv. bei einer Temperatur von mehr als 35°C zu einem überwiegend männlich fertilen Phänotyp zurückkehrt,
v. aus dem mit der Hinterlegungsnummer NCIMB 41480 hinterlegten *Brassica napus*-Saatgut erhältlich ist, und
b) Bereitstellen einer männlich fertilen *Brassica napus-*Pflanze mit dem Genotyp msmsrfrf als männliche Pflanze, wobei diese *Brassica napus*-Pflanze mit dem Genotyp msmsrfrf
i. für das Fertilitäts-Allel (ms-Allel) homozygot ist, das aus dem mit der Hinterlegungsnummer NCIMB 41481 hinterlegten *Brassica napus-*Saatgut erhältlich ist, und
ii. für das Maintainer-Allel (rf-Allel) homozygot ist, das aus dem mit der Hinterlegungsnummer NCIMB 41480 oder 41481 erhältlichen *Brassica napus*-Saatgut erhältlich ist, und
iii. überwiegend männlich fertil ist,
iv. aus dem mit der Hinterlegungsnummer NCIMB 41481 hinterlegten *Brassica napus*-Saatgut erhältlich ist, und
c) Ermöglichen der Bestäubung der weiblichen Pflanze aus Schritt a) durch die männliche Pflanze aus Schritt b), Ermöglichen der Entwicklung des Saatguts und Ernten des Saatguts, wobei das geerntete Saatgut **dadurch gekennzeichnet sind, daß** es sich um Saatgut einer bedingt männlich sterilen *Brassica napus*-Linie mit dem Genotyp Msmsrfrf handelt.

3. Verfahren zur Produktion oder Vermehrung von Saatgut einer bedingt männlich sterilen *Brassica napus*-Linie mit dem Genotyp Msmsrfrf gemäss Anspruch 2, wobei die männliche Pflanzenlinie und die weibliche Pflanzenlinie durch Introgression des Ms, ms und/oder rf-Allels in eine *Brasscia napus*-Inzuchtlinie gefolgt von mindestens einmaligem Rückkreuzen gegen diese *Brassica napus-*Inzuchtlinie bereitgestellt werden.

4. Verfahren zur Produktion von männlich fertilem Hybridsaatgut von *Brassica napus,* wobei das Verfahren die folgenden Schritte umfaßt:
a) Bereitstellen einer bedingt männlich sterilen *Brassica napus*-Pflanze mit dem Genotyp Msmsrfrf oder MsMsrfrf als weibliche Pflanze, indem das Verfahren gemäss einem der Ansprüche 1 bis 3 zur Gewinnung von Saatgut einer bedingt männlich sterilen *Brassica napus*-Linie mit dem Genotyp Msmsrfrf oder MsMsrfrf eingesetzt wird und Pflanzen aus diesem Saatgut angezogen werden, wobei diese weibliche bedingt männlich-sterile *Brassica napus-*Pflanze
i. für das Allel für männliche Sterilität (Ms-Allel) heterozygot oder homozygot ist, das aus dem mit der Hinterlegungsnummer NCIMB 41480 hinterlegten *Brassica napus*-Saatgut erhältlich ist, und
ii. für das Maintainer-Allel (rf-Allel) homozygot ist, das aus dem mit der Hinterlegungsnummer NCIMB 41480 oder 41481 hinterlegten *Brassica napus*-Saatgut erhältlich ist, und
iii. bei einer Temperatur von unter 28°C überwiegend männlich steril ist, und
iv. bei einer Temperatur von über 35°C zu einem männlich fertilen Phänotyp zurückkehrt, und
b) Bereitstellen einer männlich fertilen *Brassica napus*-Pflanze mit dem Genotyp RfRf als männliche Pflanze, wobei diese männlich fertile *Brassica napus-*Pflanze
i. für das funktionale Restorer-Allel (Rf-Allel) homozygot ist, das aus einer beliebigen als Saatgut für den Anbau im Handel erhältlichen fertilen, *Brassica napus*-Inzuchtlinie erhältlich ist, und
ii. überwiegend männlich fertilist, und
c) Ermöglichen der Bestäubung der weiblichen bedingt männlich sterilen Pflanze aus Schritt a) durch die männliche Pflanze aus Schritt b), Ermöglichen der Entwicklung des Saatguts und Ernten des fertilen Hybridsaatguts.

5. Verfahren zur Produktion von männlich fertilem Hybridsaatgut von *Brassica napus* gemäss Anspruch 4, wobei die männliche (männlich fertile) Pflanzenlinie und die weibliche (männlich sterile) Pflanzenlinie auf genetisch diversen Hintergründen beruhen und/oder wobei die weibliche (männlich sterile) Linie für das Ms-Allel heterozygot ist.

6. Verfahren gemäß einem der Ansprüche 2 bis 5, wobei die weiblichen (männlich sterilen) und die männlichen (männlich fertilen) Pflanzen in sich abwechselnden Streifen angebaut werden und/oder wobei die Blüte der männlichen (männlich fertilen) Pflanzen durch Zurückschneiden oder Behandeln mit wachstumsverzögernden Chemikalien oder durch Aussäen der männlichen (männlich fertilen) Pflanzen bis zu 3 Wochen nach dem Aussäen der weiblichen (männlich sterilen) Pflanzen verzögert wird.

7. Verfahren zur Produktion von *Brassica napus*-Hybridsaatgut, das *Brassica napus-*Pflanzen hervorbringt, die optional Korn mit einem Gesamtglucosinolatgehalt von nicht mehr als 25 µmol pro Gramm luftgetrocknetem Samen bei 9% Feuchtigkeit produzieren, wobei das Verfahren eines oder mehrere der Verfahren gemäß
a) Anspruch 1, und
b) einem der Ansprüche 2 bis 3, und
c) einem der Ansprüche 4 bis 6
umfaßt.

8. Verfahren gemäß einem der Ansprüche 1 bis 7, wobei das Ms-Allel **dadurch gekennzeichnet ist, daß** es einen kerngenisch vererbten bedingt männlich sterilen Phänotyp vermittelt, der
a) durch Behandeln mit einer Temperatur von über 35°C vorübergehend zu Fertilität restauriert werden kann,
b) in mindestens einem Teil der F₁-Pflanzen zu Fertilität restauriert wird, die durch Kreuzen einer männlich sterilen Pflanze mit dem Genotyp MsMsrfrf oder Msmsrfrf mit einer beliebigen *Brassica napus*-Pflanze, die mindestens ein dominantes Rf-Allel enthält, erhalten werden, und
c) in den F₁-Pflanzen erhalten bleibt, die durch Kreuzen einer Pflanze mit einem bedingt männlich sterilen Phänotyp, der von diesem Ms-Allel vermittelt wird, mit den männlich fertilen Pflanzen erhalten werden, die von mit der Hinterlegungsnummer NCIMB 41481 hinterlegten Saatgut abstammen.

9. Verfahren gemäß einem der Ansprüche 1 bis 8, bei dem es sich bei dem Ms-Allel um das in dem mit der Hinterlegungsnummer NCIMB 41480 hinterlegten Saatgut oder einer genetischen Variante davon vorliegenden Ms-Allel handelt, das einen bedingt männlich sterilen Phänotyp vermittelt.

10. Verfahren gemäß einem der Ansprüche 1 bis 9, bei dem der bedingt männlich sterile Phänotyp und/oder das Ms-Allel mit einer oder mehreren Eigenschaft(en) gekoppelt und/oder assoziiert ist bzw. sind, das bzw. die aus der Gruppe bestehend aus
I. einem Phänotyp des Knospenabwurfs bei einer Pflanze mit einem männlich sterilen Phänotyp, dessen Ausprägung durch das Ms-Allel vermittelt wird,
II. einem Phänotyp mit weiß gestreiften oder weiß gesprenkelten Petalen bei einer Pflanze mit einem männlich sterilen Phänotyp, dessen Ausprägung durch das Ms-Allel vermittelt wird, und
III. dem Vorliegen eines für das Ms-Allel spezifischen Markers in sowohl männlich fertilen als auch männlich sterilen Pflanzen, die mindestens eine Kopie des Ms-Allels umfassen,
ausgewählt ist bzw. sind.

11. Verfahren gemäß einem der Ansprüche 1 bis 10, bei dem der bedingt männlich sterile Phänotyp und/oder das Ms-Allel mit einem oder mehreren Markern gekoppelt und/oder assoziiert ist, der bzw. die aus der Gruppe bestehend aus
I. den Markern, die aus der Gruppe der Polymorphismen in der NR1116-Markerregion bestehend aus
a) dem Einzelnukleotidpolymorphismus-Marker mit einem A an der Position, die der Position 85 in SEQ ID NO: 3 entspricht,
b) dem Einzelnukleotidpolymorphismus-Marker mit einem G an der Position, die der Position 87 in SEQ ID NO: 3 entspricht,
c) dem Einzelnukleotidpolymorphismus-Marker mit einem A an der Position, die der Position 139 in SEQ ID NO: 3 entspricht,
d) dem Einzelnukleotidpolymorphismus-Marker mit einem C an der Position, die der Position 214 in SEQ ID NO: 3 entspricht,
e) dem Einzelnukleotidpolymorphismus-Marker mit einem G an der Position, die der Position 218 in SEQ ID NO: 3 entspricht,
f) dem Einzelnukleotidpolymorphismus-Marker mit einem G an der Position, die der Position 277 in SEQ ID NO: 3 entspricht,
g) dem Einzelnukleotidpolymorphismus-Marker mit einem A an der Position, die der Position 286 in SEQ ID NO: 3 entspricht,
h) dem Einzelnukleotidpolymorphismus-Marker mit einem T an der Position, die der Position 312 in SEQ ID NO: 3 entspricht,
i) dem Einzelnukleotidpolymorphismus-Marker mit einem T an der Position, die der Position 319 in SEQ ID NO: 3 entspricht,
j) dem Einzelnukleotidpolymorphismus-Marker mit einem C an der Position, die der Position 359 in SEQ ID NO: 3 entspricht,
k) der Deletionsmutation 5'- TTGGTGAACAATC -3' an der Position, die 221 in SEQ ID NO: 3 entspricht,
l) der Insertionsmutation 5'- GAA -3' an der Position, die 328 bis 330 in SEQ ID NO: 3 entspricht,
ausgewählt sind,
II. den Markern, die aus der Gruppe der Polymorphismen in der NR2525-Markerregion bestehend aus
a) dem Einzelnukleotidpolymorphismus-Marker mit einem A an der Position, die der Position 60 in SEQ ID NO: 6 entspricht,
b) dem Einzelnukleotidpolymorphismus-Marker mit einem T an der Position, die der Position 92 in SEQ ID NO: 6 entspricht,
c) dem Einzelnukleotidpolymorphismus-Marker mit einem T an der Position, die der Position 105 in SEQ ID NO: 6 entspricht,
d) dem Einzelnukleotidpolymorphismus-Marker mit einem C an der Position, die der Position 158 in SEQ ID NO: 6 entspricht,
e) dem Einzelnukleotidpolymorphismus-Marker mit einem T an der Position, die der Position 431 in SEQ ID NO: 6 entspricht,
f) der Einzelnukleotiddeletionsmutation an der Position, die der Position 82 in SEQ ID NO:6 entspricht, und
g) der Deletionsmutation 5'- TGAGCAAAA -3' an der Position, die der Position 17 bis 25 in SEQ ID NO: 6 entspricht,
ausgewählt sind,
III. den Markern, die aus der Gruppe der SNP-Marker bestehend aus
a) einem positiven Signal in einem SNP-Assay unter Verwendung einer SNP-Sonde, die die Nukleotidsequenz gemäß SEQ ID NO: 12 umfasst, sowie einem negativen Signal unter Verwendung einer SNP-Sonde, die die Nukleotidsequenz gemäß SEQ ID NO: 11 umfasst, und
b) einem positiven Signal in einem SNP-Assay unter Verwendung einer SNP-Sonde, die die Nukleotidsequenz gemäß SEQ ID NO: 17 umfasst, sowie einem negativen Signal unter Verwendung einer SNP-Sonde, die die Nukleotidsequenz gemäß SEQ ID NO: 18 umfasst,
ausgewählt sind,
IV. den Markern, die aus der Gruppe der SSR-Marker bestehend aus:
a) einem PCR-Fragment mit einem scheinbaren Molekulargewicht von 96,7
(+/- 1,0) Bp, das das Ergebnis einer PCR-Reaktion mit den Primern mit den Sequenzen gemäß SEQ ID NO: 1 und 2 ist, und
b) einem PCR-Fragment mit einem scheinbaren Molekulargewicht von 192,8 (+/- 0,3) Bp, das das Ergebnis einer PCR-Reaktion mit den Primern mit den Sequenzen gemäß SEQ ID NO: 4 und 5 ist,
ausgewählt sind, und
V. den Markern aus der Gruppe der Marker, die mit mindestens einer der Sequenzen gemäß SEQ ID NO: 3, 6, 11 und 18 gekoppelt sind,
ausgewählt ist bzw. sind, wobei der eine oder die mehreren Marker (Ms-Allelmarker) ferner eine isolierte Nukleotidsequenz einschliesst bzw. einschliessen, die aus der Gruppe bestehend aus
I. Sequenzen, die mindestens 80 % Sequenzidentität mit den oben in I. bis V. definierten Markersequenzen aufweisen, oder
II. Sequenzen, die unter stringenten Bedingungen mit den oben in I. bis V. definierten Markersequenzen hybridisieren, oder
III. Sequenzen, die mindestens 25 aufeinanderfolgende Nukleotide der oben in I. bis V. definierten Markersequenzen aufweisen,
ausgewählt sind.

12. Verfahren gemäß einem der Ansprüche 1 bis 7, wobei das ms-Allel durch die folgenden phänotypischen Eigenschaften **gekennzeichnet** ist:
a) es ist nicht in der Lage, die Fertilität des von dem Ms-Allel vermittelten männlich sterilen Phänotyps wiederherzustellen, und
b) es ist nicht in der Lage, in Abwesenheit eines Ms-Allels einen männlich sterilen Phänotyp zu vermitteln.

13. Verfahren gemäß einem der Ansprüche 1 bis 7 und 12, wobei das ms-Allel mit einem oder mehreren Markern gekoppelt und/oder assoziiert ist, der bzw. die aus der Gruppe bestehend aus
I. den Markern, die aus der Gruppe der Polymorphismen in der NR1116-Markerregion bestehend aus
a) dem Einzelnukleotidpolymorphismus-Marker mit einem G an der Position, die der Position 85 in SEQ ID NO: 3 entspricht,
b) dem Einzelnukleotidpolymorphismus-Marker mit einem A an der Position, die der Position 87 in SEQ ID NO: 3 entspricht,
c) dem Einzelnukleotidpolymorphismus-Marker mit einem T an der Position, die der Position 139 in SEQ ID NO: 3 entspricht,
d) dem Einzelnukleotidpolymorphismus-Marker mit einem T an der Position, die der Position 214 in SEQ ID NO: 3 entspricht,
e) dem Einzelnukleotidpolymorphismus-Marker mit einem T an der Position, die der Position 218 in SEQ ID NO: 3 entspricht,
f) dem Einzelnukleotidpolymorphismus-Marker mit einem A an der Position, die der Position 277 in SEQ ID NO: 3 entspricht,
g) dem Einzelnukleotidpolymorphismus-Marker mit einem G an der Position, die der Position 286 in SEQ ID NO: 3 entspricht,
h) dem Einzelnukleotidpolymorphismus-Marker mit einem A an der Position, die der Position 312 in SEQ ID NO: 3 entspricht,
i) dem Einzelnukleotidpolymorphismus-Marker mit einem C an der Position, die der Position 319 in SEQ ID NO: 3 entspricht,
j) dem Einzelnukleotidpolymorphismus-Marker mit einem T an der Position, die der Position 359 in SEQ ID NO: 3 entspricht,
k) der Insertionsmutation 5'- TTGGTGAACAATC -3' an der Position, die 221 in SEQ ID NO: 3 entspricht, und
l) der Deletionsmutation 5'- GAA -3' an der Position, die 328 bis 330 in SEQ ID NO: 3 entspricht,
ausgewählt sind,
II. den Markern, die aus der Gruppe der Polymorphismen in der NR2525-Markerregion bestehend aus
a) dem Einzelnukleotidpolymorphismus-Marker mit einem C an der Position, die der Position 60 in SEQ ID NO: 6 entspricht,
b) dem Einzelnukleotidpolymorphismus-Marker mit einem C an der Position, die der Position 92 in SEQ ID NO: 6 entspricht,
c) dem Einzelnukleotidpolymorphismus-Marker mit einem C an der Position, die der Position 105 in SEQ ID NO: 6 entspricht,
d) dem Einzelnukleotidpolymorphismus-Marker mit einem A an der Position, die der Position 158 in SEQ ID NO: 6 entspricht,
e) dem Einzelnukleotidpolymorphismus-Marker mit einem C an der Position, die der Position 431 in SEQ ID NO: 6 entspricht,
f) dem Einzelnukleotidpolymorphismus-Marker mit einem T an der Position, die der Position 82 in SEQ ID NO: 6 entspricht, und
g) der Insertionsmutation 5'- TGAGCAAAA -3' an der Position, die der Position 17 bis 25 in SEQ ID NO: 6 entspricht,
ausgewählt sind,
III. den Markern, die aus der Gruppe der SNP-Marker bestehend aus
a) einem positiven Signal in einem SNP-Assay unter Verwendung einer SNP-Sonde, die die Nukleotidsequenz gemäß SEQ ID NO: 11 umfasst, und einem negativen Signal unter Verwendung einer SNP-Sonde, die die Nukleotidsequenz gemäß SEQ ID NO: 12 umfasst, und
b) einem positiven Signal in einem SNP-Assay unter Verwendung einer SNP-Sonde, die die Nukleotidsequenz gemäß SEQ ID NO: 18 umfasst, und einem negativen Signal unter Verwendung einer SNP-Sonde, die die Nukleotidsequenz gemäß SEQ ID NO: 17 umfasst,
ausgewählt sind, und
IV. den Markern aus der Gruppe der SSR-Marker bestehend aus:
a) einem PCR-Fragment mit einem scheinbaren Molekulargewicht, das aus der Gruppe scheinbarer Gewichte bestehend aus 94 (+/- 0,9) Bp, 110,4 (+/- 0,5) Bp, 112,3 (+/- 0,4) Bp und 116,3 (+/- 0,4) Bp ausgewählt ist, das das Ergebnis einer PCR-Reaktion mit den Primern mit der Sequenz gemäß SEQ ID NO: 1 und 2 ist, und
b) einem PCR-Fragment mit einem scheinbaren Molekulargewicht von 183,8 (+/- 0,4) Bp oder keinem mit einem fertilen Allel assoziierte PCR-Fragment, das das Ergebnis einer PCR-Reaktion mit den Primern mit der Sequenz gemäß SEQ ID NO: 4 und 5 ist,
ausgewählt ist bzw. sind, wobei der eine oder die mehreren Marker (ms-Allelmarker) ferner eine isolierte Nukleotidsequenz einschliesst bzw. einschliessen, die aus der Gruppe bestehend aus
I. Sequenzen, die mindestens 80 % Sequenzidentität mit den oben in I. bis IV. definierten Markersequenzen aufweisen, oder
II. Sequenzen, die unter stringenten Bedingungen mit den oben in I. bis IV. definierten Markersequenzen hybridisieren, oder
III. Sequenzen, die mindestens 25 aufeinanderfolgende Nukleotide der oben in I. bis IV. definierten Markersequenzen aufweisen,
ausgewählt sind.

14. Verfahren gemäß einem der Ansprüche 1 bis 7, bei dem das rf-Allel durch die folgenden phänotypischen Eigenschaften **gekennzeichnet** ist:
a) es ist nicht in der Lage, die Fertilität des von dem Ms-Allel vermittelten männlich sterilen Phänotyps wiederherzustellen, und
b) es ist in der Lage, den von dem Ms-Allel vermittelten männlich sterilen Phänotyp aufrechtzuerhalten.

15. Verfahren gemäß einem der Ansprüche 1 bis 7 und 14, bei dem das rf-Allel ausgewählt ist aus der Gruppe bestehend aus
a) dem rf-Allel, das aus dem mit der Hinterlegungsnummer NCIMB 41480 oder 41481 hinterlegten *Brassica napus*-Saatgut erhältlich ist, und
b) Varianten davon, die in homozygoter Form in der Lage sind, den von dem Ms-Allel vermittelten Phänotyp der männlichen Sterilität aufrechtzuerhalten.

16. Verfahren gemäß einem der Ansprüche 1 bis 7, 14 und 15, bei dem das rf-Allel mit den SSR-Markern gekoppelt und/oder assoziiert ist, die aus einem PCR-Fragment mit einem scheinbaren Molekulargewicht von 240,8 (+/- 0,4) Bp bestehen, das in einer PCR-Reaktion mit den Primern erhalten wird, die die Sequenzen gemäß SEQ ID NO: 19 und 20 aufweisen.

17. Verfahren gemäß einem der Ansprüche 1 bis 7, bei dem der die Fertilität wiederherstellende Phänotyp und/oder das Rf-Allel mit einem oder mehreren Eigenschaften gekoppelt und/oder assoziiert ist bzw. sind, der bzw. die aus der Gruppe bestehend aus
a) Wiederherstellung der Fertilität in den F₁-Pflanzen, die durch Kreuzen mit der *Brassica napus*-Pflanze, die aus dem mit der Hinterlegungsnummer NCIMB 41480 hinterlegten Saatgut herangezogen wurde, erhalten wurden, und
b) Wiederherstellung der Fertilität in den F₁-Pflanzen, die durch Kreuzen mit der *Brassica napus*-Pflanze, die aus dem Saatgut herangezogen wurde, das durch Kreuzen der *Brassica napus*-Pflanze, die aus dem mit der Hinterlegungsnummer NCIMB 41480 hinterlegten Saatgut erhalten wurde, als weibliche männlich sterile Pflanze und der *Brassica napus*-Pflanze, die aus dem mit der Hinterlegungsnummer NCIMB 41481 hinterlegten Saatgut erhalten wurde, als männlich fertile Pflanze erhalten wurden
ausgewählt ist bzw. sind.

18. Verfahren gemäß einem der Ansprüche 1 bis 17, wobei das Rf-Allel und/oder das ms-Allel aus einer im Handel erhältlichen fertilen *Brassica napus*-Inzuchtlinie erhältlich ist, die aus der Gruppe der *Brassica napus*-Nichthybridsorten ausgewählt ist, die als Saatgut für Anbauzwecke im Handel erhältlich sind und die in die OECD-Liste der Sorten, die für die Zertifizierung infrage kommen, vom Dezember 2006 aufgenommen wurden.

19. Verfahren gemäß einem der Ansprüche 1 bis 7 und 17 bis 18, bei dem der die Fertilität wiederherstellende Phänotyp und/oder das Rf-Allel mit einem SSR-Marker gekoppelt und/oder assoziiert ist bzw. sind, der aus dem Fehlen eines PCR-Fragments mit einem scheinbaren Molekulargewicht von 240,8 (+/- 0,4) Bp besteht, das das Ergebnis einer PCR-Reaktion mit den Primern ist, die die Sequenzen gemäß SEQ ID NO: 19 und 20 aufweisen.

20. Oligonukleotid-Primer ausgewählt aus der Gruppe der Sequenzen gemäß SEQ ID NO : 1, 2, 4, 5, 7, 8, 9, 10, 11, 12, 13, 14, 15, 16, 17, 18, 19 und 20.

21. Sonde, die sich für den Nachweis eines Einzelnukleotidpolymorphismus eignet, die als den Nukleinsäureteil eine Sequenz umfasst, die aus der Gruppe der Sequenzen gemäß SEQ ID NO: 11, 12, 17 und 19 ausgewählt ist.

22. Isolierte Nukleotidsequenz, die aus der Gruppe bestehend aus den Sequenzen gemäss SEQ ID NO: 3, 6 und 21 ausgewählt ist, wobei diese isolierten Nukleotidsequenzen Marker sind, die für den Nachweis des Ms-Allels, Rf-Allels, und/oder rf-Allels im *Brassica*-Keimplasma und den assoziierten Phänotypen geeignet sind.

23. Verwendung einer Nukleinsäuresequenz gemäss den Ansprüchen 20 oder 22 in einer markerbasierten Selektion für die Introgression von Allelen, die aus der Gruppe bestehend aus dem Ms-Allel, dem ms-Allel, dem Rf-Allel und/oder dem rf-Allel ausgewählt sind, in ein *Brassica*-Keimplasma, dem dieser Satz Allele fehlt.

24. Verwendung einer männlich fertilen *Brassica napus*-Pflanze mit dem Genotyp RfRf in einem Verfahren zur Produktion von fertilem Hybridsaatgut von *Brassica napus* gemäss einem der Anstrüche 4 bis 6.

25. Verwendung gemäss Anspruch 23 oder 24, wobei das Verfahren ein Verfahren gemäss einem der Ansprüch 4 bis 19 ist.

26. Verwendung einer oder mehrerer *Brassica napus*-Pflanze(n), die aus der Gruppe bestehend aus einer bedingt männlich sterilen *Brassica napus*-Pflanze mit dem Genotyp MsMsrfrf und einer männlich fertilen *Brassica napus*-Pflanze mit dem Genotyp msmsrfrf ausgewählt ist, in einem Verfahren gemäss einem der Ansprüche 2, 3 und 6 bis 19 zur Produktion einer bedingt männlich sterilen *Brassica napus*-Pflanze mit dem Genotyp Msmsrfrf oder deren Saatgut.

## Revendications

1. Procédé de production ou de multiplication d'une graine d'une lignée de *Brassica napus,* conditionnellement mâle stérile ayant le génotype MsMsrfrf, ledit procédé comprenant les étapes consistant :
a) à mettre à disposition une plante de *Brassica napus* conditionnellement mâle stérile, ayant le génotype MsMsrfrf, ladite plante de *Brassica napus* conditionnellement mâle stérile étant,
i) homozygote pour l'allèle de la stérilité mâle (allèle Ms) pouvant être obtenu à partir de la graine de *Brassica napus* déposée sous le Numéro de Dépôt NCIMB 41480, et
ii) homozygote pour l'allèle mainteneur (allèle rf) pouvant être obtenu à partir de la graine de *Brassica napus* déposée sous le Numéro de Dépôt NCIMB 41480, et
iii) principalement mâle stérile après exposition avant et/ou pendant la floraison à une température inférieure à 28°C, et
iv) retournant à un phénotype principalement mâle fertile après exposition avant et/ou pendant la floraison à une température supérieure à 35°C,
ledit génotype MSMSrfrf pouvant être obtenu à partir de la graine de *Brassica napus* déposée sous le Numéro de Dépôt NCIMB 41480,
b) à exposer ladite plante de *Brassica napus* conditionnellement mâle stérile pendant au moins 4 heures à une température supérieure à 35°C, et
c) à exposer la plante de *Brassica napus* conditionnellement mâle stérile ayant subi un traitement thermique, obtenue dans l'étape b), à une température inférieure à 33°C jusqu'à développement de fleurs mâles fertiles, et
d) à permettre l'autopollinisation des plantes de *Brassica napus* ayant lesdites fleurs mâles fertiles obtenues dans l'étape c), à laisser la graine se développer et à récolter la graine, les graines récoltées étant **caractérisées en ce qu'**il s'agit de graines d'une lignée de *Brassica napus* conditionnellement mâle stérile ayant le phénotype MsMsrfrf.

2. Procédé de production d'une graine d'une lignée de *Brassica napus* conditionnellement mâle stérile ayant le génotype Msmsrfrf, ledit procédé comprenant les étapes consistant
a) à mettre à disposition en tant que plante femelle une lignée de *Brassica napus* conditionnellement mâle stérile ayant le génotype MsMsrfrf par utilisation du procédé selon la revendication 1 pour obtenir des graines d'une lignée de *Brassica napus* conditionnellement mâle stérile ayant le génotype MsMsrfrf, et par culture desdites plantes à partir desdites graines, ladite plante de *Brassica napus* conditionnellement mâle stérile ayant le génotype MsMsrfrf étant
i) homozygote pour l'allèle de la stérilité mâle (Ms) pouvant être obtenu à partir de la graine de *Brassica napus* déposée sous le Numéro de Dépôt NCIMB 41480, et
ii) homozygote pour l'allèle mainteneur (allèle rf) pouvant être obtenu à partir de la graine de *Brassica napus* déposée sous le Numéro de Dépôt NCIMB 41480 ou 41481, et
iii) principalement mâle stérile à une température inférieure à 28°C, et
iv) retournant à un phénotype mâle fertile à une température supérieure à 35°C,
v) pouvant être obtenue à partir de la graine de *Brassica napus* déposée sous le Numéro de Dépôt NCIMB 41480, et
b) à mettre à disposition en tant que plante mâle une plante de *Brassica napus* conditionnellement mâle stérile ayant le génotype msmsrfrf, ladite plante de *Brassica napus* ayant le génotype msmsrfrf étant
i) homozygote pour l'allèle de la fertilité (allèle ms) pouvant être obtenu à partir de la graine de *Brassica napus* déposée sous le Numéro de Dépôt NCIMB 41481, et
ii) homozygote pour l'allèle mainteneur (allèle rf) pouvant être obtenu à partir de la graine de *Brassica napus* déposée sous le Numéro de Dépôt NCIMB 41480 ou 41481, et
iii) principalement mâle fertile,
iv) pouvant être obtenue à partir de la graine de *Brassica napus* déposée sous le Numéro de Dépôt NCIMB 41481, et
c) à permettre à la plante mâle de l'étape b) de polliniser la plante femelle de l'étape a), à laisser la graine se développer et à récolter la graine, les graines récoltées étant **caractérisées en ce qu'**il s'agit de graines d'une lignée de *Brassica napus* conditionnellement mâle stérile ayant le génotype Msmsrfrf.

3. Procédé de production ou de multiplication d'une graine d'une lignée de *Brassica napus* conditionnellement mâle stérile ayant le génotype Msmsrfrf selon la revendication 2, dans lequel ladite lignée de plante mâle et ladite lignée de plante femelle sont mises à disposition par introgression des allèles Ms, ms et/ou rf dans une lignée de *Brassica napus* consanguine, suivie d'au moins un rétrocroisement contre ladite lignée de *Brassica napus* consanguine.

4. Procédé de production d'une graine hybride mâle fertile de *Brassica napus,* ledit procédé comprenant les étapes consistant
a) à mettre à disposition en tant que plante femelle une plante de *Brassica napus* conditionnellement mâle stérile ayant le génotype Msmsrfrf ou MsMsrfrf par utilisation du procédé selon l'une quelconque des revendications 1 à 3 pour obtenir des graines d'une lignée de *Brassica napus* conditionnellement mâle stérile ayant le génotype Msmsrfrf ou MsMsrfrf et par culture desdites plantes à partir desdites graines, ladite plante de *Brassica napus* conditionnellement mâle stérile femelle étant
i. hétérozygote ou homozygote pour l'allèle de la stérilité mâle (allèle Ms) pouvant être obtenu à partir de la graine de *Brassica napus* déposée sous le Numéro de Dépôt NCIMB 41480, et
ii. homozygote pour l'allèle mainteneur (allèle rf) pouvant être obtenu à partir de la graine de *Brassica napus* déposée sous le Numéro de Dépôt NCIMB 41480 ou 41481, et
iii. principalement mâle stérile à une température inférieure à 28°C, et
iv. retournant à un phénotype mâle fertile à une température supérieure à 35°C, et
b) à mettre à disposition en tant que plante mâle une plante de *Brassica napus* mâle fertile ayant le génotype RfRf, ladite plante de *Brassica napus* mâle fertile étant
i. homozygote pour l'allèle restaurateur fonctionnel (allèle Rf), qui peut être obtenu à partir d'une lignée de *Brassica napus* consanguine fertile quelconque, commercialisée sous forme d'une graine pour culture, et
ii. principalement mâle fertile, et
c) à permettre à la plante mâle de l'étape b) de polliniser la plante conditionnellement mâle stérile femelle de l'étape a), à laisser la graine se développer et à récolter ladite graine hybride fertile.

5. Procédé de production d'une graine hybride mâle fertile de *Brassica napus* selon la revendication 4, dans lequel ladite lignée de plante mâle (mâle fertile) et ladite lignée de plante femelle (mâle stérile) se fondent sur un fond génétiquement divers et/ou dans lequel ladite lignée femelle (mâle stérile) est hétérozygote pour l'allèle Ms.

6. Procédé selon l'une quelconque des revendications 2 à 5, dans lequel les plantes femelles (mâles stériles) et les plantes mâles (mâles fertiles) sont cultivées en bandes alternées, et/ou dans lequel la floraison des plantes mâles est retardée par rabattement ou traitement avec des produits chimiques retardateurs de croissance, ou par ensemencement des plantes mâles (mâles fertiles) jusqu'à 3 semaines plus tard que les plantes femelles (mâles stériles).

7. Procédé de production d'une graine hybride de *Brassica napus* qui donne des plantes de *Brassica napus* produisant des grains ayant en option une teneur totale en glucosinolate non supérieure à 25 µmol par gramme de graine séchée à l'air pour une humidité de 9 %, ledit procédé comprenant un ou plusieurs des procédés tels que revendiqués dans
a) la revendication 1, et
b) l'une quelconque des revendications 2 et 3, et
c) l'une quelconque des revendications 4 à 6.

8. Procédé selon l'une quelconque des revendications 1 à 7, dans lequel l'allèle Ms est **caractérisé en ce qu'**il confère un phénotype de stérilité mâle nucléaire conditionnelle, qui
a) est provisoirement restauré en fertilité par une exposition à une température supérieure à 35°C,
b) est restauré en fertilité dans au moins une partie des plantes F₁ obtenues par croisement d'une plante mâle stérile ayant le génotype MsMsrfrf ou Msmsrfrf avec une plante de *Brassica napus* quelconque comprenant au moins un allèle Rf dominant, et
c) est maintenu dans les plantes F₁ obtenues par croisement d'une plante ayant un phénotype mâle stérile conditionnel conféré par ledit allèle Ms avec les plantes mâles fertiles dérivant de la graine déposée sous le Numéro de Dépôt NCIMB 41481.

9. Procédé selon l'une quelconque des revendications 1 à 8, dans lequel l'allèle Ms est l'allèle Ms présent dans la graine déposée sous le Numéro de Dépôt NCIMB 41480 ou un variant génétique de cette dernière, qui confère un phénotype conditionnellement mâle stérile.

10. Procédé selon l'une quelconque des revendications 1 à 9, dans lequel le phénotype conditionnellement mâle stérile et/ou l'allèle Ms sont liés et/ou associés à une ou plusieurs caractéristiques choisies dans le groupe consistant en
i. un phénotype d'avortement du bourgeon dans une plante ayant un phénotype mâle stérile conféré par l'allèle Ms,
ii. un phénotype de pétales à raies blanches ou à marbrures blanches dans une plante ayant un phénotype mâle stérile conféré par l'allèle Ms, et
iii. la présence d'un marqueur spécifique de l'allèle Ms tant dans les plantes mâles fertiles que dans les plantes mâles stériles comprenant au moins une copie de l'allèle Ms.

11. Procédé selon l'une quelconque des revendications 1 à 10, dans lequel le phénotype conditionnellement mâle stérile et/ou l'allèle Ms est lié et/ou associé à un ou plusieurs marqueurs choisis dans le groupe consistant en
1. les marqueurs choisis dans le groupe de polymorphismes dans la région du marqueur NR1116 consistant en
a) le marqueur de polymorphisme nucléotidique ayant un A sur la position correspondant à la position 85 de SEQ ID N°3,
b) le marqueur de polymorphisme nucléotidique ayant un G sur la position correspondant à la position 87 de SEQ ID N°3,
c) le marqueur de polymorphisme nucléotidique ayant un A sur la position correspondant à la position 139 de SEQ ID N°3,
d) le marqueur de polymorphisme nucléotidique ayant un C sur la position correspondant à la position 214 de SEQ ID N°3,
e) le marqueur de polymorphisme nucléotidique ayant un G sur la position correspondant à la position 218 de SEQ ID N°3,
f) le marqueur de polymorphisme nucléotidique ayant un G sur la position correspondant à la position 277 de SEQ ID N°3,
g) le marqueur de polymorphisme nucléotidique ayant un A sur la position correspondant à la position 286 de SEQ ID N°3,
h) le marqueur de polymorphisme nucléotidique ayant un T sur la position correspondant à la position 312 de SEQ ID N°3,
i) le marqueur de polymorphisme nucléotidique ayant un T sur la position correspondant à la position 319 de SEQ ID N°3,
j) le marqueur de polymorphisme nucléotidique ayant un C sur la position correspondant à la position 359 de SEQ ID N°3,
k) la mutation par délétion 5'-TTGGTGAACAATC-3' sur la position correspondant à 221 dans SEQ ID N°3, et
l) la mutation par insertion 5'-GAA-3' sur la position correspondant à 328-330 dans SEQ ID N°3,
II. les marqueurs choisis dans le groupe de polymorphismes dans la région du marqueur NR2525 consistant en
a) le marqueur de polymorphisme nucléotidique ayant un A sur la position correspondant à la position 60 de SEQ ID N°6,
b) le marqueur de polymorphisme nucléotidique ayant un T sur la position correspondant à la position 92 de SEQ ID N°6,
c) le marqueur de polymorphisme nucléotidique ayant un T sur la position correspondant à la position 105 de SEQ ID N°6,
d) le marqueur de polymorphisme nucléotidique ayant un C sur la position correspondant à la position 158 de SEQ ID N°6,
e) le marqueur de polymorphisme nucléotidique ayant un T sur la position correspondant à la position 431 de SEQ ID N°6,
f) la mutation par délétion d'un nucléotide unique sur la position correspondant à la position 82 de SEQ ID N°6, et
g) la mutation par délétion 5'-TGAGCAAAA-3' sur la position correspondant aux positions 17 à 25 de SEQ ID N°6,
III. les marqueurs choisis dans le groupe de marqueurs SNP consistant en
a) un signal positif dans l'essai SNP utilisant une sonde SNP comprenant la séquence nucléotidique décrite par SEQ ID N°2 et un signal négatif utilisant une sonde SNP comprenant la séquence nucléotidique décrite par SEQ ID N°11, et
b) un signal positif dans un essai SNP utilisant une sonde SNP comprenant la séquence nucléotidique décrite par SEQ ID N°17 et un signal négatif utilisant une sonde SNP comprenant la séquence nucléotidique décrite dans SEQ ID N°18,
IV. les marqueurs choisis dans le groupe de marqueurs SSR, consistant en :
a) un fragment PCR ayant une masse moléculaire apparente de 96,7 (± 1,0) bp, résultant d'une réaction PCR avec les amorces ayant les séquences décrites par SEQ ID N°1 et 2, et
b) un fragment PCR ayant une masse moléculaire apparente de 192,8 (± 0,3) bp résultant d'une réaction PCR avec les amorces ayant les séquences décrites par SEQ ID N°4 et 5, et
V. les marqueurs choisis dans le groupe de marqueurs liés à au moins l'une des séquences présentées dans SEQ ID N°3, 6 11 et 18,
où le ou les marqueurs (marqueurs de l'allèle Ms) comprennent aussi une séquence nucléotidique isolée choisie dans le groupe consistant en les séquences qui
I. ont une identité de séquence d'au moins 80 % avec les séquences de marqueur définies ci-dessus dans les sections I à V, ou
II. s'hybrident dans des conditions stringentes à ces séquences, ou
III. comprennent au moins 25 nucléotides consécutifs de ces séquences.

12. Procédé selon l'une quelconque des revendications 1 à 7, dans lequel l'allèle ms est **caractérisé par** les propriétés phénotypiques consistant
a) à ne pas être capable de ramener à fertilité le phénotype mâle stérile conféré par l'allèle Ms, et
b) à ne pas être capable de conférer un phénotype mâle stérile en l'absence d'un allèle Ms.

13. Procédé selon l'une quelconque des revendications 1 à 7 et 12, dans lequel l'allèle ms est lié et/ou est associé à un ou plusieurs marqueurs choisis dans le groupe consistant en :
I. les marqueurs choisis dans le groupe de polymorphismes dans la région du marqueur NR1116 consistant en
a) le marqueur de polymorphisme nucléotidique ayant un G sur la position correspondant à la position 85 de SEQ ID N°3,
b) le marqueur de polymorphisme nucléotidique ayant un A sur la position correspondant à la position 87 de SEQ ID N°3,
c) le marqueur de polymorphisme nucléotidique ayant un T sur la position correspondant à la position 139 de SEQ ID N°3,
d) le marqueur de polymorphisme nucléotidique ayant un T sur la position correspondant à la position 214 de SEQ ID N°3,
e) le marqueur de polymorphisme nucléotidique ayant un T sur la position correspondant à la position 218 de SEQ ID N°3,
f) le marqueur de polymorphisme nucléotidique ayant un A sur la position correspondant à la position 277 de SEQ ID N°3,
g) le marqueur de polymorphisme nucléotidique ayant un G sur la position correspondant à la position 286 de SEQ ID N°3,
h) le marqueur de polymorphisme nucléotidique ayant un A sur la position correspondant à la position 312 de SEQ ID N°3,
i) le marqueur de polymorphisme nucléotidique ayant un C sur la position correspondant à la position 319 de SEQ ID N°3,
j) le marqueur de polymorphisme nucléotidique ayant un T sur la position correspondant à la position 359 de SEQ ID N°3,
k) la mutation par délétion 5'-TTGGTGAACAATC-3' sur la position correspondant à 221 dans SEQ ID N°3, et
l) la mutation par insertion 5'-GAA-3' sur la position correspondant à 328-330 dans SEQ ID N°3,
II. les marqueurs choisis dans le groupe de polymorphismes dans la région du marqueur NR2525 consistant en
a) le marqueur de polymorphisme nucléotidique ayant un C sur la position correspondant à la position 60 de SEQ ID N°6,
b) le marqueur de polymorphisme nucléotidique ayant un C sur la position correspondant à la position 92 de SEQ ID N°6,
c) le marqueur de polymorphisme nucléotidique ayant un C sur la position correspondant à la position 105 de SEQ ID N°6,
d) le marqueur de polymorphisme nucléotidique ayant un A sur la position correspondant à la position 158 de SEQ ID N°6,
e) le marqueur de polymorphisme nucléotidique ayant un C sur la position correspondant à la position 431 de SEQ ID N°6,
f) le marqueur de polymorphisme nucléotidique ayant un T sur la position correspondant à la position 82 de SEQ ID N°6, et
g) la mutation par insertion 5'-TGAGCAAAA-3' sur la position correspondant aux positions 17 à 25 de SEQ ID N°6,
III. les marqueurs choisis dans le groupe de marqueurs SNP consistant en
a) un signal positif dans l'essai SNP utilisant une sonde SNP comprenant la séquence nucléotidique décrite par SEQ ID N°11 et un signal négatif utilisant une sonde SNP comprenant la séquence nucléotidique décrite par SEQ ID N°12, et
b) un signal positif dans un essai SNP utilisant une sonde SNP comprenant la séquence nucléotidique décrite par SEQ ID N°18 et un signal négatif utilisant une sonde SNP comprenant la séquence nucléotidique décrite dans SEQ ID N°17,
IV. les marqueurs choisis dans le groupe de marqueurs SSR consistant en :
a) un fragment PCR ayant une masse moléculaire apparente choisie dans le groupe de masses apparentes consistant en 94 (± 0,9) bp, 110,4 (± 0,5) bp, 112,3 (± 0,4) bp et 116,3 (± 0,4) bp, résultant d'une réaction PCR avec les amorces ayant les séquences présentées dans SEQ ID N°1 et 2, et
b) un fragment PCR ayant une masse moléculaire apparente de 183,8 (± 0,4) bp, ou un fragment PCR non associé à un allèle fertile, résultant d'une réaction PCR avec les amorces ayant les séquences présentées dans SEQ ID N°4 et 5,
où le ou les marqueurs comprennent aussi une séquence nucléotidique isolée choisie dans le groupe consistant en les séquences qui
a) ont une identité de séquence d'au moins 80 % avec les séquences de marqueur définies ci-dessus dans les sections I à IV, ou
b) s'hybrident dans des conditions stringentes à ces séquences, ou
c) comprennent au moins 25 nucléotides consécutifs de ces séquences.

14. Procédé selon l'une quelconque des revendications 1 à 7, dans lequel l'allèle rf est **caractérisé par** les propriétés phénotypiques consistant
a) à ne pas être capable de ramener à fertilité le phénotype mâle stérile conféré par l'allèle Ms, et
b) à être capable de maintenir le phénotype mâle stérile conféré par l'allèle Ms.

15. Procédé selon l'une quelconque des revendications 1 à 7 et 14, dans lequel l'allèle rf est choisi dans le groupe consistant en
a) l'allèle rf pouvant être obtenu à partir de la graine de *Brassica napus* déposée sous le Numéro de Dépôt NCIMB 41480 ou 41481, et
b) les variants de ce dernier, qui se présentent sous une forme homozygote capable de maintenir le phénotype de stérilité mâle conféré par l'allèle Ms.

16. Procédé selon l'une quelconque des revendications 1 à 7, 14 et 15, dans lequel l'allèle rf est lié et/ou est associé aux marqueurs SSR consistant en un fragment PCR ayant une masse moléculaire apparente de 240,8 (± 0,4) bp résultant d'une réaction PCR avec les amorces ayant les séquences présentées dans SEQ ID N°19 et 20.

17. Procédé selon l'une quelconque des revendications 1 à 7, dans lequel le phénotype de restauration de la fertilité et/ou l'allèle Rf sont liés et/ou associés à une ou plusieurs caractéristiques choisies dans le groupe consistant en
a) la restauration de la fertilité dans les plantes F₁ obtenues par croisement avec la plante de *Brassica napus* cultivée à partir de la graine déposée sous le Numéro de Dépôt NCIMB 41480, et
b) la restauration de la fertilité dans les plantes F₁ obtenues par croisement avec la plante de *Brassica napus* cultivée à partir de la graine obtenue par croisement de la plante de *Brassica napus* obtenue à partir de la graine déposée sous le Numéro de Dépôt NCIMB 41480 en tant que plante mâle stérile femelle et la plante de *Brassica napus* obtenue à partir de la graine déposée sous le Numéro de Dépôt NCIMB 41481 en tant que plante mâle fertile.

18. Procédé selon l'une quelconque des revendications 1 à 17, dans lequel l'allèle Rf et/ou l'allèle ms sont obtenus à partir d'une lignée de *Brassica napus* consanguine fertile du commerce choisie dans le groupe consistant en les variétés de *Brassica napus* non hybrides commercialisées sous forme de graines à des fins de culture, incluses dans la liste de l'OCDE de variétés éligibles à une certification, en date de décembre 2006.

19. Procédé selon l'une quelconque des revendications 1 à 7 et 17 à 18, dans lequel le phénotype de restauration de la fertilité et/ou l'allèle Rf sont liés et/ou associés à un marqueur SSR consistant en l'absence d'un fragment PCR ayant une masse moléculaire apparente de 240,8 (± 0,4) bp résultant d'une réaction PCR avec les amorces ayant les séquences présentées dans SEQ ID N° 19 et 20.

20. Amorces oligonucléotidique choisie dans le groupe de séquences décrites par SEQ ID N°1, 2, 4, 5, 7, 8, 9, 10, 11, 12, 13, 14, 15, 16, 17, 18, 19 et 20.

21. Sonde comprenant en tant que partie acide nucléique une séquence choisie dans le groupe de séquences décrites par SEQ ID N°11, 12, 17 et 19, la sonde convenant à la détection d'un polymorphisme nucléotidique.

22. Séquence nucléotidique isolée choisie dans le groupe consistant en les séquences présentées dans SEQ ID N°3, 6 et 21, lesdites séquences nucléotidiques isolées étant des marqueurs utiles pour la détection de l'allèle Ms, l'allèle ms, l'allèle Rf et/ou l'allèle rf dans le plasma germinatif de *Brassica* et les génotypes associés.

23. Utilisation d'une séquence d'acide nucléique selon les revendications 20 ou 22 lors d'une sélection, à base d'un marqueur, pour l'introgression d'allèles choisis dans le groupe consistant en l'allèle Ms, l'allèle ms, l'allèle Rf et/ou l'allèle rf dans un plasma germinatif de *Brassica* ne possédant pas ledit ensemble d'allèles.

24. Utilisation d'une plante de *Brassica napus* mâle fertile ayant le phénotype RfRf dans un procédé de production d'une graine hybride fertile de *Brassica napus* selon l'une quelconque des revendications 4 à 6.

25. Utilisation selon la revendication 23 ou 24, lequel procédé étant un procédé tel que revendiqué dans l'une quelconque des revendications 4 à 19.

26. Utilisation d'une ou plusieurs plantes de *Brassica napus* choisies parmi les plantes de *Brassica napus* conditionnellement mâles stériles ayant le génotype MsMsrfrf et/ou les plantes de *Brassica napus* mâles fertiles ayant le génotype msmsrfrf dans le procédé selon l'une quelconque des revendications 2, 3 et 6 à 19 pour produire une plante de *Brassica napus* conditionnellement mâle stérile ayant le génotype Msmsrfrf, ou une graine de cette dernière.


### SEQUENCE LISTING

<110> Syngenta Participation AG
<120> New hybrid system for Brassica napus
<130> 71598 EP
<150> EP 07290741.3
   <151> 2007-06-13
<160> 31
<170> PatentIn version 3.4
<210> 1
   <211> 20
   <212> DNA
   <213> Artificial
<220>
   <223> Forward Primer Sequence for Marker NR1116
<400> 1
   tcttcaaggg attcattcgg 20
<210> 2
   <211> 20
   <212> DNA
   <213> Artificial
<220>
   <223> Reverse Primer Sequence for Marker NR1116
<400> 2
   gaaacttcgt cgaatcctcg 20
<210> 3
   <211> 381
   <212> DNA
   <213> Artificial
<220>
   <223> NR1116 SNP region reference sequence (consensus sequence of sterile and fertile haplotype)
<220>
   <221> repeat_region
   <222> (1)..(16)
   <223> SSR repeat region (part)
<220>
   <221> mutation
   <222> (85)..(85)
   <223> SNP mutation; fertile haplotype = G; sterile haplotype =A
<220>
   <221> mutation
   <222> (87)..(87)
   <223> SNP mutation; fertile haplotype = A; sterile haplotype =G
<220>
   <221> mutation
   <222> (139)..(139)
   <223> SNP mutation; fertile haplotype = T; sterile haplotype =A
<220>
   <221> mutation
   <222> (214)..(214)
   <223> SNP mutation; fertile haplotype = T; sterile haplotype =C
<220>
   <221> mutation
   <222> (218)..(218)
   <223> SNP mutation; fertile haplotype = T; sterile haplotype =G
<220>
   <221> mutation
   <222> (245)..(257)
   <223> SNP mutation; fertile haplotype = insertion; sterile haplotype = deletion
<220>
   <221> mutation
   <222> (277)..(277)
   <223> SNP mutation; fertile haplotype = A; sterile haplotype = G
<220>
   <221> mutation
   <222> (286)..(286)
   <223> SNP mutation; fertile haplotype = G; sterile haplotype = A
<220>
   <221> mutation
   <222> (312)..(312)
   <223> SNP mutation; fertile haplotype = A; sterile haplotype = T
<220>
   <221> mutation
   <222> (319)..(319)
   <223> SNP mutation; fertile haplotype = C; sterile haplotype = T
<220>
   <221> mutation
   <222> (328)..(330)
   <223> SNP mutation; fertile haplotype = deletion; sterile haplotype = insertion
<220>
   <221> mutation
   <222> (359)..(359)
   <223> SNP mutation: fertile haplotype = T; sterile haplotype = C
<400> 3
   gagagagaga gagagacact tcgatgaata tagcttcgag gattcgacga agtttcttta     60
   gagaggagaa gaggaaactt cctagtataa atggatcctc gagcaggaac gaagatgatg    120
   atttgctagg agtgactgtt gaattgatcg atcacgtcag atctttcacc attgacacgt    180
   ttaagaactt ctctctctac ggtaatttcg aattcgattc tcaatttgat gatttttttc    240
   tcgattggtg aacaatcaag cagtagtgat atggggatta tgtttgtgga actagacgaa    300
   gaagcgtgtg taaatcctct ggaagaagaa gatgaagaag tgagctcctc tgagaatgtg    360
   aagaagtatt gtctgattgc c                                              381
<210> 4
   <211> 21
   <212> DNA
   <213> Artificial
<220>
   <223> Forward Primer sequence for marker NR2525 (Acc.No. BZ061557)
<400> 4
   attaccattt ccaacgaatc t 21
<210> 5
   <211> 23
   <212> DNA
   <213> Artificial
<220>
   <223> Reverse Primer sequence for marker NR2525 (Acc.No. BZ061557
<400> 5
   gtctctttct caactcttgt atc 23
<210> 6
   <211> 434
   <212> DNA
   <213> Artificial
<220>
   <223> NR2525 SNP region reference sequence
<220>
   <221> mutation
   <222> (17)..(25)
   <223> deletion (sterile) -> "TGAGCAAAA" (fertile) insertion mutation
<220>
   <221> mutation
   <222> (60)..(60)
   <223> A (sterile) -> C (fertile) SNP mutation
<220>
   <221> mutation
   <222> (82)..(82)
   <223> single nucleotide deletion (sterile) -> T insertion (fertile) SNP mutation
<220>
   <221> mutation
   <222> (92)..(92)
   <223> T (sterile) -> C (fertile) SNP mutation
<220>
   <221> mutation
   <222> (105)..(105)
   <223> T (sterile) -> C (fertile) SNP mutation
<220>
   <221> mutation
   <222> (158)..(158)
   <223> C (sterile) -> A (fertile) SNP mutation
<220>
   <221> mutation
   <222> (431)..(431)
   <223> T (sterile) -> C (fertile) SNP mutation
<400> 6
   cagagaaaat gattaatgag caaaagcaaa agtttacaac acaaagggac tttctgctac     60
   cctcaacagt catcaggcct ttaaaattcc tcaagattaa agctctcaat taatcctaat    120
   agcaacctta gatttaacat tacttcttca tttgacaaac attaccacag ctaattgggc    180
   ttatttcact attcatatca ataacaatag tttcccaatc aactaaaaac agaagggaaa    240
   acccaccttc gtttaacatt ctaaaatcca aataattgga ctcaatatga agctaaaagc    300
   cctaacaatc cgacaagttc acggcctaca ttgaagcaga gaaccagaaa acgcaacaaa    360
   taaatatcag aaaccacatt accatttcca acgaatctat aggagctgct tgagagaagt    420
   gaatccatgg ccgg                                                      434
<210> 7
   <211> 23
   <212> DNA
   <213> Artificial
<220>
   <223> Oligonucleotide primer HiNK6440
<400> 7
   gttcacttct catcttcttc cag 23
<210> 8
   <211> 25
   <212> DNA
   <213> Artificial
<220>
   <223> oligonucleotide sequence for PCR primer HiNK6441
<400> 8
   gagagagaca cttcgatgaa tatag 25
<210> 9
   <211> 22
   <212> DNA
   <213> Artificial
<220>
   <223> oligonucleotide sequence for PCR primer HiNK6442
<400> 9
   tcctggcaat cagacaatac tt 22
<210> 10
   <211> 21
   <212> DNA
   <213> Artificial
<220>
   <223> oligonucleotide sequence for PCR primer HiNK6697
<400> 10
   acacacgctt cttcgtctag t 21
<210> 11
   <211> 14
   <212> DNA
   <213> Artificial
<220>
   <223> Nucleotide part of SNP probe HiNK6700 (Fertile allele specific probe)
<220>
   <221> mutation
   <222> (6)..(6)
   <223> SNP mutation
<220>
   <221> mutation
   <222> (10)..(10)
   <223> SNP mutation
<400> 11
   cgaattcgat tctc 14
<210> 12
   <211> 14
   <212> DNA
   <213> Artificial
<220>
   <223> Nucleotide part of SNP probe HiNK6701 (Sterile allele specific probe)
<220>
   <221> misc_feature
   <222> (6)..(6)
   <223> SNP mutation
<220>
   <221> misc_feature
   <222> (10)..(10)
   <223> SNP mutation
<400> 12
   cgaatccgag tctc 14
<210> 13
   <211> 19
   <212> DNA
   <213> Artificial
<220>
   <223> oligonucleotide sequence for PCR primer HiNK6702
<400> 13
   agtaacatca gcggggaac 19
<210> 14
   <211> 22
   <212> DNA
   <213> Artificial
<220>
   <223> oligonucleotide sequence for PCR primer HiNK6707
<400> 14
   tttaagagca ttggaactct cc 22
<210> 15
   <211> 24
   <212> DNA
   <213> Artificial
<220>
   <223> oligonucleotide sequence for PCR primer HiNK6771
<400> 15
   tttacaacac aaagggcttt ctgc 24
<210> 16
   <211> 25
   <212> DNA
   <213> Artificial
<220>
   <223> oligonucleotide sequence for PCR primer HiNK6772
<400> 16
   tgtaggccgt gaacttgtcg gattg 25
<210> 17
   <211> 17
   <212> DNA
   <213> Artificial
<220>
   <223> Nucleotide part of SNP probe HiNK6775 (Sterile allele specific probe)
<220>
   <221> mutation
   <222> (9)..(9)
   <223> SNP mutation
<400> 17
   atttgacaca cattacc 17
<210> 18
   <211> 17
   <212> DNA
   <213> Artificial
<220>
   <223> Nucleotide part of SNP probe HiNK6776 (Fertile allele specific probe)
<220>
   <221> mutation
   <222> (9)..(9)
   <223> SNP mutation
<400> 18
   atttgacaaa cattacc 17
<210> 19
   <211> 19
   <212> DNA
   <213> Artificial
<220>
   <223> Forward primer sequence for Marker 2219
<400> 19
   attatcctct cgccatttc 19
<210> 20
   <211> 21
   <212> DNA
   <213> Artificial
<220>
   <223> Reverse primer sequence for Marker 2219
<400> 20
   aaactcctga acacctccta c 21
<210> 21
   <211> 1032
   <212> DNA
   <213> Brassica napus
<220>
   <221> misc_feature
   <222> (20)..(22)
   <223> n is a, c, g, or t
<220>
   <221> misc_feature
   <222> (25) ..(27)
   <223> n is a, c, g, or t
<220>
   <221> misc_feature
   <222> (30)..(30)
   <223> n is a, c, g, or t
<220>
   <221> misc_feature
   <222> (40)..(41)
   <223> n is a, c, g, or t
<220>
   <221> misc_feature
   <222> (62)..(62)
   <223> n is a, c, g, or t
<220>
   <221> misc_feature
   <222> (66)..(66)
   <223> n is a, c, g, or t
<220>
   <221> misc_feature
   <222> (73)..(73)
   <223> n is a, c, g, or t
<220>
   <221> primer_bind
   <222> (461)..(480)
   <223> NR1116 forward primer binding site
<220>
   <221> repeat_region
   <222> (499)..(534)
   <223> SSR repeat region
<220>
   <221> primer_bind
   <222> (555)..(574)
   <223> NR1116 reverse primer binding site
<220>
   <221> misc_feature
   <222> (801)..(801)
   <223> n is a, c, g, or t
<220>
   <221> misc_feature
   <222> (819)..(819)
   <223> n is a, c, g, or t
<220>
   <221> misc_feature
   <222> (901)..(901)
   <223> n is a, c, g, or t
<220>
   <221> misc_feature
   <222> (906)..(906)
   <223> n is a, c, g, or t
<400> 21
   caccaatatt agcacacacn nnccnnnttn cctttttttn ngggcgctaa gctggtaacc 60
   gnaatnctgt tancccgggg gaacccgccc acttcatctt ctgtcgaacc aatccacctg 120
   acagacaaat ctcctcacat cggaccacca caactctctg tccgttgagc aactccttag 180
   cgatgatgga agcaagacgc cccaacatgt ggtgcctcgc gtcgaccacg acacgcttcg 240
   cgcatatccc tgaaccagac accatctctc cactgattcg attactctcg ggctgcttcc 300
   agaagattat gagtgtagac tcagtggcgg attatatacg acgcggctag tgaaacaatt 360
   agggtttctc gtctaaaacc taatgttaat gggcttttgt aattagattt taggcccaat 420
   aaaagcctct ttacctttac tttcttctgt ttcttgtcat tcttcaaggg attcattcgg 480
   gttcttcttg tgtcaccaga gagagagaga gagagagaga gagagagaga gagacacttc 540
   gatgaatata gcttcgagga ttcgacgaag tttctttaga gaggagaaga ggaaacttcc 600
   tagtataaat ggatcctcga gcaggaacga agatgatgat ttgctaggag tgactgttga 660
   attgatcgat cacgtcagat ctttcaccat tgacacgttt aagaacttct ctctctacgg 720
   taatttcgaa ttcgattctc aatttgatga tttttttctc gattggtgaa caatcaagca 780
   gtagtgatat ggggattatg nttgtgggac tagacgaang agcgtgtgta aatcctctgg 840
   aagaagatga gaagtgaact cctctgagaa tgtgaagaaa gtattgtctg attgccagga 900
   naaaangccg ttctcgtctt gtccaaatcc aaggttttga tacttccttc gcctaaagat 960
   tggacctgcc cagttttggt tgtaaaaata atttgttttc gcgattataa cattgggtaa 1020
   atttttaaga gc 1032
<210> 22
   <211> 741
   <212> DNA
   <213> Brassica napus
<220>
   <221> primer_bind
   <222> (514)..(534)
   <223> NR2525-Forward primer binding site
<220>
   <221> repeat_region
   <222> (643)..(688)
   <223> location of microsatellite sequence
<220>
   <221> primer_bind
   <222> (709)..(731)
   <223> NR2525-Reverse primer binding site
<400> 22
   aacggagatc tgattctcgc cctgtggtgg aattctgttt gatatgacct aagtaacatc 60
   agcggggaac aaagaaaatg tttacaacaa agaaaatgat taatgagcaa aagcaaaaca 120
   agtttacaac acactttatg catcagctga aaggaacagt gacacaaagg gactttctgc 180
   taccctcaaa cagtcatcag gcctttaaaa ttcctcaaga ttaaagcttt caataaatcc 240
   taatagcaac cttagattta acattactta ttcatttgac acacattaca acagctaatt 300
   gggcttattt cactattcgt atcaataaca atagtttccc aatcaacaac acctactaaa 360
   aacaaaaggg aaagaaaacc caccttcgtt tacattctaa aatccaaata attggactca    420
   atatgaagct aaaaccccta acactcgaca agttcactgc ctacattgaa gcagagaatc    480
   agaaaacgca acaaataaag atacagaaac cacattacca tttccaacga atctatagga 540
   gctgcttgag agaagtgaat ccatggccgg agagttccaa tgctcttaaa ccctaaaaga 600
   gttagatcta ctggcaattt taagtaaagt gagctgcttt aaagagagag agagagagag 660
   agagagagag agagagagag agggagagat gaatagagca cagatatcga tacaagagtt 720
   gagaaagaga ctcattgccg t 741
<210> 23
   <211> 754
   <212> DNA
   <213> Brassica napus
<220>
   <221> primer_bind
   <222> (298)..(316)
   <223> NR2219 forward primer binding site
<220>
   <221> repeat_region
   <222> (411)..(464)
   <223> SSR repeat region
<220>
   <221> primer_bind
   <222> (526)..(546)
   <223> NR2219 reverse primer binding site
<400> 23
   ggaagtgctt tttagtggag agtgcttcct gaaactcttc aggctttggc gatccaaaga 60
   gcacgatttg caataaaact tagcagatga tgatgatgat gattcaccag tgcttgtctt 120
   agttagtaat ttttcccaga gccactcttt gcgagtgcgc acgaacaatc ctgcgcgagg 180
   tttgactgaa taagccagaa aaaggtcgta acacgtgcgt tgttgtagcc gagacagatc 240
   ccaattgaag acatctaaca cctgattgga tatgatcatc attttagccg gaggaggatt 300
   atcctctcgc catttcacca tatctttata catgagtgcg cacgtgcatc ctatttcatt 360
   accatatatt cctttattaa ctaaaaaggc ccatatcttc cgcagatact tatatatata 420
   tatatatata tatatatata tatatatata tatatatata tataaaaaag ctggactaac 480
   cagatctggt atgtaccaca gcgactccag tggaagatag agctcgtagg aggtgttcag 540
   gagtttgttt ttggtctcca taggctgtga ctgtgatgga gacaggacca gtgtagccta 600
   gctccttgaa agctccttcc aaactgggac ggaccccacg agcatcgata ccctctggaa 660
   ccggacagtc aaacatgtcc caccacaccg ctattttagc cgccgccgcc gcagcagcat 720
   cgttcccacc ccattctctc tacacctttt aaca 754
<210> 24
   <211> 20
   <212> DNA
   <213> Artificial
<220>
   <223> Forward primer sequence for Marker 3454
<400> 24
   gatggtgatg gtgataggtc 20
<210> 25
   <211> 21
   <212> DNA
   <213> Artificial
<220>
   <223> Reverse primer sequence for Marker 3454
<400> 25
   gaagagaagg agtcagagat g 21
<210> 26
   <211> 581
   <212> DNA
   <213> Brassica napus
<220>
   <221> misc_feature
   <222> (1)..(581)
   <223> Sequence for Marker 3454
<400> 26
   ctcagaagcg gtgtggatct tgtctttcct cgtctcttcc tcgtcgctaa gaccagaacc      60
   catttcttga ttgctgcttg agatgttgga ctcttgaagt ttctcatctg atgacctttt     120
   gagattagtg agactcttgg tacccacctc aaaggatggg gatggtgatg gtgatggtga     180
   taggtcttct ctgtatgacg aatggtccag tagtactttc tgccttttga gacgtggacc      240
   atcatcacca ccacagcttt cggtttcatc cagttcttct tcttctgttg ttttattatt     300
   gcacgcttgt tgttcaactg ggaatggaga gtgacctatg ctcgtcactt catcatcatc     360
   actctcgcaa tcatcattgt cttcatcatc agaatacagt agtgcgttaa tttcctcagt     420
   gtcttcgtgc atctctgact ccttctcttc gccattgaca tgatcttcat ggaggaatat     480
   cttttctggg aatggcatga tctttactta accctttctc taggtttaga aaattgagaa     540
   atttctctgg atcagtagcc acaaaagagg gaaaacgaac t                         581
<210> 27
   <211> 19
   <212> DNA
   <213> Artificial
<220>
   <223> M13F tail sequence added to SFP probe in construction of forward primer
<400> 27
   cacgacgttg taaaacgac 19
<210> 28
   <211> 18
   <212> DNA
   <213> Artificial
<220>
   <223> M13R tail sequence added to SFP probe in construction of reverse primer
<400> 28
   caggaaacag ctatgacc 18
<210> 29
   <211> 20
   <212> DNA
   <213> Artificial
<220>
   <223> forward primer for PUT-161a-Brassica_napus-59218
<400> 29
   acagagacag aggaggtagc 20
<210> 30
   <211> 20
   <212> DNA
   <213> Artificial
<220>
   <223> reverse primer for PUT-161a-Brassica_napus-59218
<400> 30
   atcataatcc ctcgttcttt 20
<210> 31
   <211> 708
   <212> DNA
   <213> Brassica napus
<400> 31
   gcactggagg aggctgaagc tcttaagaag ctgactccta gacaagaacc tgtggtggat    60
   tcaaccaaat acactgctgc agatgtgcgc attacggacc agaaactgcg tttatgtgac   120
   atatgcggag cattcttgag cgtctatgac agtgatcgtc ggttagctga tcattttgga   180
   gggaagcttc atttgggtta catgctgatc cgtgataaac tagcagagct tcaggaggaa   240
   aagaacaaag ttcacaagga acgggtcgaa gagaggagat caaaggagag gagcagagag   300
   cgagaatcaa gtagagacag agacagagga ggtagccgtg accgtggaag agatatagac   360
   ggtagaagca gagatcgcga caggcaccat gaccaccgtg aacatgacag aaactataat   420
   cagtcacgtg gctatgactc aagaagccgg cgcagctcga ggtcccggtc tagggaaaga   480
   acgagggatt atgatcgccg cagacgtcat gaccgctact aagacgctgt cagagaaggt   540
   tgcaagcaag tttgagatgt tttcaaagat gcgtttagga tcaccaatct ggagttacaa   600
   acacttgttt tcgtatgtgt taaaagatat ttgagattgt aagttgctaa gtttgtaaga   660
   ggagtttcgt tggatttctt caaactttta atatgttgtt gacgaaaa                708